(19) Europäisches Patentamt
European Patent Office
Office européen des brevets

(11) EP 2 388 264 A1

(12) **EUROPEAN PATENT APPLICATION**
published in accordance with Art. 153(4) EPC

(43) Date of publication:
**23.11.2011 Bulletin 2011/47**

(21) Application number: **10731303.3**

(22) Date of filing: **15.01.2010**

(51) Int Cl.:
***C07H 15/26*** *(2006.01)* ***A61K 47/48*** *(2006.01)*
***A61K 49/00*** *(2006.01)* ***A61K 49/04*** *(2006.01)*
***A61K 51/00*** *(2006.01)*

(86) International application number:
**PCT/JP2010/050432**

(87) International publication number:
**WO 2010/082634 (22.07.2010 Gazette 2010/29)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **16.01.2009 US 145267 P**
**20.01.2009 JP 2009010158**

(71) Applicant: **Toyo Suisan Kaisha, Ltd.**
**Tokyo 108-8501 (JP)**

(72) Inventors:
- **OHTA, Keisuke**
  **Chiba 270-0101 (JP)**
- **MIURA, Masahiko**
  **Chiba 270-2253 (JP)**
- **SAKAGUCHI, Kengo**
  **Ibaraki 300-2667 (JP)**
- **SUGAWARA, Fumio**
  **Saitama 352-0012 (JP)**
- **ISHIMA, Masahiro**
  **Chiba 274-0823 (JP)**
- **MURATA, Hiroshi**
  **Chiba 278-0022 (JP)**

(74) Representative: **Lau, Sarah Jane**
**Kilburn & Strode LLP**
**20 Red Lion Street**
**London WC1R 4PJ (GB)**

(54) **COMPOUND RETAINED IN TUMOR**

(57) A novel compound which specifically resides in a tumor, a method for allowing it to reside in a tumor, and a method for detecting, diagnosing, and treating tumor with use thereof are provided. The present invention relates to a compound represented by chemical formula (I)

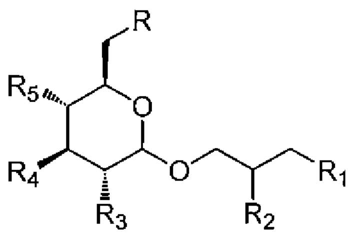

(I)

wherein R is an anionic group binding to hydrogen, $R_1$ is OH, OCOH, $OCO(CH_2)_hCH_3$, or an acting group, h being an integer of 0 or more, $R_2$ is H, OH, OCOH, $OCO(CH_2)_iCH_3$, or an acting group, i being an integer of 0 or more, $R_3$ is OH, $SO_3H$, or an acting group, $R_4$ is OH, $SO_3H$, or an acting group, and $R_5$ is OH, $SO_3H$, or an acting group, at least one of $R_1$, $R_2$, $R_3$, $R_4$, and $R_5$ containing an acting group, or pharmaceutically acceptable salts thereof.

EP 2 388 264 A1

## Description

Technical Field

The present invention relates to novel compound having a tumor-resident property.

Background Art

In the medical field, MRI, PET, X-raying, CT, and the like are used as diagnostic imaging techniques. Methods for detection and diagnosis using these techniques have been developed and used. In the methods, such substances that give rise to a difference in X-ray absorption in comparison with the surrounding tissues, substances that have an influence on nuclear magnetic resonance, or the like are used as a contrast agent. Such a contrast agent provides contrast for X-ray or MRI imaging by distribution in tissues and generates an image of the tissue shape.

In recent years, due to MRI, PET, X-raying, CT, and the like, not only a technique for obtaining information of the tissue pattern based on the aforementioned contrast but also a technique for detecting a tumor itself has been developed. Contrast agents used in such techniques are, for example, those prepared by labeling molecules like glucose, which is easily taken into carcinoma tissues whose metabolism is enhanced compared to normal cells, by a radioactive substance, fluorescent substance, magnetic substance, or the like and those prepared by labeling, in the same manner, molecules such as sugar chains specifically existing on a carcinoma cell surface and proteins aggregating by targeting at antigens. These contrast agents are utilized for detection and/or imaging of carcinoma tissues by a detector such as PET, MRI, or CT scanner.

On the other hand, as a delivery method of a drug to carcinoma tissues, technique of a drug delivery system delivering by encapsulating drugs such as an anticancer agent in minute particles or associating therewith has been developed. Such a drug delivery system is, for example, a system in which molecules having an affinity with tumor tissues and molecules preventing from being eliminated as a foreign substance are incorporated into the particle surface.

Summary of the Invention

[PROBLEMS TO BE SOLVED BY THE INVENTION]

An object of the present invention is to provide a novel compound which specifically resides in a tumor.

Another object of the present invention is to provide a method for allowing the compound to reside in a tumor.

Another object of the present invention is to provide a method for detecting tumor with use of the compound.

Another object of the present invention is to provide a method for diagnosing tumor with use of the compound.

Another object of the present invention is to provide a method for treating tumor with use of the compound.

[MEANS FOR SOLVING THE PROBLEMS]

The aforementioned problem can be solved by the present invention described below.

(1) A compound represented by chemical formula (I):

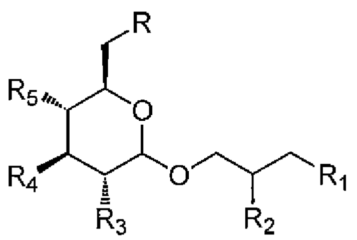

(I)

wherein R is an anionic group binding to hydrogen,
$R_1$ is OH, OCOH, $OCO(CH_2)_hCH_3$, or an acting group, h being an integer of 0 or more,
$R_2$ is H, OH, OCOH, $OCO(CH_2)_iCH_3$, or an acting group, i being an integer of 0 or more,
$R_3$ is OH, $SO_3H$, or an acting group,
$R_4$ is OH, $SO_3H$, or an acting group, and
$R_5$ is OH, $SO_3H$, or an acting group,

at least one of $R_1$, $R_2$, $R_3$, $R_4$, and $R_5$ containing an acting group, or pharmaceutically acceptable salts thereof.

(2) A compound represented by chemical formula (II):

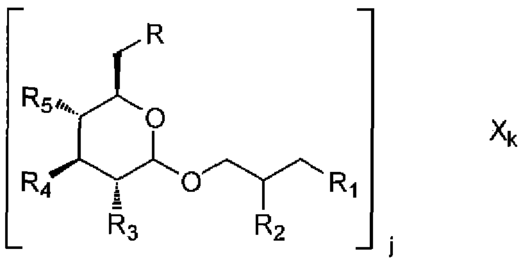

(II)

wherein R is an anionic group binding to hydrogen,
$R_1$ is OH, OCOH, $OCO(CH_2)_hCH_3$, or an acting group, h being an integer of 0 or more,
$R_2$ is H, OH, OCOH, $OCO(CH_2)_iCH_3$, or an acting group, i being an integer of 0 or more,
$R_3$ is OH, $SO_3H$, or an acting group,
$R_4$ is OH, $SO_3H$, or an acting group,
$R_5$ is OH, $SO_3H$, or an acting group,
j is an integer of 1 or more,
X is an acting group or a cationic group, and
k is an integer of 1 or more,
at least one of $R_1$, $R_2$, $R_3$, $R_4$, $R_5$, and X containing an acting group,
or pharmaceutically acceptable salts thereof.

(3) A method for allowing the compounds represented by formula (I) or (II) to reside in a tumor, wherein the compound or salts described in (1) or (2) are administered to a subject.

(4) A method for detecting tumor, comprising:

administering the compound or salts described in (1) or (2) to a subject, wherein the acting group is a labeled substance;
detecting the labeled substance when the compound or salts exist in a tumor in a higher concentration than in other tissues except a tumor; and
detecting tumor in the subject based on results of the detecting.

(5) A method for diagnosing tumor, comprising:

administering the compound or salts described in (1) or (2) to a subject, wherein the acting group is a labeled substance;
detecting the labeled substance when the compound or salts exist in a tumor in a higher concentration than in other tissues except a tumor; and
diagnosing presence or absence of tumor in the subject based on results of the detecting.

(6) A method for treating tumor, comprising administering the compound or salts described in (1) or (2) to a subject, wherein the acting group is an antitumor substance.

[EFFECTS OF THE INVENTION]

According to an aspect of the present invention, a novel compound which specifically resides in a tumor is provided.

According to an aspect of the present invention, a method for allowing the compound to reside in a tumor is provided.

Further, according to an aspect of the present invention a method for detecting tumor with use of the compound is provided.

According to another aspect of the present invention, a method for diagnosing tumor with use of the compound is provided.

Furthermore, according to another aspect of the present invention, a method for treating tumor with use of the compound is provided,

Brief Description of the Drawings

FIG. 1 is a graph showing retention of biotinated αSQMG C18:0 in a tumor.
FIG. 2 is a graph showing retention of biotinated αSQAP C18:0 in a tumor.
FIG. 3 is a concentration-time transition curve in blood plasma and organs of nude mice to which αSQMG C18:0 was administered intraperitoneally.
FIG. 4 is a concentration-time transition curve in blood plasma and organs of nude mice to which αSQAP C18:0 was administered intraperitoneally.
FIG. 5 is a concentration-time transition curve in blood plasma and organs of nude mice to which αSQAP C18:0 was administered intravenously.

The Embodiments for Carrying Out the Invention

As a result of diligent study by the present inventors, a novel compound which specifically distributes in a tumor was found.

According to one embodiment of the present invention, the novel compound is a compound represented by the following formula (I):

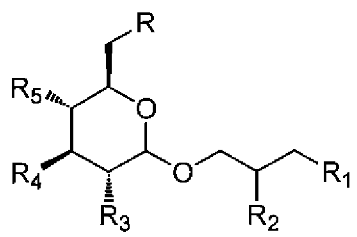

(I)

wherein R is an anionic group binding to hydrogen,
$R_1$ is OH, OCOH, $OCO(CH_2)_hCH_3$, or an acting group, h being an integer of 0 or more, preferably from 0 to 30, more preferably from 0 to 26, further preferably from 1 to 22,
$R_2$ is H, OH, OCOH, $OCO(CH_2)_iCH_3$, or an acting group, i being an integer of 0 or more, preferably from 0 to 30, more preferably from 0 to 26, further preferably from 1 to 22,
$R_3$ is OH, $SO_3H$, or an acting group,
$R_4$ is OH, $SO_3H$, or an acting group, and
$R_5$ is OH, $SO_3H$, or an acting group,
at least one of $R_1$, $R_2$, $R_3$, $R_4$, and $R_5$ containing an acting group, preferably any one of $R_1$, $R_2$, $R_3$, $R_4$, and $R_5$ containing an acting group,
or pharmaceutically acceptable salts thereof.

According to one embodiment, the compound of the present invention may be the compound of formula (I), wherein R is $SO_3H$,
$R_3$ is OH,
$R_4$ is OH,
$R_5$ is OH,
$R_1$ is OH, OCOH, $OCO(CH_2)_hCH_3$ (an acting group), O- (an acting group), OCO- (an acting group), or $OCO(CH_2)_hCH_2$-(an acting group), h being an integer of 0 or more, preferably from 0 to 30, more preferably from 0 to 26, further preferably from 1 to 22, and
$R_2$ is H, OH, OCOH, $OCO(CH_2)_iCH_3$, an acting group, O- (an acting group), OCO- (an acting group), or $OCO(CH_2)_iCH_2$-(an acting group), i being an integer of 0 or more, preferably from 0 to 30, more preferably from 0 to 26, further preferably from 1 to 22,
at least one of $R_1$ and $R_2$ containing an acting group,
or pharmaceutically acceptable salts thereof.

According to a further embodiment, the compound of the present invention is the compound represented by formula (I),
wherein R is $SO_3H$,
$R_1$ is OH, OCOH, $OCO(CH_2)_hCH_3$ or an acting group, h being an integer of 0 or more, preferably from 0 to 30, more preferably from 0 to 26, further preferably from 1 to 22,
$R_2$ is H, OH, OCOH, $OCO(CH_2)_iCH_3$, or an acting group, i being an integer of 0 or more, preferably from 0 to 30, more

preferably from 0 to 26, further preferably from 1 to 22,
$R_3 = R_4 = R_5$ is OH, or at least one of $R_3$, $R_4$, and $R_5$ is an active agent and the others are OH,
at least one of $R_1$, $R_2$, $R_3$, $R_4$, and $R_5$ containing an acting group,
or pharmaceutically acceptable salts thereof.

In the compound of the formula (I), when $R_1$ and/or $R_2$ is $OCO(CH_2)_hCH_3$ or $OCO(CH_2)_iCH_3$, they may be a linear or branched, saturated or unsaturated fatty acid.

Further, according to a further embodiment, the compound of the present invention may be sulfopyranosyl (acyl)glycerol, sufopyranosyl(acyl)propanediol, or sulfoquinovosylacyl propanediol, wherin at least one of the side residues binds to an acting group, or pharmaceutically acceptable salts thereof.

In the compound of the aforementioned formula (I), when a sugar backbone constituting pyranoside, pyranose, exists, the pyranose may be α-D-glucose, β-D-glucose, α-D-galactose, β-D-galactose, α-D-mannose, β-D-mannose, and the like.

These sugar backbones of the pyranoside may be in either configuration of a boat or a chair form, or a mixed form. However, a chair form is more preferable from the viewpoint of stability.

In the compound of formula (I), when there is an asymmetrical carbon, the absolute configuration may be S or R. For example, when the compound comprises sulfopyranosyl(acyl)glycerol and an acting group, the carbon at position 2 of the glycerol portion is an asymmetrical carbon. Also in this case the absolute configuration may be S or R, or a mixed form.

When the compound of formula (I) comprises sulfoquinovosylacyl propanediol and an acting group, the quinovose ring contained therein may be in a boat or chair form, or a mixed form. However, in general a chair form is preferable due to the stability. Further, the steric configuration of the propanediol with respect to the quinovose ring may be α-anomer or β-anomer, or the mixed configuration.

In the compound of formula (I), examples of the anionic group binding to hydrogen include, but not limited to, $SO_3H$, $OSO_3H$, $PO_3H_2$, $CO_2H$, and the like, preferably $SO_3H$, $OSO_3H$, $PO_3H_2$, more preferably $SO_3H$.

The term "acting group" used herein refers to a group exhibiting a desired action of delivering to a tumor. The compound of formula (I) may contains, but not limited to, an active substance such as a labeled substance and an antitumor substance as an acting group. The active substance is maintained by a covalent bond in any position in the compound represented by formula (I). The acting group may be contained in one or more in one molecule of the compound of formula (I). When two or more acting groups exist in one molecule of the compound of formula (I), these acting groups may be identical or different from each other or one another.

The acting group may be one of a coupled pair which is capable of specifically coupling to the other. Examples of such a coupled pair include, but not limited to, biotin and avidin, an antigen and a specific antibody against the antigen, and an antigen and a specific receptor with respect to the antigen. In the case of such a compound, the compound of the present invention comprising one of the coupled pair as an acting group is administered to a subject and then the other of the pair, with which a drug or labeled substance, is associated is administered to the subject after desired time interval, thereby specifically delivering the drug or labeled substance to tumor.

The acting group may or may not include a linker for linking the active substance and a glycolipid portion.

The term "labeled substance" used herein is a marker which is used in technique for diagnosing tumor utilizing imaging technique which is generally used in noninvasive diagnosis. The labeled substance may be any known vital-stainable labeled substance. For example, such a labeled substance includes, but not limited to, a radioactive substance, paramagnetic metal, radiopaque metal, and pigment.

Examples of the radioactive substance include, but not limited to, $^{11}C$, $^{13}N$, $^{15}O$, $^{18}F$, $^{58}Co$, $^{59}Fe$, $^{62}Cu$, $^{64}Cu$, $^{67}Ga$, $^{68}Ga$, $^{75}Br$, $^{76}Br$, $^{77}Br$, $^{82}Br$, $^{89}Sr$, $^{90}Y$, $^{111}I$, $^{113}Sn$ $^{117m}Sn$, $^{153}Sm$, $^{165}Dy$, $^{166}Ho$, $^{169}Er$, $^{186}Re$, $^{201}Tl$, $^{212}Bi$, and $^{213}Bi$.

Examples of the paramagnetic metal include, but not limited to, Cr (III), Mn (III), Fe (II), Fe (III), Pr (III), Nd (III), Sm (III), Yb (III), Gd (III), Tb (III), Dy (III), Ho (III), and Er (III).

Examples of the radiopaque metal include, but are not limited to, I, Bi, W, Ta, Hf, La, Ln, Ba, Mo, Nb, Zr, Sr, and the like.

Examples of the pigment may include, but not limited to, a known pigment for vital staining, for example, a pigment such as fluorescent pigment. For example, the fluorescent pigment includes those having the following skeleton as a fluorescent chromophore group: naphthalene, anthracene, quinoline, acridine, coumarin, salicylic acid, anthranilic acid, NBD (7-nitrobenz-2-oxa-1,3-diazole), stilbene, resorufin, BODIPY (4,4-difluoro-4-bora-3a,4a-diaza-s-indecene), carbocyanine, and thiacarbocyanine, tetramethylindocarbocyanine. Examples of the fluorescent pigment include the substances disclosed in R. P. Haugland, Molecular Probes Handbook, 6th edition.

The term 'antitumor substance' used herein may be a substance which attacks tumor by cytotoxicity. Therefore, the antitumor substance may be any known substance which is used for treating a tumor. The antitumor substance may be, for example, but not limited to, an alkylating agent, antimetabolite, anticancer antibiotic, metallic complex, plant alkanoids, topoisomerase inhibitor, microtubular polymerization inhibitor, microtubular depolymerization inhibitor, molecular target drug, and hormonal agent. Examples of the antitumor substance include, but not limited to, Aceglatone, Aclarubicin hydrochloride, Actinomycin D (referred to also as Dactinomycin), Amrubicin hydrochloride, Anastrozole, Arsenic trioxide, Asparaginase (referred to also as L-Asparaginase), Bicalutamide, Bleomycin hydrochloride, Bortezomib, Buslufan, Capecitabine, Carboplatin, Carboquone, Carmofur, Celmoleukin, Cisplatin, Cladribine, Cyclophosphamide, Cytarabine, Dacarbazine, Daunorubicin hydrochloride (referred to also as Daunomycin), Docetaxcel, Doxifluridine, Doxorubicin hydrochloride (referred to also as Adriamycin), Enocitabine, Epirubicin hydrochloride, Erlotinib hydrochloride, Estramustine phosphate sodium hydrate, Etoposide, Exemestane, Fadrozole hydrochloride hydrate, Fludarabine phosphate, Fluorouracil, Flutamide, Gefitinib, Gemcitabine hydrochloride, Goserelin acetate, Hydroxycarbamide (refered to also as Hydroxyurea), Idarubicin hydrochloride, Ifosfamide, Imatinib mesylate, Irinotecan hydrochloride, Letrozole, Leuprorelin acetate, Medroxyprogestrone acetate, Melphalan, Mepitiostane, Mercaptopurine hydrate, Methotrexate, Mitomycin C, Mitotane, Mitoxantrone hydrochloride, Nedaplatin, Nelarabine, Neocarzinostatine, Nimustine hydrochloride, Octreotide acetate, Oxaliplatin, Paclitaxel, Pemetrexed disodium, Pentostatin, Peplomycin sulfate, Pirarubicin, Porfimer sodium, Procarbazine hydrochloride, Ranimustine, Sizofiran, Sobuzoxane, Sorafenib tosylate, Talaporfin sodium, Tamibarotene, Tamoxifen citrate, Teceleukin, Tegafur, Temozolomide, Thiotepa, Topotecan hydrochloride (referred to also as Nogitecan hydrochloride), Toremifene citrate, Tretinoin, Ubenimex, Vinblastine sulfate, Vincristine sulfate, Vindesine sulfate, Vinorelbine ditartrate, and Zinostatin stimalamer.

The active substance may be any known substance which is used for treating tumor. For example, it may be a thermal neutron capturing substance, and examples thereof include compounds comprising an element such as B and Gd.

The compound according to the present invention may be pharmaceutically acceptable salts of the compound represented by formula (I). Such salts may be those obtained by binding an ionic group generated by ionization of at least one of the side residues represented by R, $R_1$, $R_2$, $R_3$, $R_4$, and/ or $R_5$ of the compound of formula (I) and an ion which is capable of binding to the ionic group through an ionic bond. Further, the ionized compound of formula (I) may bind to the ionized substance with the ratio of one molecule with respect to one molecule, that is, 1 : 1, or n : m. Here, m and n are an integer of 1 or more. For example, the salt may consist of plural molecules of the ionized compounds of formula (I) and one molecule of an ionic substance. The salt may consist of one molecule of the ionized compound of formula (I) and plural molecules of the ionic substances.

When the ionized compound of formula (I) has negative charge, the ionic substance forming a pair therewith may be a cation having positive charge. The cation may be a metal ion including monovalent metal ions such as $Na^+$ and $K^+$ and a divalent metal ion such as $Ca^{2+}$. For example, the cation may be, but not limited to, lithium, sodium, potassium, calcium, magnesium, manganese, iron, zinc, copper, strontium, lead, silver, barium, aluminum, chromium, cobalt, nickel, ammonium, monoalkylammonium, dialkylammonium, trialkylammoniumu, tetraalkylammonium, monohydroxyalkylammonium dihydroxyalkylammonium, trihydroxyalkylammonium, and tetrahydroxyalkylammonium, and the like.

Further, the compound according to the present invention may be a complex. Such a complex is a compound represented by the following formula (II):

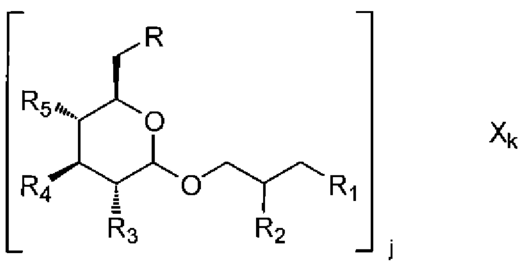

(II)

wherein R, $R_1$, $R_2$, $R_3$, $R_4$, and $R_5$ are as defined in formula (I),
j is an integer of one or more, and
X is an acting group or a cationic group, and
k is an integer of one or more,
preferably at least one of R, $R_1$, $R_2$, $R_3$, $R_4$, $R_5$, and X being an acting group,
or pharmaceutically acceptable salts thereof.

Here, the cationic group may be a cation having positive charge. The cationic group may be a metal ion including monovalent metal ions such as $Na^+$ and $K^+$ and a divalent metal ion such as $Ca^{2+}$. For example, the cationic group may be, but not limited to, lithium, sodium, potassium, calcium, magnesium, manganese, iron, zinc, copper, strontium, lead, silver, barium, aluminum, chromium, cobalt, nickel, ammonium, monoalkylammonium, dialkylammonium, trialkylammoniumu, tetraalkylammonium, monohydroxyalkylammonium, dihydroxyalkylammonium, trihydroxyalkylammonium, and tetrahydroxyalkylammonium.

When X of formula (II) contains an acting group, the acting group may be a similar active substance to those defined in formula (I), and in this case the active substance and glycolipid may bind to each other through a coordinate bond.

The compounds of formulae (I) and (II) and pharmaceutically acceptable salts thereof can be synthesized by utilizing a known reaction pathway.

Nonexclusive examples of the compound according to the present invention are described below as compounds of formulae (1)-(13).

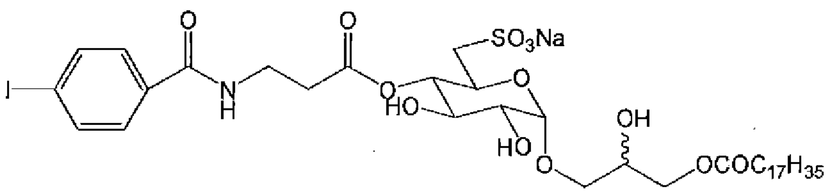

(1)

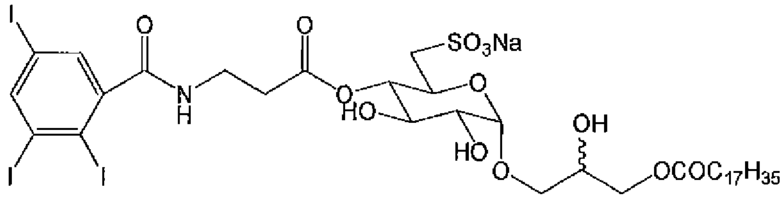

(2)

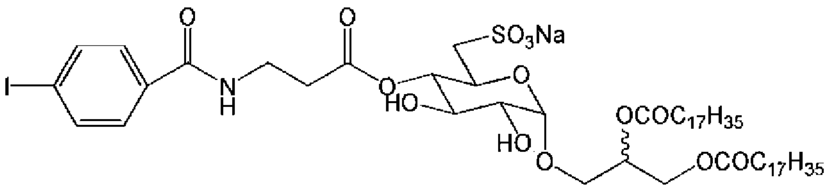

(3)

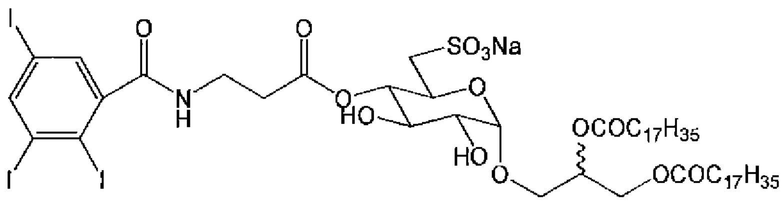

(4)

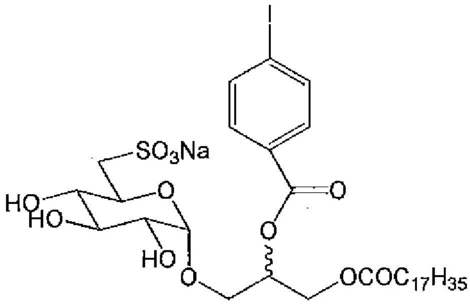

(5)

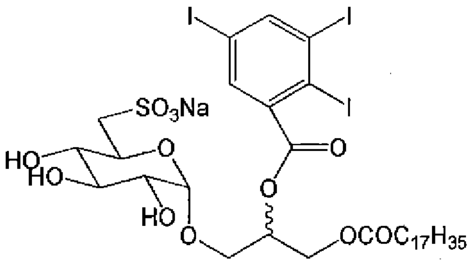

(6)

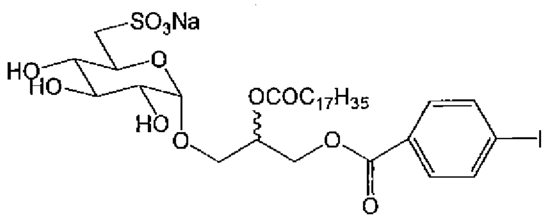

(7)

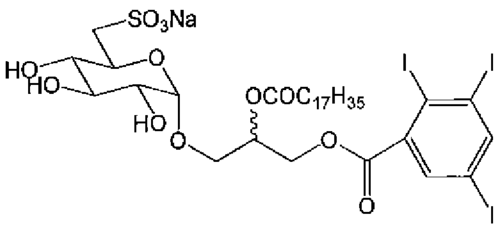

(8)

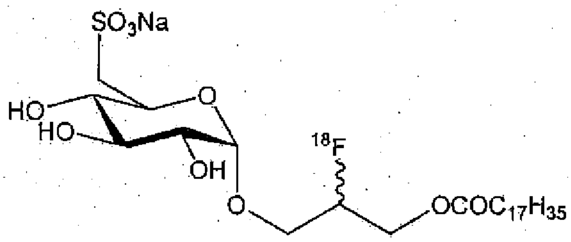

(9)

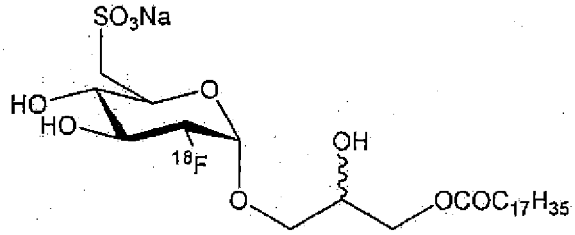

(10)

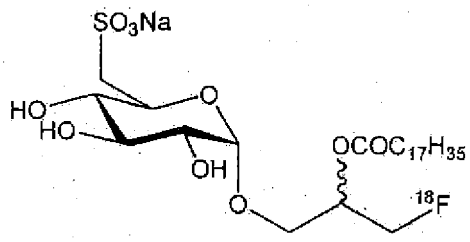

(11)

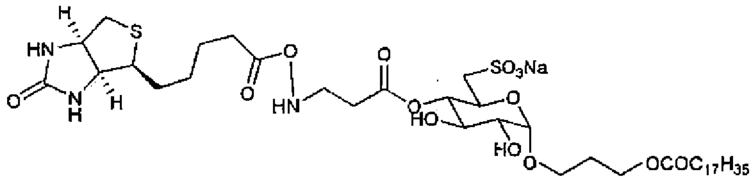

(12)

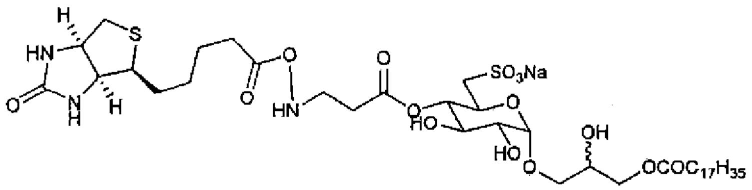

(13)

The compound according to the present invention exhibits a resident property for a long period in tumor tissues in comparison with other tissues. Namely, other tissues and tumor tissues greatly differ from each other in the distribution concentration. Therefore, administration of the compound according to the present invention, that is, the compound of formula (I), the pharmaceutically acceptable salts thereof, or the compound of formula (II) to the subject allows the compound of formula (I) or formula (II) or the salts reside therein. Thus, according to the present invention, a method for allowing the compound of formula (I), formula (II), or pharmaceutically acceptable salts thereof reside in a tumor is also provided.

As is described above, the extinction time of the compound according to the present invention in the subject differs between a tumor tissue and other tissues, and the compound resides for a longer period in a tumor in comparison with other tissues. Namely, the extinction time of the compound in a tumor is longer than that in other tissues except a tumor.

Further, the compound contains an acting group in the structure. Thus, the compound enables detection, diagnosis, and treatment of tumor by taking advantage of the effect exhibited by the tumor-resident property and the acting group of the compound. In other words, the compound according to the present invention can be utilized as diagnostic agent, therapeutic agent, and contrast agent for tumor

When tumor is detected, a labeled substance may be selected as the acting group. When the compound or the salts exist in a higher concentration in a tumor than in other tissues except a tumor after administration of the compound or the pharmaceutically acceptable salts containing a labeled substance to a subject, it is possible to detect

the labeled substance and, based on the detection results, presence or absence of tumor in the subject and/or portion and tissues having tumor.

Similarly, when a labeled substance is selected as the acting group, it is possible to diagnose presence or absence of tumor in the subject and/or to diagnose which tissue has tumor, based on detection results by detecting the labeled substance at the time when the compound or pharmaceutical acceptable salts thereof exist in a higher concentration in tumor than in other tissues except a tumor, after administering the compound or the salts containing the labeled substance.

The detection of the labeled substance may be carried out by any known method in accordance with the type of the labeled substance contained as an acting group.

For example, when the labeled substance is a radioactive substance, the labeled substance may be detected by a diagnostic imaging method such as PET and SPECT and/or a detection method using a gamma probe. In this case the compound according to the present invention may be recognized as a radioactive diagnostic agent.

When the labeled substance is a paramagnetic material, the labeled substance may be detected by a known method utilizing magnetic nuclear resonance such as MRI. In this case, the compound according to the present invention may be recognized as a nuclear magnetic resonance diagnostic agent.

When the labeled substance is a radiopaque substance, the labeled substance may be detected by either known CT or X-ray photography. In this case, the compound according to the present invention may be recognized as an X-ray contrast agent.

When the labeled substance is a fluorescent pigment, the labeled substance may be detected by a known fluorescent detection method. In this case, the compound according to the present invention may be recognized as a fluorescent diagnostic agent.

Further, the detection may be carried out by a noninvasive means, but not limited to the above-mentioned detection means. Here, noninvasive means may be those which can be carried out without surgical procedure to the subject. From the viewpoint of QOL of the subject, it is desirable to detect a labeled substance by such a noninvasive means.

Use of an antitumor substance as the active agent enables treatment of tumor. Such a method may comprise administrating the compound of formula (I) or (II) or pharmaceutically acceptable salts thereof to a subject. The compound according to the present invention which is used for such a method can be recognized as an anticancer agent or an antitumor agent.

Further, also when the substance contained as the acting group is a thermal neutron capturing substance, treatment of tumor can be carried out. Such a method may comprise administering the compound of formula (I) or (II) or pharmaceutically acceptable salts thereof and irradiating neutrons at the time when the compounds or salts exist in a higher concentration in a tumor than tissues except a tumor. The neutron irradiation can be carried out by any known means. The compound according to the present invention which is used for such a method may be recognized as a cancer treatment agent or a tumor treatment agent.

The conditions of the aforementioned radiation irradiation and administration of the compound according to the present invention, as is well-known in the field of radiation therapy, can be selected appropriately by healthcare professionals and other professionals, depending on the type of radiation source, radiation method, radiation part, and radiation time; the type of the compound, administration route, and administration timing; type and severity degree of diseases to be treated; age, body weight, health condition, and clinical record of the subject to be radiated; and the like.

The term 'subject' used herein means humans; animals other than humans, for example, mammals such as, cats, dogs, horses, cattle, sheep, mice, rats, and rabbits; reptiles; amphibians; fish; and birds.

As is described above, the compound according to the present invention can be used as an active ingredient such as a diagnostic agent, therapeutic agent, and contrast agent. The compound can be administered, for example, orally and parenterally. Further, the compound can be combined with an appropriate excipient, diluent, or the like which is pharmaceutically acceptable in accordance with the administration route thereof, thereby making a pharmaceutical preparation.

Examples of dosage forms suitable for oral administration include, but not limited to, solid, semi-solid, liquid, and gas forms, and specific examples of thereof include tablets, capsules, powders, granules, solutions, suspending agents, syrups, and elixirs.

For manufacture and formulation of the compound into tablets, capsules, powders, granules, solutions, suspending agents, and the like, the compound may be mixed to a binder, tablet disintegrant, lubricant agent, and the like, and further, according to need, mixed to diluents, buffers, wetting agents, preservation agents, flavoring agents, and the like with use of a known method. For example, the binder includes crystalline cellulose, cellulose derivatives, cornstarch, and gelatin. The tablet disintegrant includes, for example, cornstarch, potato starch, and carboxymethylcellulose sodium. The lubricant agent includes, for example, talc, and magnesium stearate. Conventionally-used additives such as lactose and mannitol may be further used.

Further, the compound in the form of liquid or minute powder may be filled in a non-pressurized container such

as an aerosol container and nebulizer together with gaseous or liquid air spray or, according to need, together with a known auxiliary agent such as a moistening agent, to administer it in the form of an aerosol agent or inhalant. As the air spray, pressurized gas such as dichlorofluoromethane, propane, and nitrogen may be used.

When a pharmaceutical preparation containing the compound as an active ingredient is parenterally administered, it may be given by, for example, rectal administration, or injection.

When it is given by rectal administration, it may be administered as a suppository. With respect to the suppository, the pharmaceutically active substance can be mixed to an excipient such as cacao butter, carbon wax, and polyethylene glycol which are melted at a body temperature but are solidified at room temperature and are formed by a known method, thereby making a preparation.

Administration by injection may be conducted through, for example, hypodermic, intradermal, intravenous, or intramuscular injection. With respect to these preparations for injection, the compound of the present invention is dissolved, suspended, or emulsified in an aqueous or non-aqueous solvent such as plant oil, synthetic resin acid glyceride, higher fatty acid esters, and propylene glycol, thereby making a preparation, if desired, together with a conventionally used additive such as a solubilizing agent, osmoregulatory agent, emulsifying agent, stabilizing agent, and preservative.

In order to make the compound according to the present invention into a form such as solution, suspension, syrup, and elixir, a pharmaceutically acceptable solvent such as injectable sterilized water and normal saline solution may be used.

The compound according to the present invention may be also combined with a pharmaceutically acceptable compound having other activity, thereby making a pharmaceutical preparation.

The pharmaceutical preparation according to the present invention may be appropriately established and adjusted according to the dosage form, administration route, tumor type and location to be detected, and degree and stage of diseases to be treated. For example, the effective dose of the compound according to the present invention may be, but not limited to, from 0.01 to 1000 mg/kg body weight per day via oral administration, from 0.01 to 500 mg/kg body weight per day via injection, and from 0.01 to 500 mg/kg body weight per day via rectal administration.

The compound according to the present invention can be used for treating tumor. Examples of tumor include, but not limited to: neurogenic tumor such as cerebral tumorquamous cell carcinoma and adenocarcinoma such as head and neck cancer, skin cancer, esophagus cancer, thyroid cancer, stomach cancer, lung cancer, gellbladder cancer, biliary tract cancer, pancreas cancer, liver cancer, prostate cancer, uterus cancer, ovarian cancer, breast cancer, kidney cancer, bladder cancer, and colon cancer; and melanoma, osteoma, soft tissue tumor, and lymphoma, leukemia, and myeloma. The term "treatment" used herein refers to the reduction, destruction, and/or inhibition of enhancement of the above-described tumor.

The pharmaceutical preparation according to the present invention may contain, as an active ingredient, an effective dose of at least one or more selected from the group consisting of the compounds represented by formula (I) and formula (II) and pharmaceutically acceptable salts thereof. The pharmaceutical preparation may contain plural kinds of different compounds among the compounds according to the present invention. In addition, the compound according to the present invention may be combined with other radiosensitizer, and antitumor agent, or other substances having pharmacological activity and/or pharmaceutical activity without affecting its activity.

The sulfoquionovosylacyl propanediol, an example of glycolipid portion contained in the compound according to the present invention, may be hereinafter referred to as "SQAP." In a term "αSQAP Cp:q", "α" represents an α anomer, and "Cp:q" represents that the number of carbon atoms contained in the $R_1$ group of SQAP is "p", and the number of double bonds is "q", wherein "p" is an integer of 1 or more, and "q" is an integer of 0 or more. Accordingly, for example, "αSQAP C18:0" represents an α anomer of sulfoquinovosylacyl propanediol wherein the number of carbon atoms contained in $R_1$ of the compound is 18, and the number of double bonds is 0.

Further, the sulfoquionovosylacylglycerol, an example of glycolipid portion contained in the compound according to the present invention, may be hereinafter referred to as "SQMG." In a term "αSQMG Cr:s", "α" represents an α anomer, and "Cr:s" represents that the number of carbon atoms contained in the $R_1$ group of SQMG is "r", and the number of double bonds is "s", wherein "r" is an integer of 1 or more, and "s" is an integer of 0 or more. Accordingly, for example, "αSQMG C18:0" represents an α anomer of sulfoquinovosylacylglycerol wherein the number of carbon atoms contained in $R_1$ of the compound is 18, and the number of double bonds is 0.

## EXAMPLES

### Example 1. Synthesis examples

#### Synthesis Example 1

The steps for preparing the sulfopyranosyl glycerol derivative according to the present invention is described in the following scheme 1 taking, as an example, α-sulfoquinovosylacylgrlycerol biotin derivative, which may be referred

to as biotinated αSQMG hereinafter.

[Scheme 1]

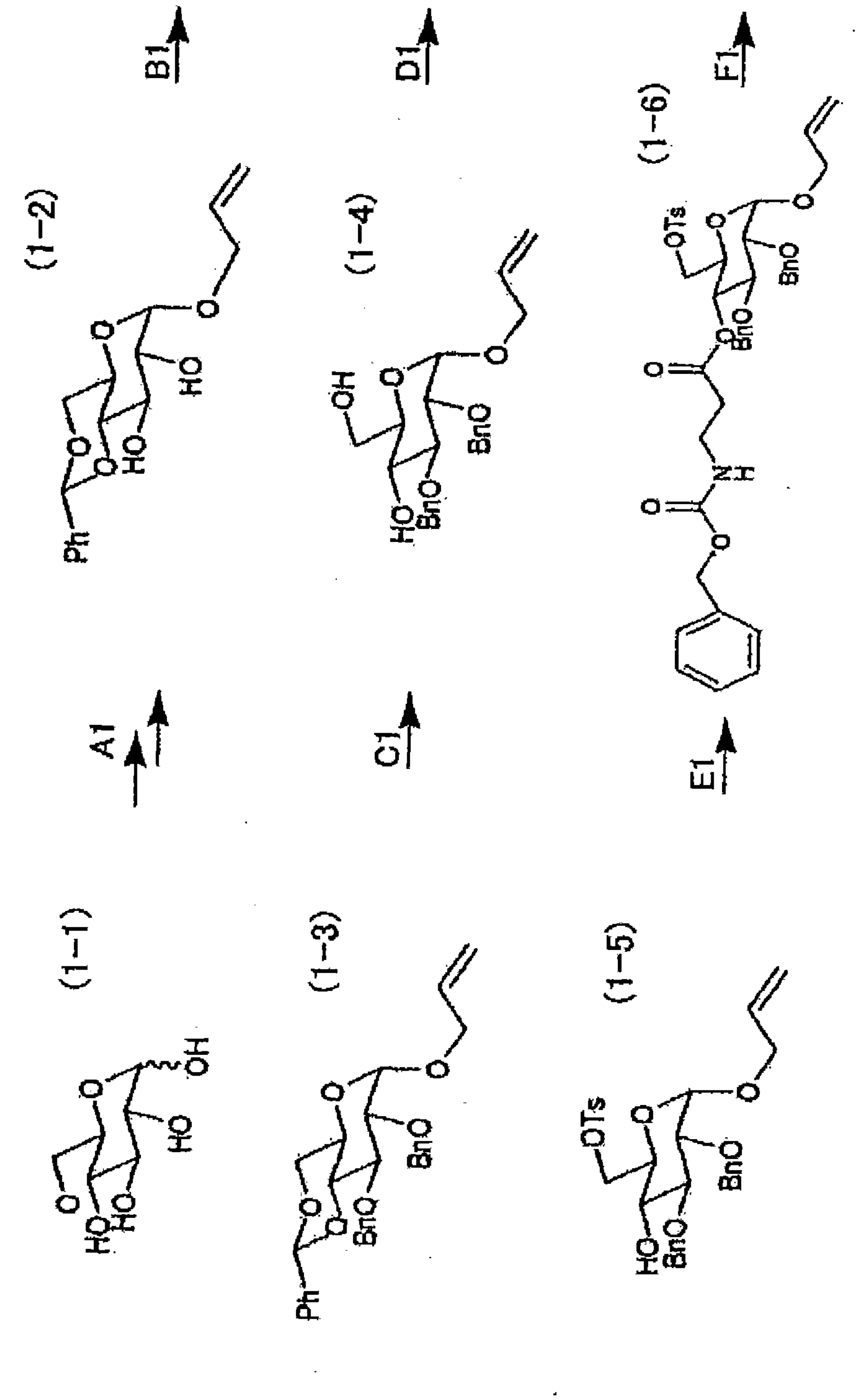


(1-1) (1-2) (1-3) (1-4) (1-5) (1-6)

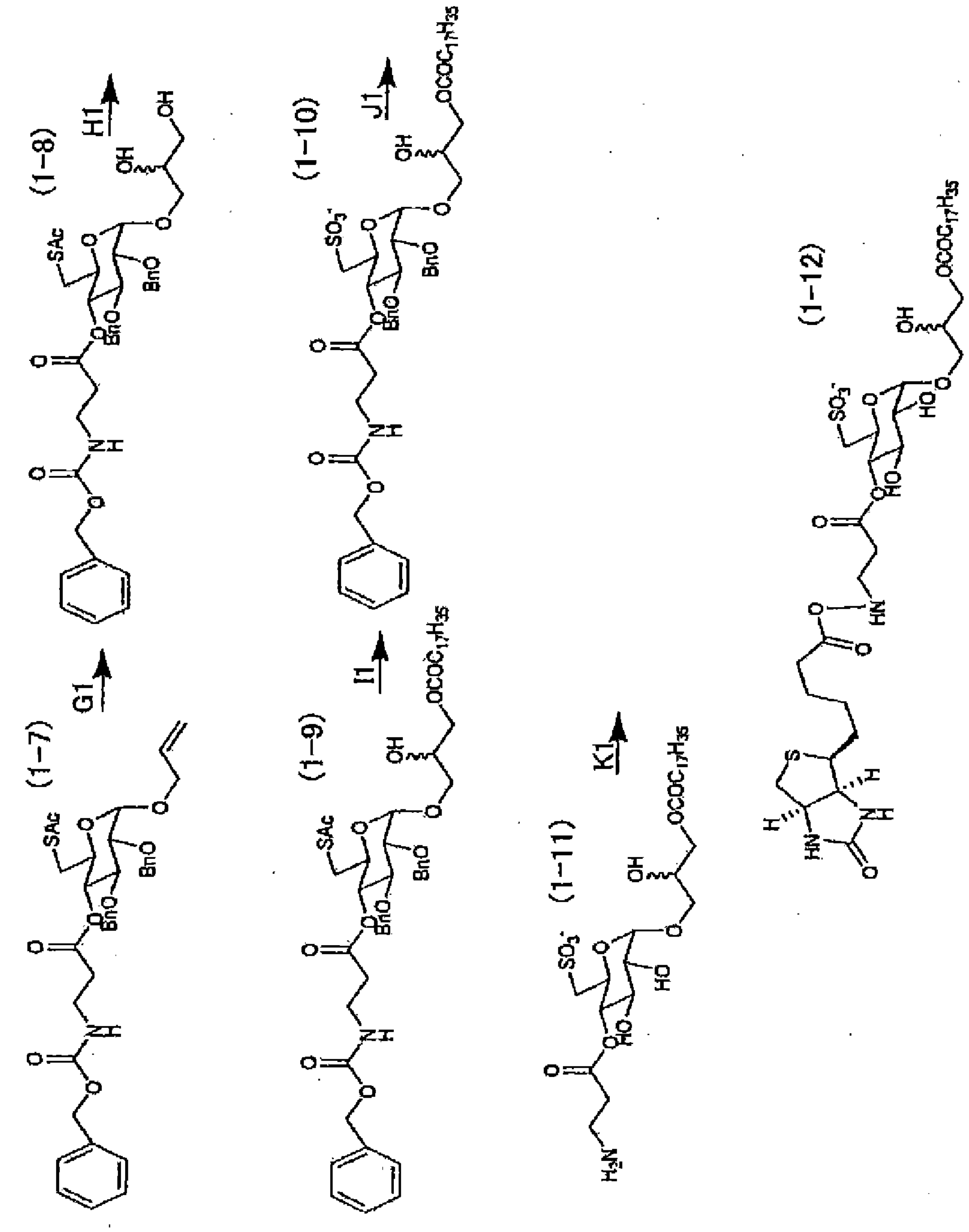


Each of the steps is described below in detail.

Route A1: 1-O-allyl-4,6-O-benzylidene-α-D-glucopyranoside (1-2)

The compound (1-1) (100 g, 555 mmol) was suspended in allyl alcohol (500 mL), to which trifluoromethanesulfonic acid (1.00 mL) was added at 0°C, and the reaction liquid was vigorously stirred at 80°C for 48 hours. After the sufficient progress of the reaction was confirmed, triethylamine (3 mL) was added to stop the reaction, and the reaction liquid was concentrated under reduced pressure. Subsequently, the residue was suspended in anhydrous acetonitrile (500 mL), to which benzaldehyde dimethyl acetal (127 g, 1.5 equivalents) and p-toluenesulfonic acid monohydrate (5.28 g, 0.05 equivalent) were added. The reaction liquid was stirred at 40°C for 4 hours, to which triethylamine (10 mL) was added to stop the reaction, and the reaction liquid was concentrated under reduced pressure. The residue was poured

to hexane (2000 mL) and water (500 mL), and the mixed liquid was vigorously stirred. The generated precipitate was collected by filtration, and rinsed with water and hexane. The precipitate was crystallized from heated ethanol twice to obtain the title compound (1-2) in the form of colorless needle crystals {34.5 g (112 mmol), 20.2% yield}.
LRMS 331 m/Z $(M+Na)^+$.

Route B1; 1-O-allyl-2,3-di-O-benzyl-4,6-O-benzylidene-α-D-glucopyranoside (1-3)

To a solution of the compound (1-2) (30.0 g, 97.3 mmol) in anhydrous N,N-dimethylformamide (DMF, 300 mL), added were benzyl bromide (41.6 g, 2.5 equivalents) and sodium hydroxide (11.7 g, 3.0 equivalents), and the reaction liquid was vigorously stirred at room temperature for 24 hours. After the sufficient progress of the reaction was confirmed, the reaction liquid was poured to chilled water (900 mL), and extracted with ethyl acetate (3 × 300 mL). The organic layers were combined and washed with saturated saline (2 × 100 mL), dried with sodium sulfate, filtered, and concentrated under reduced pressure. The obtained residue was crystallized from heated ethanol twice to obtain the title compound (1-3) in the form of colorless needle crystals (33.5 g). The filtrate was concentrated, purified with silica gel chromatography (hexane-ethyl acetate, 15:1 → 10:1 → 8:1), crystallized from heated ethanol to obtain the compound (1-3) (6.63 g) {40.1 g (82.1 mmol) in total, 84.4% yield} LRMS 511 m/Z $(M+Na)^+$.

Route C1; 1-O-allyl-2,3-di-O-benzyl-α-D-glucopyranoside (1-4)

The compound (1-3) (15.6 g, 32.0 mmol) was dissolved in acetic acid (90 mL). Subsequently, distilled water (50 mL) was further added to the solution, and the mixture was stirred for 1 hour under heating and reflux. After the sufficient progress of the reaction was confirmed, the reaction liquid cooled to at room temperature, and the solvent was removed by evaporation. Distilled water (15 mL) was then added to the solution, and further concentrated under reduced pressure four times. The concentrate was purified with silica gel flash chromatography (hexane-ethyl acetate, 4:1 → 2: 1 → 1:1 → 1:2) to obtain the title compound (1-4) {12.1 g (30.2 mmol), 94.5% yield}.
LRMS 423 m/Z $(M+Na)^+$.

Route D1 D;1-O-allyl-2,3,4-di-O-benzyl-6-O-tosyl-α-D-glucopyranoside (1-5)

To a solution of the compound (1-4) (12.1 g, 30.2 mmol) in anhydrous pyridine (120 mL), added were p-toluenesulfonyl chloride (7.5 g, 39.3 mmol) and 4-dimethylaminopyridine (369 mg, 3.0 mmol), and the reaction liquid was stirred at 0°C for 16 hours. After the sufficient progress of the reaction was confirmed the reaction liquid was then poured slowly into iced water (100 mL), and the aqueous layers were extracted with ethyl acetate (3 × 200 mL). The organic layers were combined, washed with 1N HCl solution until pH becomes 4, washed with a saturated sodium hydrogen carbonate solution (2 × 100 mL) and saturated saline (2 × 100 mL), dried with sodium sulfate, filtered, and concentrated under reduced pressure. The concentrate was purified with silica gel flash chromatography (hexane-ethyl acetate, 6:1 → 4:1 → 2:1) to obtain the title compound (1-5).
{15.3 g (27.6 mmol), 91.4% yield}.
LRMS 577 m/Z $(M+Na)^+$.

Route E1; 1-O-allyl-2,3-di-O-benzyl-4-O-(carbobenzoxy-β-alanyl)-6-O-tosyl-α-D-glucopyranoside (1-6)

The compound (1-5) (15.3 g, 27.6 mmol), N-carbobenzoxy-β-alanine(12.32g, 55.2mmol), 1-methyl-3-(3-dimethylaminopropyl)carbodiimide hydrochloride salt (EDCI•HCl) (15.8 mg, 82.8 mmol), and 4-dimethylaminopyridine (6.7 g, 55.2 mmol) were dissolved in the mixed solution of anhydrous dichloromethane (200 mL) and anhydrous pyridine (50 mL) and reacted at room temperature for 18 hours. After the sufficient progress of the reaction was confirmed, water (10 mL) was poured into the reaction liquid to stop the reaction, and the solution was concentrated under reduced pressure. The obtained residue was purified with silica gel chromatography (hexane-ethyl acetate, 4:1 → 3:1 → 2:1 → 3:2) to obtain the title compound (1-6) [17.1 g (22.5 mmol), 81.5% yield].
LRMS 782 m/z $(M+Na)^+$.

Route F1; 1-O-allyl-2,3-di-O-benzyl-4-O-(carbobenzoxy-β-alanyl)-6-O-thioacetyl-α-D-glucopyranoside (1-7)

To a solution of the compound (1-6) (17.1 g, 22.5 mmol) in anhydrous N,N-dimethylforamide (300 mL), added was potassium thioacetate (5.1 g, 45.0 mmol), and the mixture was stirred at 90°C for 3 hours. After the sufficient progress of the reaction was confirmed, the reaction liquid was poured to chilled water (400 mL), and extracted with ethyl acetate (3 × 150 mL). The organic layers were combined and washed with saturated saline (2 × 100 mL), dried with sodium sulfate, filtered, and concentrated under reduced pressure. The obtained residue was purified with silica

gel chromatography (hexane-ethyl acetate, 4:1 → 3:1 → 2:1→) 3:2) to obtain the title compound (1-7) {14.2 g (20.3 mmol), 90.0% yield}.

LRMS 686 m/Z $(M+Na)^+$.

Route G1; 3-O-[2,3-di-O-benzyl-4-O-(carbobenzoxy-β-alanyl)-6-thioacetyl-α-D-quinovopyranosyl]-glycerol (1-8)

The compound (1-7) (14.2 g, 20.4 mmol) was dissolved in t-butylalcohol : distilled water = 4 : 1 solution (200 mL). To the solution, added were 0.04 M osmium tetraoxide-t-butyl alcohol (3 mL) and trimethylamine N-oxide (3.4 g, 30.5 mmol), stirred with a stirrer at room temperature for 24 hours. After the sufficient progress of the reaction was confirmed, 3 g of actived charcoal was added and stirred for 30 minutes to adsorb the catalyst, and the catalyst was removed by suction filtration through celite in Kiriyama funnel, followed by three times of washing of the remained reaction product on the celite with ethyl acetate and collecting thereof. Distilled water (200 mL) was added to the collected filtrate and extracted with ethyl acetate (3 × 150 mL). The organic layers were combined and washed with saturated saline (2 × 100 mL), dried with sodium sulfate, filtered, and concentrated under reduced pressure. The obtained residue was purified with silica gel chromatography (hexane-ethyl acetate, 2:1 → 1:1 → 2:3 → 1:2 → 1:4 → 1:8) to obtain the title compound (1-8) {13.2 g (18.9 mmol), 93.0% yield}.
LRMS 720 m/Z $(M+Na)^+$.

Route H1; 3-O-[2,3-di-O-benzyl-4-O-(carbobenzoxy-β-alanyl)-6-thioacetyl-α-D-quinovopyranosyl]-1-0-stearoyl-glycerol (1-9)

To a mixed solution of anhydrous dichloromethane (200 mL) and anhydrous pyridine (50 mL) of the compound (1-8) (13.1 g, 18.8 mmol), added were stearoylchloride (8.5 g, 28.2 mmol), and the mixture was stirred at room temperature for 2 hours. After the sufficient progress of the reaction was confirmed, methanol (5 mL) was added to stop the reaction, and concentrated under reduced pressure. The residue was suspended in a minor amount of ethyl acetate, poured to water (200 mL), and extracted with ethyl acetate (3 × 100 mL). The organic layers were combined, washed with saturated saline (2 × 100 mL), dried with sodium sulfate, filtered, and concentrated under reduced pressure. The obtained residue was purified with silica gel chromatography (hexane-ethyl acetate, 6:1 → 4:1 → 2:1 → 3:2 → 1:1) to obtain the title compound (1-9) in the form of a colorless oily substance {7.1 g (7.4 mmol), 39.4% yield}.

LRMS 987 m/Z $(M+Na)^+$.

Route I1; 3-O-[2,3-di-O-benzyl-4-O-(carbobenzoxy-β-alanyl)-5-sulfo-α-D-quinovopyranosyl]-1-0-stearoyl-glycerol (1-10)

To a solution of the compound (1-9) (7.1 g, 7.4 mmol) in acetic acid (160 g, 4 mol), added were Oxone (18.1 g, 29.5 mmol) and potassium acetate (4.0 g), and the mixture was vigorously stirred at room temperature for 48 hours. After the sufficient progress of the reaction was confirmed, the reaction liquid was poured to a chilled 7.5 M sodium hydroxide solution (500 mL), and extracted with ethyl acetate (4 × 100 mL). The organic layers were combined, washed with saturated sodium hydrogen carbonate solution (2 × 100 mL) and saturated saline solution (2 × 100 mL), dried with sodium sulfate, filtered, and concentrated under reduced pressure. The obtained residue was purified with silica gel chromatography (chloroform-methanol, 100:1 → 50:1 → 20:1 → 15:1 → 12:1) to obtain the title compound (1-10) in the form of a colorless waxy substance {5.2 g (5.24 mmol), 71.2% yield}.
LRMS 968 m/Z $(M-Na)^-$.

Route J1; 3-O-[4-O-(β-alanyl)-6-sulfo-α-D-quinovopyranosyl]-1-0-stearoyl-glycerol (1-11)

To a solution of the compound (1-10) (490 mg, 494 μmol) in methanol (20 mL) and acetic acid (0.6 mL), added was 10% palladium-activated carbon (300 mg), and the mixture was stirred at room temperature for 48 hours in a hydrogen gas atmosphere. After the sufficient progress of the reaction was confirmed, palladium-activated carbon was filtrated off, and the filtrate was concentrated under reduced pressure. The obtained residue was purified with silica gel chromatography (chloroform-methanol-distilled water, 100:25:3 → 65:25:4 → 65:35:5 → 65:45:6) to obtain the title compound (1-11) {221 mg (327 μmol), 66.1% yield}.
LRMS 654 m/Z $(M-Na)^-$.

Route K1; 3-O-[4-O-(biotinyl-β-alanyl)-6-sulfo-α-D-quinovopyranosyl]-1-0-stearoyl-glycerol (1-12)

The compound (1-11) (273.2 mg, 403 μmol) was dissolved in a mixture solution of anhydrous N,N-dimethylformamide (20 mL) and triethylamine (1 mL), and biotion-p-nitrophenylester (162 mg, 443 μmol) was added and reacted

at room temperature for 24 hours while stirring with a stirrer. After the sufficient progress of the reaction was confirmed, toluene and methanol were added and the solvent was distilled away and concentrated under reduced pressure while azeotropy. The obtained residue was purified with silica gel chromatography (chloroform-methanol-distilled water, 40: 10:1.2 → 70:30:2.6 → 30:10:1.5 → 65:25:4) to obtain the title compound (1-12) {320.8 mg (355 μmol), 88.0% yield}.

LRMS 880 m/Z $(M-Na)^-$.

Synthesis Example 2

A process for producing the sulfopyranosylacyl propanediol derivative according to an embodiment of the present invention is illustrated in the following scheme 2, giving an α-sulfoquinovosylacyl propanediol biotin derivative, which may be referred to as biotinated αSQAP hereinafter, as an example thereof:

[Scheme 2]

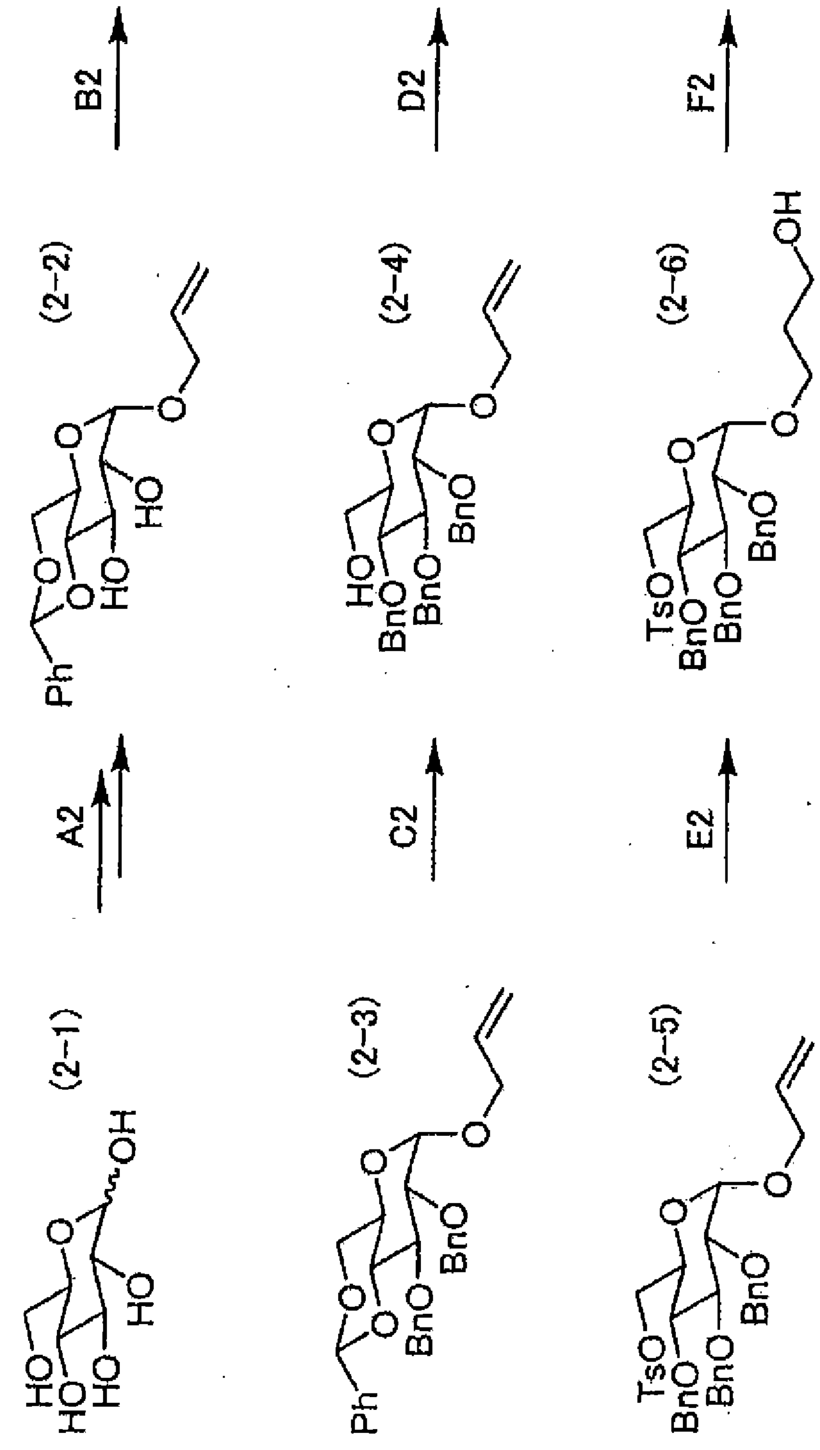

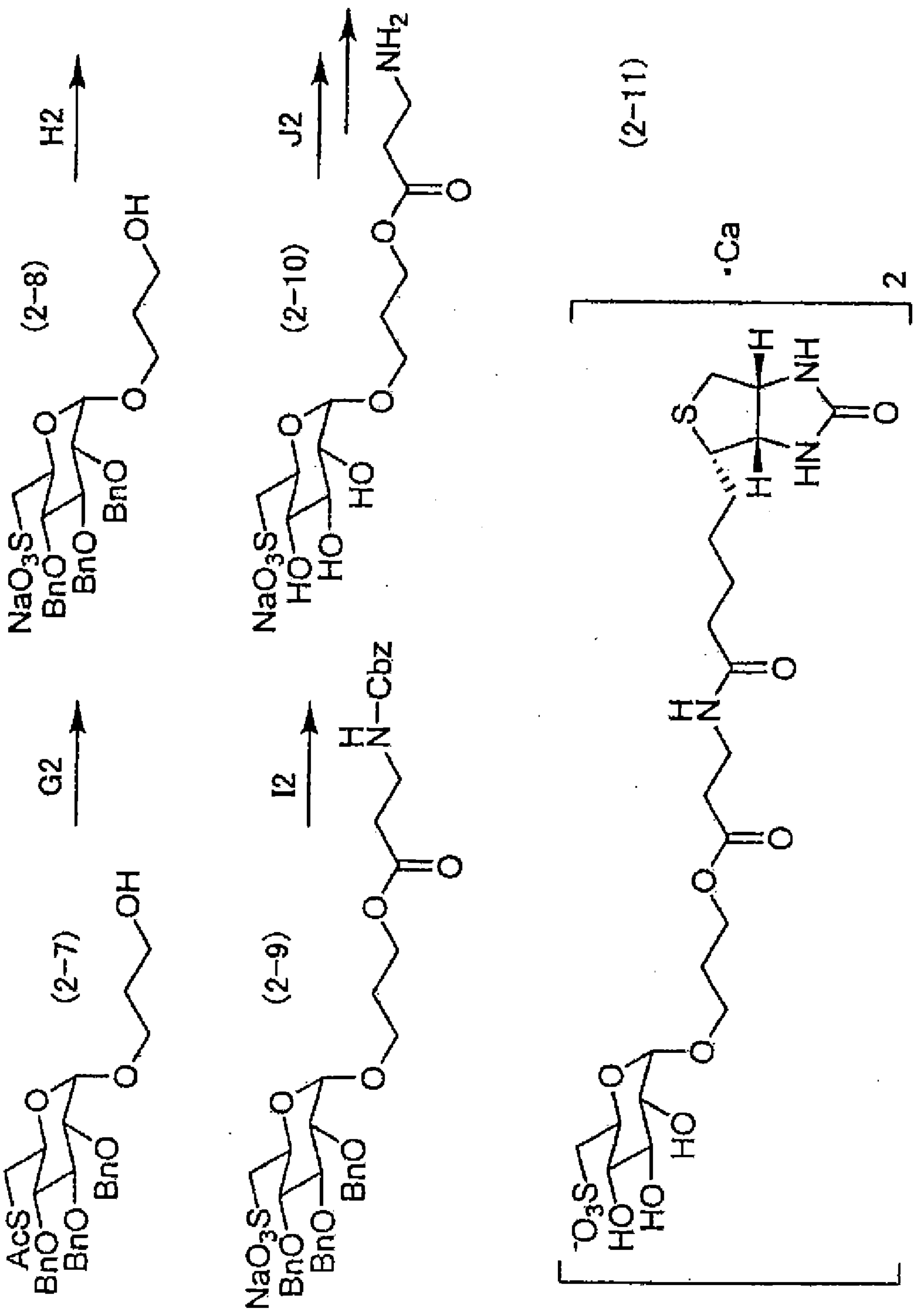


(2-7) (2-8) (2-9) (2-10) (2-11)

The following will describe a synthesis example through individual steps in detail:

<Synthesis Example>

Route A2; 1-O-allyl-4,6-O-benzylidene-α-D-glucopyranoside (2-2):

Trifluoromethanesulfonic acid (1.00 mL) was added to a liquid suspension of D-glucose (2-1) (100 g, 555 mmol) in allyl alcohol (500 mL) at 0°C, and the reaction liquid was vigorously stirred at 80°C for 48 hours. After the sufficient progress of the reaction was confirmed, thereto was added triethylamine (3 mL) to terminate the reaction. The resultant was concentrated under reduced pressure. Next, the residue was suspended in anhydrous acetonitrile (500 mL), and thereto were added benzaldehydedimethylacetal (127 g, 1.5 equivalent) and p-toluenesulfonic acid monohydrate (5.28 g, 0.05 equivalent). The reaction liquid was stirred at 40°C for 4 hours, and then thereto was added triethylamine (10 mL) to terminate the reaction. The resultant was concentrated under reduced pressure. The residue was poured into hexane (200 mL) and water (500 mL), and then the mixed solution was vigorously stirred. The resultant precipitate was filtrated, and rinsed with water and hexane. The precipitate was crystallized two times from heated ethanol to yield the title compound (2-2) as colorless needle crystals {34.5 g (112 mmol), 20.2% yield}. $[a]^{23}_D$ +97.5° (c1.00 $CH_3OH$), LRMS m/z 331 $[M + Na]^+$, mp 139-141°C
$^1H$ NMR (400MHz, $CD_3OD$); δ 7.51-7.47 (m, 2H, ArH), 7.37-7.32 (m, 3H, ArH), 5.99 (dddd, 1H, J=17.2, 10.5, 6.08, 5.32Hz, H2), 5.56 (s, 1H, PhCH), 5.36 (dq, 1H, J=17.3, 1.68Hz, H3a), 5.20 (ddt, 1H, J=10.4, 1.80, 1.28Hz, H3b), 4.88 (d, 1H, J=3.86Hz, H1'), 4.25-4.18 (m, 2H, H1a & H6'a), 4.07 (ddt, 1H, J=13.0, 6.10, 1.36Hz, H1b), 3.85 (t, 1H, J=9.38Hz, H3'), 3.81-3.71 (m, 2H, H5' & H6'b), 3.52 (dd, 1H, J=9.38, 3.86Hz, H2'), 3.45 (t, 1H, J=9.24Hz, H4')
$^{13}C$ NMR (100MHz, $CD_3OD$); δ 139.1 (Ar-ipso), 135.4 (C2), 129.9 (Ar), 129.0 (Ar), 127.5 (Ar), 117.8 (C3), 103.0 (PhCH), 100.0 (C1'), 82.9 (C4'), 74.0 (C2'), 72.0 (C3'), 69.9 (C6'), 69.7 (C1), 64.1 (C5).

Route B2; 1-O-allyl-2,3-di-O-benzyl-4,6-O-benzylidene-α-D-glucopyranoside (2-3):

To a solution of the compound (2-2) (30.0 g, 97.3 mmol) in anhydrous N,N-dimethylformamide (DMF, 300 mL) were added benzylbromide (41.6 g, 2.5 equivalent) and sodium hydroxide (11.7 g, 3.0 equivalent), and the reaction solution was vigorously stirred at room temperature for 24 hours. After the sufficient progress of the reaction was confirmed, the reaction solution was poured into cold water (900 mL), and the resultant was extracted with ethyl acetate (3 × 300 mL). The organic layers were combined with each other, and the combination was washed with saturated saline (2 × 100 mL), dried over sodium sulfate, filtrated and then concentrated under reduced pressure. The resultant residue was crystallized two times from heated ethanol to yield the title compound (2-3) as a colorless needle crystal (33.5 g). The filtrate was concentrated, purified by silica gel chromatography (hexane/ethyl acetate, 15:1→10:1→8:1), and then crystallized from heated ethanol to yield the same compound (2-3) (6.63 g) {total amount: 40.1 g (82.1 mmol), 84.4% yield}. $[\alpha]^{26}_D$ -1.46°(c1.03 $CHCl_3$), LRMS m/z 511 $[M + Na]^+$, mp 86-87°C
$^1H$ NMR (400MHz, $CDCl_3$); δ 7.50-7.47 (m, 2H, ArH), 7.40-7.24 (m, 13H, ArH), 5.94 (dddd, 1H, J=17.0, 10.4, 6.70, 5.24Hz, H2), 5.56 (s, 1H, PhCH), 5.33 (dq, 1H, J=17.2, 1.56Hz, H3a), 5.24 (ddt, 1H, J=10.3, 1.56, 1.12Hz, H3b), 4.92 (d, 1H, J=11.2Hz, $ArCH_2$), 4.84 (d, 1H, J=11.2Hz, $ArCH_2$), 4.83 (d, 1H, J=12.1Hz, $ArCH_2$), 4.80 (d, 1H, J=3.76Hz, H1'), 4.68 (d, 1H, J=12.1Hz, $ArCH_2$), 4.26 (dd, 1H, J=10.2, 4.84Hz, H6'a), 4.18 (ddt, 1H, J=12.9, 5.18, 1.40Hz, H1a), 4.79 (t, 1H, J=9.30Hz, H3'), 4.03 (ddt, 1H, J=12.9, 6.68, 1.20Hz, H1b), 3.89 (dt, 1H, J=9.96, 4.80Hz, H5'), 3.70 (t, 1H, J=10.3Hz, H6'b), 3.61 (t, 1H, J=9.44Hz, H4'), 3.57 (dd, 1H, J=8.72, 3.80Hz, H2')
$^{13}C$ NMR (100MHz, $CDCl_3$); δ 138.7 (Ar-ipso), 138.1 (Ar-ipso), 137.3 (Ar-ipso), 133.5 (C2), 128.9-127.5 (m, Ar), 126.0 (Ar), 118.4 (C3), 101.2 (PhCH), 96.7 (C1'), 82.1 (C3'), 79.1 (C2'), 78.6 (C4'), 75.3 ($ArCH_2$), 73.6 ($ArCH_2$), 69.0 (C6'), 68.4 (C1), 62.5 (C5').

Route C2; 1-O-ally-2,3,4-tri-O-benzyl-α-D-glucopyranoside (2-4):

The compound (2-3) (20.0 g, 40.9 mmol) was added to a mixed solution of anhydrous dichloromethane (100 mL) and anhydrous diethyl ether (100 mL) wherein lithium aluminum hydride (2.02 g, 1.3 equivalent) was suspended. Next, to the reaction solution was added 200 mL of a solution of aluminum chloride (7.09 g, 1.03 equivalent) in anhydrous diethyl ether, and then the solution was stirred for 4 hours while heated and refluxed. After the sufficient progress of the reaction was confirmed, water (10 mL) was slowly added thereto, dropwise. After one night, the precipitate was filtrated, and rinsed with diethyl ether. The filtrate was washed with water (2 × 100 mL), and then the water layers were combined with each other. The combination was extracted with diethyl ether (2 × 100 mL). The organic layers were combined with each other, and the combination was washed with saturated saline (2 × 200 mL), dried over sodium sulfate, filtrated, and concentrated under reduced pressure. The resultant residue was purified by silica gel chromatography (hexane/ethyl acetate, 5:1→4:1→3:1→2:1) to yield the title compound (2-4) as a colorless oily substance {18.1 g (36.9 mmol), 90.2% yield}. $[a]^{22}_D$ +45.0° (c1.21 $CHCl_3$), LRMS m/z 513 $[M + Na]^+$
$^1H$ NMR (400MHz, $CDCl_3$); δ 7.37-7.26 (m, 15H, ArH), 5.92 (dddd, 1H, J=17.1, 10.4, 6.66, 5.24Hz, H2), 5.31 (dq, 1H,

J=17.2, 1.52Hz, H3a), 5.22 (ddt, 1H, J=10.3, 1.46, 1.10Hz, H3b), 5.00 (d, 1H, J=10.9Hz, $ArCH_2$), 4.89 (d, 1H, J=11.0Hz, $ArCH_2$), 4.84 (d, 1H, J=10.9Hz, $ArCH_2$), 4.77 (d, 1H, J=12.0Hz, $ArCH_2$), 4.77 (d, 1H, J=3.60Hz, H1'), 4.65 (d, 1H, J=12.1Hz, $ArCH_2$), 4.64 (d, 1H, J=11.0Hz, $ArCH_2$), 4.14 (ddt, 1H, J=12.9, 5.22, 1.34Hz, H1a), 4.04 (t, 1H, J=9.36Hz, H3'), 3.99 (ddt, 1H, J=12.9, 6.64, 1.08Hz, H1b), 3.79-3.66 (m, 3H, H5' & H6'a & H6'b), 3.54 (t, 1H, J=9.28Hz, H4'), 3.51 (dd, 1H, J=9.60, 3.64Hz, H2'), 1.69 (t, 1H, J=12.0Hz, 6'-OH)
$^{13}C$ NMR (100MHz, $CDCl_3$); δ 138.7 (Ar-ipso), 138.1 (Ar-ipso), 138.1 (Ar-ipso), 133.6 (C2), 128.4-127.6 (m, Ar), 118.3 (C3), 95.6 (C1'), 81.9 (C3'), 79.9 (C2'), 77.3 (C4'), 75.7 ($ArCH_2$), 75.0 ($ArCH_2$), 73.2 ($ArCH_2$), 70.8 (C5'), 68.2 (C1), 61.7 (C6').

Route D2; 1-O-ally-2,3,9-tri-O-benzyl-6-O-tosyl-α-D-glucopyranoside (2-5):

To a solution of the compound (2-4) (25.1 g, 51.2 mmol) in anhydrous pyridine (250 mL) were added p-toluenesulfonyl chloride (14.6 g, 1.5 equivalent) and 4-dimethylaminopyridine (626 mg, 0.1 equivalent), and the reaction solution was stirred at room temperature for 16 hours. After the sufficient progress of the reaction was confirmed, water was added thereto so as to terminate the reaction. The reaction solution was concentrated under reduced pressure. A small amount of ethyl acetate was used to suspend the residue, and the suspended residue was poured into 0.5 M hydrochloric acid (200 mL). The resultant was extracted with ethyl acetate (3 × 200 mL). The organic layers were combined with each other, and the combination was washed with an aqueous saturated sodium hydrogen carbonate solution (2 × 100 mL) and saturated saline (2 × 100 mL), dried over sodium sulfate, filtrated and then concentrated under reduced pressure. The resultant residue was crystallized two times from heated ethanol to yield the title compound (2-5) as a colorless needle crystal (25.0 g). The filtrate was concentrated and purified by silica gel chromatography (hexane/ethyl acetate, 5:1→4:1→3:1) to yield the same compound (2-5) (4.00 g) {total amount: 29.0 g (45.0 mmol), 87.9% yield}. $[a]^{25}_D$ +32.1°(c1.02 $CHCl_3$), LRMS m/z 667 $[M+Na]^+$, mp 86-87°C
$^1H$ NMR (400MHz, $CDCl_3$); δ 7.76 (ddd, 2H, J=8.32, 1.96, 1.76Hz, ArH), 7.35-7.26 (m, 15H, ArH), 7.17-7.12 (m, 2H, ArH), 5.88 (dddd, 1H, J=17.2, 10.3, 6.62, 5.24Hz, H2), 5.28 (dq, 1H, J=17.2, 1.56Hz, H3a), 5.20 (ddt, 1H, J=10.3, 1.60, 1.12Hz, H3b), 4.99 (d, 1H, J=10.9Hz, $ArCH_2$), 4.82 (d, 1H, J=10.6Hz, $ArCH_2$), 4.78 (d, 1H, J=10.8Hz, $ArCH_2$), 4.74 (d, 1H, J=12.1Hz, $ArCH_2$), 4.72 (d, 1H, J=3.58Hz, H1'), 4.62 (d, 1H, J=12.1Hz, $ArCH_2$), 4.42 (d, 1H, J=10.6Hz, $ArCH_2$), 4.22 (dd, 1H, J=10.5, 4.20Hz, H6'a), 4.16 (dd, 1H, J=10.5, 2.12Hz, H6'b), 4.07 (ddt, 1H, J=12.9, 5.24, 1.40Hz, H1a), 3.98 (t, 1H, J=9.24Hz, H3'), 3.93 (ddt, 1H, J=12.9, 6.64, 1.16Hz, H1b), 3.81 (ddd, 1H, J=10.1, 4.12, 2.04Hz, H5'), 3.48 (dd, 1H, J=9.62, 3.58Hz, H2'), 3.45 (dd, 1H, J=10.0, 8.90Hz, H4'), 2.39 (s, 3H, Ts-Me)
$^{13}C$ NMR (100MHz, $CDCl_3$); δ 144.8 (Ar-ipso), 138.5 (Ar-ipso), 137.9 (Ar-ipso), 137.7 (Ar-ipso), 133.4 (C2), 132.8 (Ar-ipso), 129.8 Ar(), 128.4-127.6 (m, Ar), 118.4 (C3), 95.4 (C1'), 81.8 (C3'), 79.6 (C2'), 76.9 (C4'), 75.7 ($ArCH_2$), 75.0 ($ArCH_2$), 73.2 ($ArCH_2$), 68.6 (C5'), 68.5 (C6'), 68.3 (C1), 21.6 (Ts-Me).

Route E2; 1-O-(2,3,4-tri-O-benzyl-6-O-tosyl-α-D-glucopyranosyl)propane-1,3-diol (2-6):

To a solution of the compound (2-5) (29.0 g, 45.0 mmol) in anhydrous tetrahydrofuran (THF, 150 mL) was added a 0.5 M solution (180 mL, 90.0 mmol) of 9-borabicyclo[3,3,1]nonane (9-BBN) in tetrahydrofuran at 0°C under an atmosphere of argon. After 1 hour, the temperature of the reaction solution was returned to room temperature, and subsequently the solution was stirred for 10 hours. The reaction solution was again cooled to 0°C. Thereto were successively added water (20 mL), a 3 M solution (70 mL) of sodium hydroxide, and a 35% solution (70 mL) of hydrogen peroxide in water. After 1 hour, the temperature was returned to room temperature. The solution was then stirred for 12 hours. After the sufficient progress of the reaction was confirmed, this solution was extracted with ethyl acetate (3 × 100 mL). The organic layers were combined with each other, and the combination was washed with saturated saline (2 × 100 mL), dried over sodium sulfate, filtrated, and then concentrated under reduced pressure. The resultant residue was purified by silica gel chromatography (hexane/ethyl acetate, 3:2→1:1→2:3) to yield the title compound (2-6) as a colorless oily substance {28.2 g (42.5 mmol), 94.4% yield}. $[\alpha]^{24}_D$ +26.6° (c1.02 $CHCl_3$), LRMS m/z 685 $[M + Na]^+$
$^1H$ NMR (400MHz, $CDCl_3$); δ 7.76-7.74 (m, 2H, ArH), 7.35-7.26 (m, 15H, ArH), 7.16-7.12 (m, 2H, ArH), 4.94 (d, 1H, J=10.9Hz, $ArCH_2$), 4.82 (d, 1H, J=10.7Hz, $ArCH_2$), 4.77 (d, 1H, J=10.9Hz, $ArCH_2$), 4.75 (d, 1H, J=12.0Hz, $ArCH_2$), 4.61 (d, 1H, J=12.0Hz, $ArCH_2$), 4.61 (d, 1H, J=3.64Hz, H1'), 4.43 (d, 1H, J=10.7Hz, $ArCH_2$), 4.20-4.13 (m, 2H, H6'a & H6'b), 3.92 (t, 1H, J=9.24Hz, H3'), 3.84-3.74 (m, 4H, H1a & H3a & H3b & H5'), 3.48-3.40 (m, 3H, H1b & H2' & H4'), 2.52 (t, 1H, J=4.74Hz, 3-OH), 2.39 (s, 3H, Ts-Me), 1.88-1.75 (m, 2H, H2a & H2b)
$^{13}C$ NMR (100MHz, $CDCl_3$); δ 144.8 (Ar-ipso), 138.4 (Ar-ipso), 137.9 (Ar-ipso), 137.6 (Ar-ipso), 132.7 (Ar-ipso), 129.8 (Ar), 128.5-127.6 (m, Ar), 97.1 (C1'), 81.8 (C3'), 79.5 (C2'), 76.8 (C4'), 75.6 ($ArCH_2$), 75.0 ($ArCH_2$), 73.4 ($ArCH_2$), 68.7 (C5'), 68.6 (C6'), 67.5 (C1), 61.5 (C3), 31.5 (C2), 21.6 (Ts-Me).

Route F2; 1-O-(2,3,4-tri-O-benzyl-6-thioacetyl-α-D-quinovopyranosyl)-propane-1,3-diol (2-7):

Potassium thioacetate (7.28 g, 1.5 equivalent) was added to a solution of the compound (2-6) (28.2 g, 42.5 mmol) in anhydrous DMF (300 mL), and the solution was stirred at 90°C for 3 hours. After the sufficient progress of the reaction was confirmed, the reaction solution was poured into cold water (900 mL), and the resultant was extracted with ethyl acetate (3 × 300 mL). The organic layers were combined with each other, and the combination was washed with saturated saline (2 × 200 mL), dried over sodium sulfate, filtrated and concentrated under reduced pressure. The resultant residue was purified by silica gel chromatography (hexane/ethyl acetate, 2:1→3:2→1:1→2:3) to yield the title compound (2-7) as a light brown oily substance {21.9 g (38.6 mmol), 90.8% yield}. $[\alpha]^{23}_D$ +33.0°(c1.02 $CHCl_3$), LRMS m/z 584 $[M + Na]^+$

$^1H$ NMR (400MHz, $CDCl_3$); δ 7.37-7.24 (m, 15H, ArH), 4.95 (d, 1H, J=10.8Hz, $ArCH_2$), 4.89 (d, 1H, J=10.6Hz, $ArCH_2$), 4.80 (d, 1H, J=10.8Hz, $ArCH_2$), 4.77 (d, 1H, J=12.1Hz, $ArCH_2$), 4.63 (d, 1H, J=12.0Hz, $ArCH_2$), 4.63 (d, 1H, J=3.52Hz, H1'), 4.61 (d, 1H, J=10.7Hz, $ArCH_2$), 3.94 (t, 1H, J=9.22Hz, H3'), 3.88 (ddd, 1H, J=9.86, 6.10, 4.88Hz, H1a), 3.83-3.73 (m, 3H, H3a & H3b & H5'), 3.50 (dd, 1H, J=9.60, 3.64Hz, H2'), 3.45 (ddd, 1H, J=9.92, 5.24, 2.28Hz, H1b), 3.41 (dd, 1H, J=13.6, 3.00Hz, H6'a), 3.30 (dd, 1H, J=9.54, 9.06Hz, H4'), 3.02 (dd, 1H, J=13.7, 7.64Hz, H6'b), 2.67 (br, 1H, 3-OH), 2.32 (s, 3H, SAc-Me), 1.92-1.78 (m, 2H, H2a & H2b)

$^{13}C$ NMR (100MHz, $CDCl_3$); δ 195.0 (SAC-C=O), 138.5 (Ar-ipso), 137.9 (Ar-ipso), 137.8 (Ar-ipso), 128.5-127.6 (m, Ar), 95.9 (C1'), 81.8 (C3'), 80.4 (C4'), 79.8 (C2'), 75.7 ($ArCH_2$), 75.2 ($ArCH_2$), 73.4 ($ArCH_2$), 69.5 (C5'), 67.2 (C1), 61.5 (C3), 31.5 (C2), 30.8 (C6'), 30.5 (SAc-Me).

Route G2; 3-O-(2,3,4-tri-O-benzyl-6-sulfo-α-D-quinovopyranosyl)-propane-1,3-diol sodium salt (2-8):

To a solution of the compound (2-7) (500 mg, 0.88 mmol) in acetic acid (5 mL) were added Oxone (1.63 g, 2.65 mmol) and potassium acetate (25 mg). The solution was vigorously stirred at room temperature for 2 days. After the sufficient progress of the reaction was confirmed, the reaction solution was poured into a 7.5 M cold solution (15 mL) of sodium hydroxide, and the resultant was extracted with chloroform (4 × 30 mL). The organic layers were combined with each other, and the combination was washed with saturated aqueous sodium hydrogen carbonate (2 × 20 mL) and saturated saline (2 × 20 mL), dried over sodium sulfate, filtrated and concentrated under reduced pressure. The resultant residue was further dissolved in methanol (20 mL), and thereto was added an appropriate amount of sodium methoxide (NaOMe). The reactive components were then caused to react with each other at room temperature for 3 hours. While the reaction solution was cooled with ice, cold water was added to the solution to terminate the reaction. The solution was then concentrated under reduced pressure. The concentrated substance was purified by silica gel chromatography (chloroform/methanol, 100:0→3:1) to yield the title compound (2-8) as a colorless waxy substance {498 mg (0.87 mmol), 98.7% yield}. LRMS m/z 571 [M - Na]-.

Route H2; 3-O-(2,3,4-tri-O-benzyl-6-sulfo-α-D-quinovopyranosyl)-1-O-(carbobenzoxy-β-alanyl)-propane-1,3-diol sodium salt (2-9):

Into an anhydrous DMF (15 mL) mixed solution were dissolved the compound (2-8) (900 mg, 1.51 mmol), N-carbobenzoxy-β-alanine (440 mg, 1.97 mmol), 1-ethyl-3-(3-dimethylaminopropyl)carbodiimide hydrochloride (EDCI-HCl) (871 mg, 4.55 mmol), and 4-dimethylaminopyridine (55 mg, 0.15 mmol), and then this solution was left to stand overnight so that reaction between the reactive components would proceed. After the sufficient progress of the reaction was confirmed, water (1 mL) was poured into the reaction solution to terminate the reaction. Thereafter, the solution was concentrated under reduced pressure. The resultant residue was purified by silica gel chromatography (chloroform/methanol, 100:0→12:1) to yield the title compound (2-9) {517 mg (0.65 mmol), 42.8% yield). LRMS m/z 776 [M - Na]-.

Route 12; 3-O-(6-sulfo-α-D-quinovopyranosyl)-1-O-(β-alanyl)-propane-1,3-diol sodium salt (1-10):

To a solution of the compound (2-9) (517 mg, 0.65 mmol) in methanol (3.0 mL), dichloromethane (3.0 mL) and acetic acid (0.2 mL) was added 20% palladium hydroxide-activated carbon (50 mg), and then this solution was left to stand overnight at room temperature in an atmosphere of hydrogen so that reaction between the reactive components would proceed. Since the reaction did not advance sufficiently, thereto were further added methanol (3.0 mL) and 10% palladium-activated carbon (50 mg). The solution was then stirred at room temperature under an atmosphere of hydrogen for 2 days. After the sufficient progress of the reaction was confirmed, the palladium-activated carbon was filtrated off with celite, and the filtrate was concentrated under reduced pressure. The resultant residue was purified by silica gel chromatography (chloroform/methanol, 4:1→2:1→1:1→2:3) to yield the title compound (2-10) {250.8 mg (0.63 mmol), 98.2% yield}. LRMS m/z 372 [M - Na]-.

Route J2; 3-O-(6-sulfo-α-D-quinovopyranosyl)-1-O-(biotinyl-β-alanyl)-propane-1,3-diol calcium salt (1-11):

The compound (2-10) (226 mg, 0.57 mmol) was dissolved in a mixed solution of anhydrous DMF (5 mL) and triethylamine (2 mL), and then thereto was added a solution wherein a biotin-p-nitrophenylester(313 mg, 0.86 mg) was dissolved in anhydrous DMF (2 mL). The solution was stirred, at room temperature with a stirrer so that the reactive components would react with each other, and left to stand overnight. After the sufficient progress of the reaction was confirmed, toluene and methanol were added to the solution. Under reduced pressure, the solvents were then removed by evaporation to concentrate the solution, while azeotropy. The resultant residue was purified by silica gel chromatography (chloroform/methanol, 10:1→1:4) to yield a sodium salt thereof. Into the column was filled an ion exchange resin (Amberlite (registered trade name) IR-120, 15.7 mL) changed to a calcium form with a solution of calcium chloride in water. A solution of the sodium salt was repeatedly passed through the column to change the sodium salt into a calcium salt. The title compound (2-11) {210 mg (340 μmol), 59.5% yield} was produced as a result of this method.
LRMS m/z 598 $[M - H]^-$, m/z 638 $[M - H + Ca]^+$.

Synthesis Example 3

A process for producing the sulfopyranosylacylglycerol derivative according to an embodiment of the present invention is illustrated in the following scheme 3, giving an α-sulfoquinovosylmonoacylglycerol monoiodide derivative as an example thereof:

[Scheme 3]

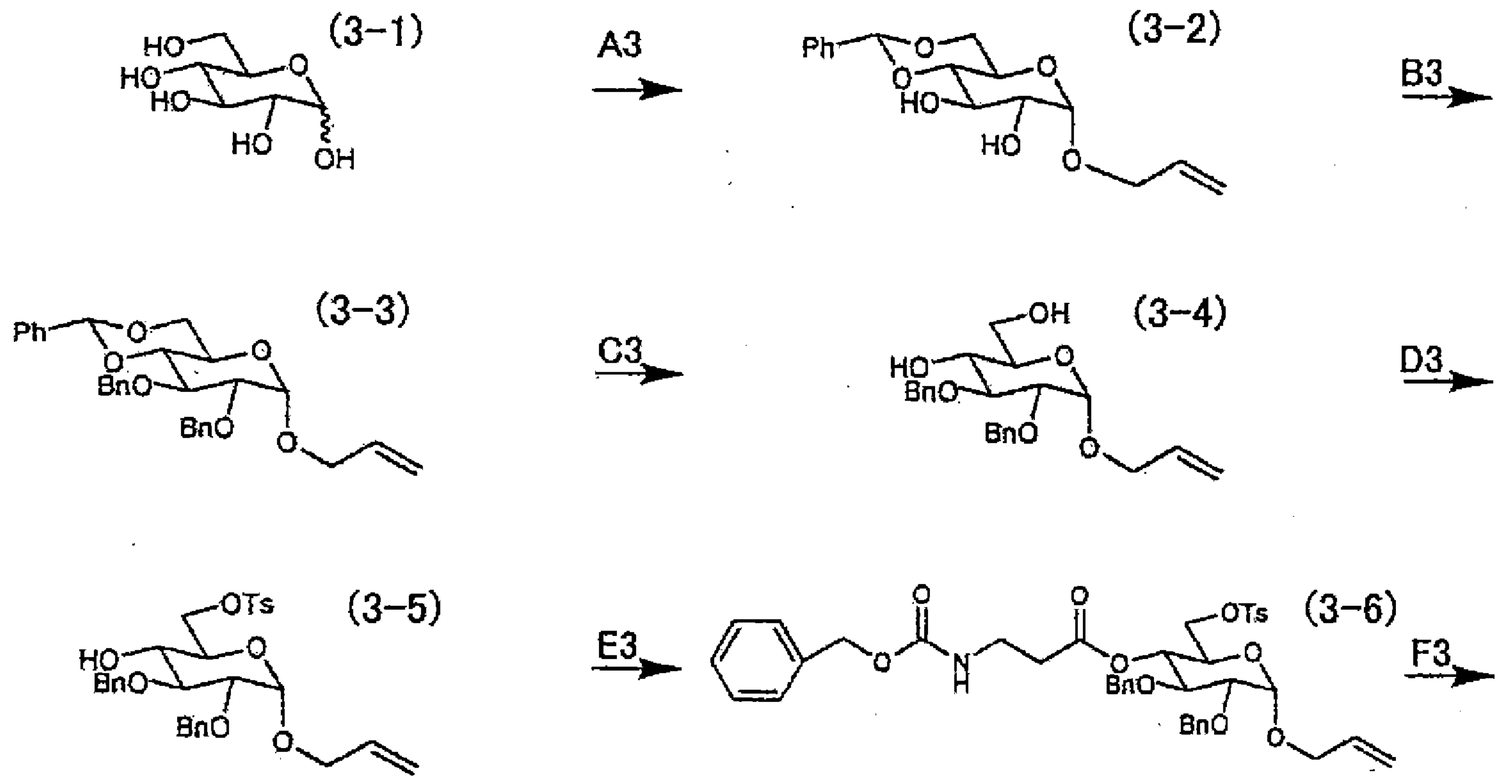


(3-1) (3-2) (3-3) (3-4) (3-5) (3-6)

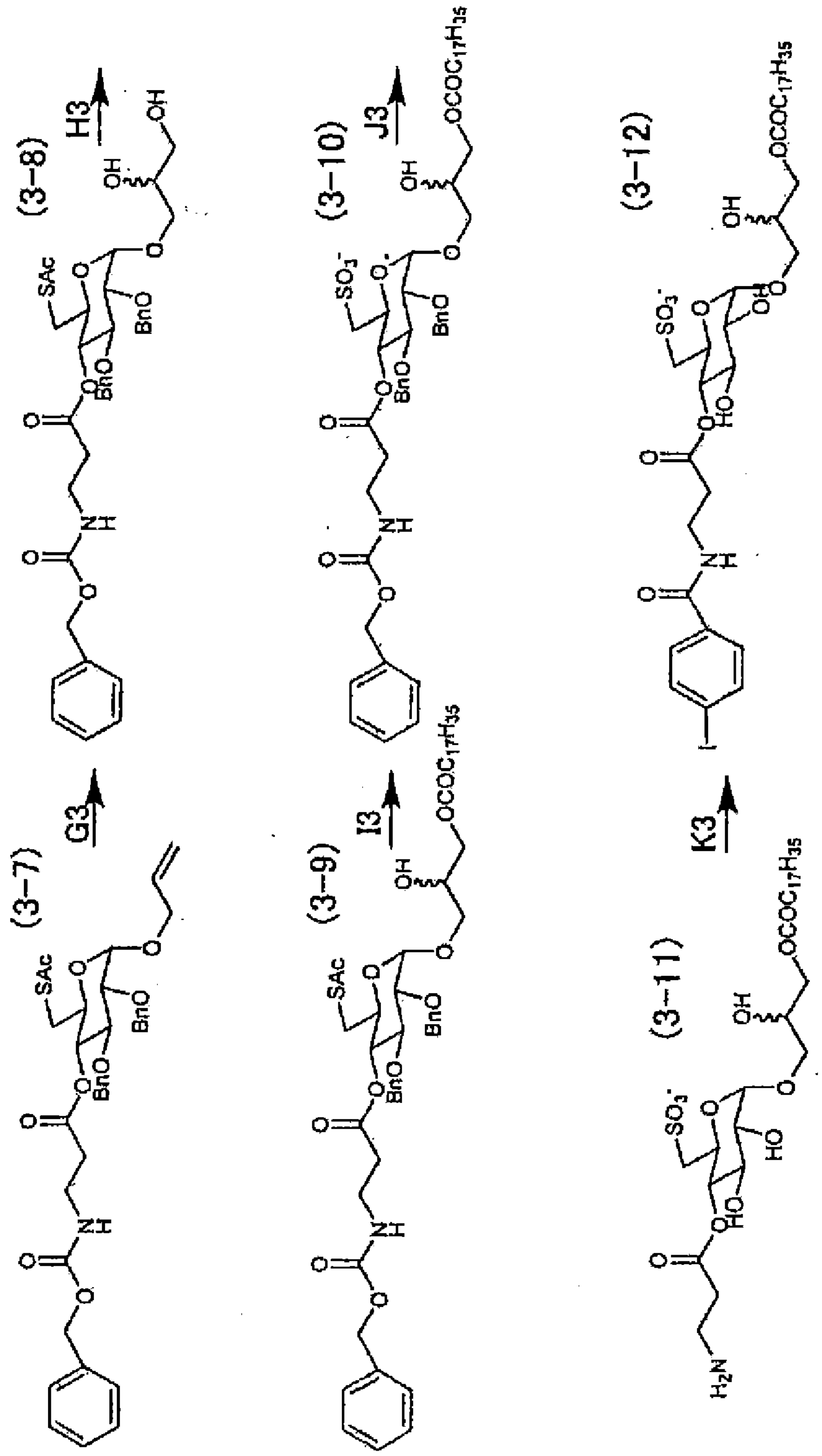


(3-7) (3-8) (3-9) (3-10) (3-11) (3-12)

The following will describe a synthesis example through individual steps in detail:

<Synthesis Example>

Route A3; 1-O-allyl-4,6-O-benzylidene-α-D-glucopyranoside (3-2):

Trifluoromethanesulfonic acid (1.00 mL) was added to a liquid suspension of the compound (3-1) (100 g, 555 mmol) in allyl alcohol (500 mL) at 0°C, and the reaction liquid was vigorously stirred at 80°C for 48 hours. After the sufficient progress of the reaction was confirmed, thereto was added triethylamine (3 mL) to terminate the reaction. The resultant was concentrated under reduced pressure. Next, the residue was suspended in anhydrous acetonitrile (500 mL), and thereto were added benzaldehydedimethylacetal (127 g, 1.5 equivalent) and p-toluenesulfonic acid monohydrate (5.28 g, 0.05 equivalent). The reaction liquid was stirred at 40°C for 4 hours, and then thereto was added triethylamine (10 mL) to terminate the reaction. The resultant was concentrated under reduced pressure. The residue was poured into hexane (2000 mL) and water (500 mL), and then the mixed liquid was vigorously stirred. The resultant precipitate was filtrated, and rinsed with water and hexane. The precipitate was crystallized two times from heated ethanol to yield the title compound (3-2) as a colorless needle crystal {34.5 g (112 mmol), 20.2% yield}.
LRMS m/z 331 $[M+Na]^+$.

Route B3; 1-O-allyl-2,3-di-O-benzyl-4,6-O-benzylidene-α-D-glucopyranoside (3-3):

To a solution of the compound (3-2) (30.0 g, 97.3 mmol) in anhydrous N,N-dimethylformamide (DMF, 300 mL) were added benzylbromide (41.6 g, 2.5 equivalent) and sodium hydroxide (11.7 g, 3.0 equivalent), and the reaction solution was vigorously stirred at room temperature for 24 hours. After the sufficient progress of the reaction was confirmed, the reaction solution was poured into cold water (900 mL), and the resultant was extracted with ethyl acetate (3 × 300 mL). The organic layers were combined with each other, and the combination was washed with saturated saline (2 × 100 mL), dried over sodium sulfate, filtrated and then concentrated under reduced pressure. The resultant residue was crystallized two times from heated ethanol to yield the title compound (3-3) as a colorless needle crystal (33.5 g). The filtrate was concentrated, purified by silica gel chromatography (hexane/ethyl acetate, 15:1→10:1→8:1), and then crystallized from heated ethanol to yield the same compound (3-3) (6.63 g) {total amount: 40.1 g (82.1 mmol), 84.4% yield}.
LRMS m/z 511 $[M+Na]^+$.

Route C3; 1-O-ally-2,3-di-O-benzyl-α-D-glucopyranoside (3-4):

The compound (3-3) (15.6 g, 32.0 mmol) was dissolved in acetic acid (90 mL), and further distilled water (50 mL) was added thereto. The solution was stirred for 1 hour while heated and refluxed. After the sufficient progress of the reaction was confirmed, the solution was cooled to room temperature, and then the solvent was removed by evaporation. Distilled water (15 mL) was added thereto, and the solution was again concentrated under reduced pressure; this operation was repeated 4 times. Thereafter, the resultant was purified by silica gel flash chromatography (hexane/ethyl acetate, 4:1→2:1→1:1→1:2) to yield the title compound (3-4) (12.1 g (30.2 mmol), 94.5% yield).
LRMS m/z 423 $[M+Na]^+$.

Route D3; 1-O-ally-2,3,4-di-O-benzyl-6-O-tosyl-a-D-glucopyranoside (3-5):

To a solution of the compound (3-4) (12.1 g, 30.2 mmol) in anhydrous pyridine (120 mL) were added p-toluenesulfonyl chloride (7.5 g, 39.3 mmol) and 4-dimethylaminopyridine (369 mg, 3.0 mmol), and the reaction solution was stirred at 0°C for 16 hours. After the sufficient progress of the reaction was confirmed, the reaction solution was slowly poured into ice water (100 mL). The water layer was subjected to extraction with ethyl acetate (3 × 200 mL). The organic layers were combined with each other. The combination was washed with an aqueous 1 N HCl solution until the pH became 4, washed with saturated aqueous sodium hydrogen carbonate (2 × 100 mL) and saturated saline (2 × 100 mL), dried over sodium sulfate, filtrated and then concentrated under reduced pressure. The concentrated product was purified by silica gel flash chromatography (hexane/ethyl acetate, 6:1→4:1→3:1) to yield the title compound (3-5) {15.3 g (27.6 mmol), 91.4% yield}.
LRMS m/z 577 $[M+Na]^+$.

Route E3; 1-O-allyl-2,3-di-O-benzyl-4-O-(carbobenzoxy-β-alanyl)-6-O-tosyl-α-D-glucopyranoside (3-6):

Into a mixed solution of anhydrous dichloromethane (200 mL) and anhydrous pyridine (50 mL) were dissolved the compound (3-5) (15.3 g, 27.6 mmol), N-carbobenzoxy-β-alanine (12.32 g, 55.2 mmol), 1-ethyl-3-(3-dimethylaminopropyl)carbodiimide hydrochloride (EDCI·HCl) (15.8 mg, 82.8 mmol) and 4-dimethylaminopyridine (6.7 g, 55.2 mmol),

and then the reactive components were caused to react with each other at room temperature for 18 hours. After the sufficient progress of the reaction was confirmed, water (10 mL) was poured into the reaction solution to terminate the reaction. Thereafter, the solution was concentrated under reduced pressure. The resultant residue was purified by silica gel chromatography (hexane/ethyl acetate, 4:1→3:1→2:1→3:2) to yield the title compound (3-6) {17.1 g (22.5 mmol), 81.5% yield}.

LRMS m/z 782 $[M+Na]^+$.

Route F3; 1-O-allyl-2,3-di-O-benzyl-4-O-(carbobenzoxy-β-alanyl)-6-thioacetyl-α-D-glucopyranoside (3-7):

Potassium thioacetate (5.1 g, 45.0 mmol) was added to a solution of the compound (3-6) (17.1 g, 22.5 mmol) in anhydrous N,N-dimethylformamide (300 mL), and the solution was stirred at 90°C for 3 hours. After the sufficient progress of the reaction was confirmed, the reaction solution was poured into cold water (400 mL), and the resultant was extracted with ethyl acetate (3 × 150 mL). The organic layers were combined with each other, and the combination was washed with saturated saline (2 × 100 mL), dried over sodium sulfate, filtrated and concentrated under reduced pressure. The resultant residue was purified by silica gel chromatography (hexane/ethyl acetate, 4:1→3:1→2:1→3:2) to yield the title compound (3-7) {14.2 g (20.3 mmol), 90.0% yield}.
LRMS m/z 686 $[M+Na]^+$.

Route G3; 3-O-[2,3-di-O-benzyl-4-O-(carbobenzoxy-β-alanyl)-6-thioacetyl-α-D-quinovopyranosyl-glycerol (3-8):

The compound (3-7) (14.2 g, 20.4 mmol) was dissolved in a solution (200 mL) of t-butyl alcohol and distilled water (4:1), and then thereto were added a 0.04 M osmium tetraoxide solution in t-butyl alcohol (3 mL) and trimethylamine N-oxide (3.4 g, 30.5 mmol). The solution was stirred with a stirrer at room temperature for 24 hours. After the sufficient progress of the reaction was confirmed, 3 g of activated carbon was added thereto and then the solution was stirred for 30 minutes to cause the catalyst to be adsorbed on the carbon. The solution was subjected to suction filtration through a Kiriyama funnel containing celite, so as to remove the catalyst. The reaction product remaining on the celite was washed three times with ethyl acetate so as to be collected. Distilled water (200 mL) was added to the collected filtrate, and the resultant was extracted with ethyl acetate (3 × 150 mL). The organic layers were combined with each other, and the combination was washed with saturated saline (2 × 100 mL), dried over sodium sulfate, filtrated and concentrated under reduced pressure. The resultant residue was purified by silica gel chromatography (hexane/ethyl acetate, 2:1→1:1→2:3→1:2→1:4→1:8) to yield the title compound (3-8) {13.2 g (18.9 mmol), 93.0% yield}.
LRMS m/z 720 $[M+Na]^+$.

Route H3; 3-O-[2,3-di-O-benzyl-4-O-(carbobenzoxy-β-alanyl)-6-thioacetyl-α-D-quinovopyranosyl]-1-O-stearoyl-glycerol (3-9):

Stearoyl chloride (8.5 g, 28.2 mmol) was added to a mixed solution of the compound (3-8) (13.1 g, 18.8 mmol) in anhydrous dichloromethane (200 mL) and anhydrous pyridine (50 mL), and then the solution was stirred at room temperature for 2 hours. After the sufficient progress of the reaction was confirmed, methanol (5 mL) was added thereto so as to terminate the reaction. The solution was concentrated under reduced pressure. A small amount of ethyl acetate was used to suspend the residue, and the suspended residue was poured into water (200 mL). The resultant was extracted with ethyl acetate (3 × 100 mL). The organic layers were combined with each other, and the combination was washed with saturated saline (2 × 100 mL), dried over sodium sulfate, filtrated and then concentrated under reduced pressure. The resultant residue was purified by silica gel chromatography (hexane/ethyl acetate, 6:1→4:1→2:1→3:2→1:1) to yield the title compound (3-9) as a colorless oily substance {7.1 g (7.4 mmol), 39.4% yield}.
LRMS m/z 987 $[M+Na]^+$.

Route I3; 3-O-[2,3-di-O-benzyl-4-O-(carbobenzoxy-β-alanyl)-6-sulfo-α-D-quinovopyranosyl]-1-O-stearoyl-glycerol (3-10):

Oxone (18.1 g, 29.5 mmol) and potassium acetate (4.0 g) were added to a solution of the compound (3-9) (7.1 g, 7.4 mmol) in acetic acid (160 g, 4 mol), and then the solution was vigorously stirred at room temperature for 48 hours. After the sufficient progress of the reaction was confirmed, the reaction solution was poured into a cold 7.5 M sodium hydroxide solution (500 mL) and the resultant was extracted with ethyl acetate (4 × 100 mL). The organic layers were combined with each other, and the combination was washed with saturated aqueous sodium hydrogen carbonate (2 × 100 mL) and saturated saline (2 × 100 mL), dried over sodium sulfate, filtrated and then concentrated under reduced pressure. The resultant residue was purified by silica gel chromatography (chloroform/methanol, 100:1→50:1→20:1→15:

1→12:1) to yield the title compound (3-10) as a colorless waxy substance {5.2 g (5.24 mmol), 71.2% yield}.
LRMS m/z 968 $[M-Na]^-$.

Route J3; 3-O-[4-O-(β-alanyl)-6-sulfo-α-D-quinovopyranosyl]-1-O-stearoyl-glycerol (3-11):

To a solution of the compound (3-10) (490 mg, 494 μmol) in methanol (20 mL) and acetic acid (0.6 mL) was added 10% palladium-activated carbon (300 mg), and then the solution was stirred at room temperature under an atmosphere of hydrogen gas for 48 hours. After the sufficient progress of the reaction was confirmed, the palladium-activated carbon was filtrated off. The filtrate was concentrated under reduced pressure. The resultant residue was purified by silica gel chromatography (chloroform/methanol/distilled water, 100:25:3→65:25:4→65:35:5→65:45:6) to yield the title compound (3-11) {221 mg (327 μmol), 66.1% yield}.
LRMS m/z 654 $[M-Na]^-$.

Route K3; 3-O-[4-O-(4-iodobenzoyl-β-alanyl)-6-sulfo-α-D-quinovopyranosyl]-1-O-stearoyl-glycerol (3-12) :

The compound (3-11) (221 mg, 327 μmol) was dissolved in a mixed solution of anhydrous dichloromethane (15 mL), pyridine (5 mL) and triethylamine (2 mL), and thereto was added (4-iodobenzoyl)-p-nitrophenyl ester (241 mg, 653 μmol). While the solution was stirred with a stirrer, the reactive components were caused to react with each other at room temperature for 24 hours. After the sufficient progress of the reaction was confirmed, toluene and methanol were added to the solution. Under reduced pressure, the solvents were then removed by evaporation to concentrate the solution, while azeotropy. The resultant residue was purified by silica gel chromatography (chloroform/methanol/distilled water, 30:5:0.5→30:6:0.7→30:8:09→30:10:1.1) to yield the title compound (3-12) {262 mg (238 μmol), 72.8% yield}.
LRMS m/z 884 $[M - Na]^-$.

Synthesis Example 4

A process for producing the sulfopyranosylacylglycerol derivative according to an embodiment of the present invention is illustrated in the following scheme 4, giving an α-sulfoquinovosylmonoacylglycerol triiodide derivative as an example thereof:

[Scheme 4]

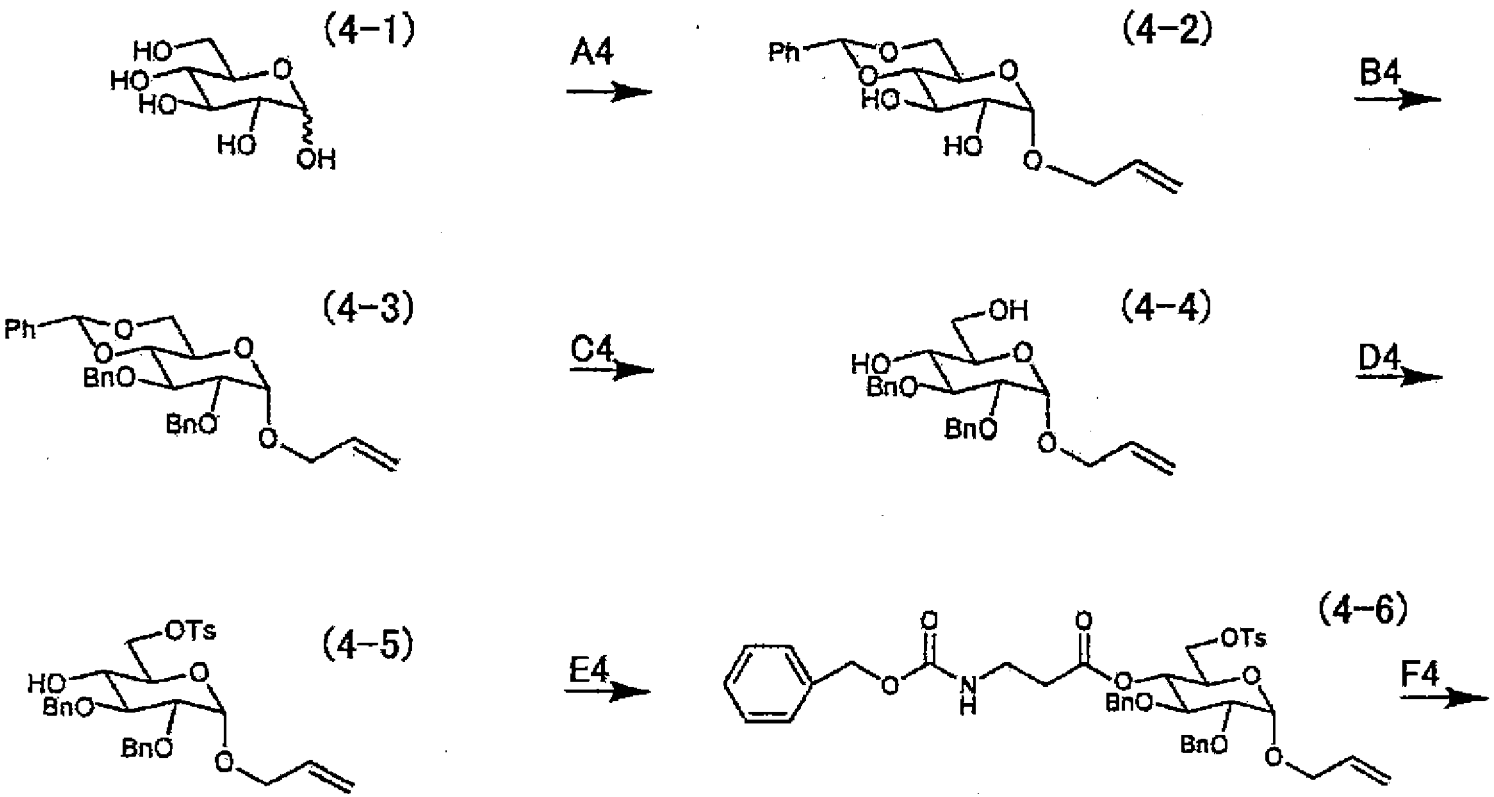


(4-1) (4-2) (4-3) (4-4) (4-5) (4-6)

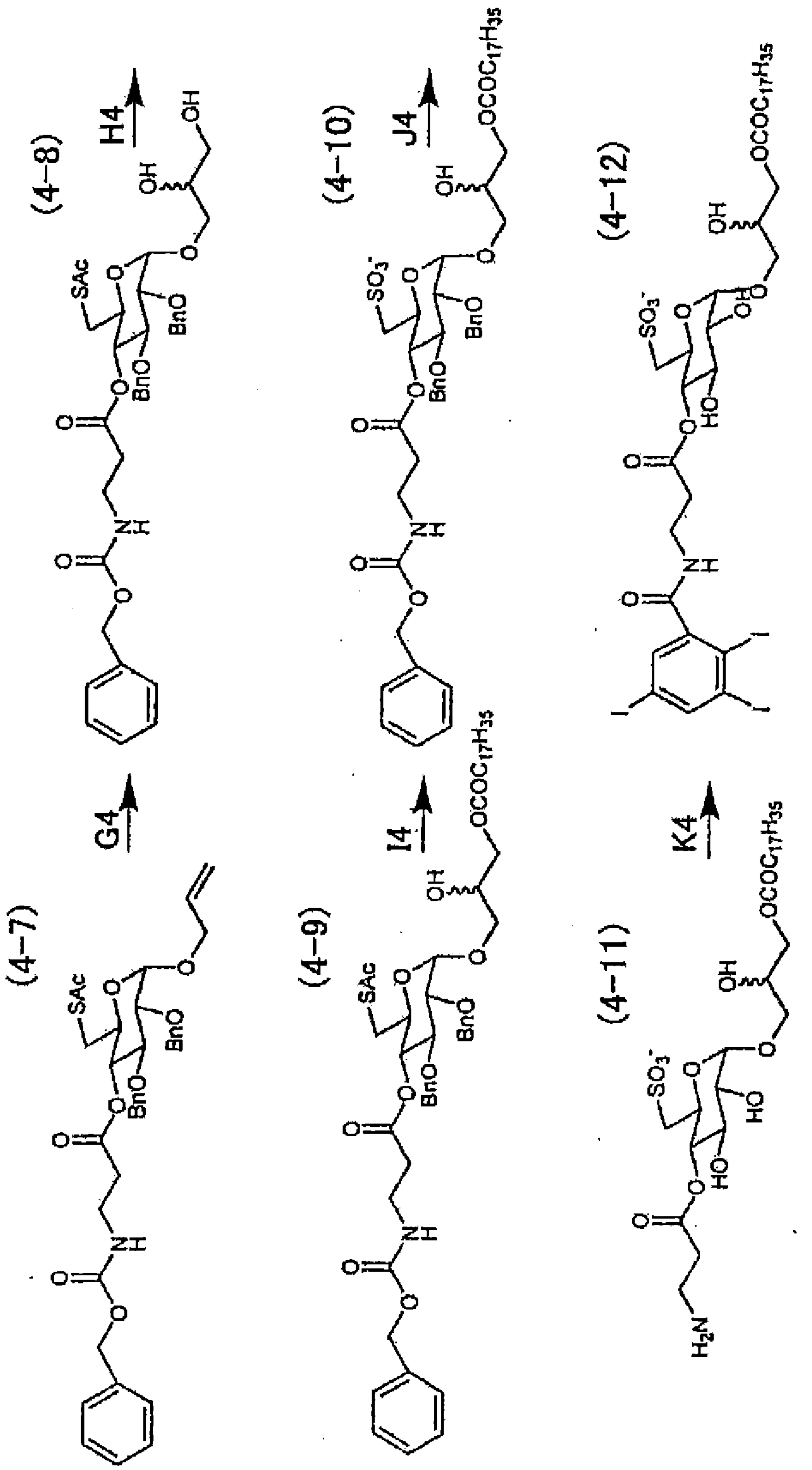


(4-7) (4-8) (4-9) (4-10) (4-11) (4-12)

The following will describe a synthesis example through the individual steps in detail:

<Synthesis Example>

Route A4; 1-O-allyl-4,6-O-benzylidene-α-D-glucopyranoside (4-2):

Trifluoromethanesulfonic acid (1.00 mL) was added to a liquid suspension of the compound (4-1) (100 g, 555 mmol) in allyl alcohol (500 mL) at 0°C, and the reaction liquid was vigorously stirred at 80°C for 48 hours. After the sufficient progress of the reaction was confirmed, thereto was added triethylamine (3 mL) to terminate the reaction. The resultant was concentrated under reduced pressure. Next, the residue was suspended in anhydrous acetonitrile (500 mL), and thereto were added benzaldehydedimethylacetal (127 g, 1.5 equivalent) and p-toluenesulfonic acid monohydrate (5.28 g, 0.05 equivalent). The resultant liquid was stirred at 40°C for 4 hours, and then thereto was added triethylamine (10 mL) to terminate the reaction. The resultant was concentrated under reduced pressure. The residue was poured into hexane (2000 mL) and water (500 mL), and then the mixed liquid was vigorously stirred. The resultant precipitate was filtrated, and rinsed with water and hexane. The precipitate was crystallized two times from heated ethanol to yield the title compound (4-2) as a colorless needle crystal {34.5 g (112 mmol), 20.2% yield}.
LRMS m/z 331 $[M+Na]^+$.

Route B4; 1-O-allyl-2,3-di-O-benzyl-4,6-O-benzylidene-α-D-glucopyranoside (4-3):

To a solution of the compound (4-2) (30.0 g, 97.3 mmol) in anhydrous N,N-dimethylformamide (DMF, 300 mL) were added benzylbromide (41.6 g, 2.5 equivalent) and sodium hydroxide (11.7 g, 3.0 equivalent), and the reaction solution was vigorously stirred at room temperature for 24 hours. After the sufficient progress of the reaction was confirmed, the reaction solution was poured into cold water (900 mL), and the resultant was extracted with ethyl acetate (3 × 300 mL). The organic layers were combined with each other, and the combination was washed with saturated saline (2 × 100 mL), dried over sodium sulfate, filtrated and then concentrated under reduced pressure. The resultant residue was crystallized two times from heated ethanol to yield the title compound (4-3) as a colorless needle crystal (33.5 g). The filtrate was concentrated, purified by silica gel chromatography (hexane/ethyl acetate, 15:1→10:1→8:1), and then crystallized from heated ethanol to yield the same title compound (4-3) (6.63 g) {total amount: 40.1 g (82.1 mmol), 84.4% yield}.
LRMS m/z 511 $[M+Na]^+$.

Route C4; 1-O-ally-2,3-di-O-benzyl-α-D-glucopyranoside (4-9):

The compound (4-3) (15.6 g, 32.0 mmol) was added to acetic acid (90 mL), and further distilled water (50 mL) was added thereto. The solution was stirred for 1 hour while heated and refluxed. After the sufficient progress of the reaction was confirmed, the solution was cooled to room temperature, and then the solvent was removed by evaporation. Distilled water (15 mL) was added thereto, and the solution was again concentrated under reduced pressure; this operation was repeated 4 times. Thereafter, the resultant was purified by silica gel flash chromatography (hexane/ethyl acetate, 4:1→2:1→1:1→1:2) to yield the title compound (4-4) {12.1 g (30.2 mmol), 94.5% yield}.
LRMS m/z 423 $[M+Na]^+$.

Route D4; 1-O-ally-2,3,4-di-O-benzyl-6-O-tosyl-α-D-glucopyranoside (4-5):

To a solution of the compound (4-4) (12.1 g, 30.2 mmol) in anhydrous pyridine (120 mL) were added p-toluenesulfonyl chloride (7.5 g, 39.3 mmol) and 4-dimethylaminopyridine (369 mg, 3.0 mmol), and the reaction solution was stirred at 0°C for 16 hours. After the sufficient progress of the reaction was confirmed, the reaction solution was slowly poured into ice water (100 mL). The water layer was subjected to extraction with ethyl acetate (3 × 200 mL). The organic layers were combined with each other. The combination was washed with an aqueous 1 N HCl solution until the pH become 4, washed with saturated aqueous sodium hydrogen carbonate (2 × 100 mL) and saturated saline (2 × 100 mL), dried over sodium sulfate, filtrated and then concentrated under reduced pressure. The concentrated product was purified by silica gel flash chromatography (hexane/ethyl acetate, 6:1→4:1→2:1) to yield the title compound (4-5) {15.3 g (27.6 mmol), 91.4% yield}.
LRMS m/z 577 $[M+Na]^+$.

Route E4; 1-O-allyl-2,3-di-O-benzyl-4-O-(carbobenzoxy-β-alanyl)-6-O-tosyl-a-D-glucopyranoside (4-6):

Into a mixed solution of anhydrous dichloromethane (200 mL) and anhydrous pyridine (50 mL) were dissolved

the compound (4-5) (15.3 g, 27.6 mmol), N-carbobenzoxy-β-alanine (12.32 g, 55.2 mmol), 1-ethyl-3-(3-dimethylaminopropyl)carbodiimide hydrochloride (EDCI·HCl) (15.8 mg, 82.8 mmol) and 4-dimethylaminopyridine (6.7 g, 55.2 mmol), and then the reactive components were caused to react with each other at room temperature for 18 hours. After the sufficient progress of the reaction was confirmed, water (10 mL) was poured into the reaction solution to terminate the reaction. Thereafter, the solution was concentrated under reduced pressure. The resultant residue was purified by silica gel chromatography (hexane/ethyl acetate, 4:1→3:1→2:1→3:2) to yield the title compound (4-6) {17.1 g (22.5 mmol), 81.5% yield}.
LRMS m/z 782 $[M+Na]^+$.

Route F4; 1-O-allyl-2,3-di-O-benzyl-4-O-(carbobenzoxy-β-alanyl)-6-thioacetyl-α-D-glucopyranoside (4-7):

Potassium thioacetate (5.1 g, 45.0 mmol) was added to a solution of the compound (4-6) (17.1 g, 22.5 mmol) in anhydrous N,N-dimethylformamide (300 mL), and the solution was stirred at 90°C for 3 hours. After the sufficient progress of the reaction was confirmed, the reaction solution was poured into cold water (400 mL), and the resultant was extracted with ethyl acetate (3 × 150 mL). The organic layers were combined with each other, and the combination was washed with saturated saline (2 × 100 mL), dried over sodium sulfate, filtrated and concentrated under reduced pressure. The resultant residue was purified by silica gel chromatography (hexane/ethyl acetate, 4:1→3:1→2:1→3:2) to yield the title compound (4-7) {14.2 g (20.3 mmol), 90.0% yield}.
LRMS m/z 686 $[M+Na]^+$.

Route G4; 3-O-[2,3-di-O-benzyl-4-O-(carbobenzoxy-β-alanyl)-6-thioacetyl-α-D-quinovopyranosyl-glycerol (4-8):

The compound (4-7) (14.2 g, 20.4 mmol) was dissolved in a solution (200 mL) of t-butyl alcohol and distilled water (4:1), and then thereto were added a 0.04 M osmium tetraoxide solution in t-butyl alcohol (3 mL) and trimethylamine N-oxide (3.4 g, 30.5 mmol). The solution was stirred with a stirrer at room temperature for 24 hours. After the sufficient progress of the reaction was confirmed, 3 g of activated carbon was added thereto and then the solution was stirred for 30 minutes to cause the catalyst to be adsorbed on the carbon. The solution was subjected to suction filtration through a Kiriyama funnel containing celite, so as to remove the catalyst. The reaction product remaining on the celite was washed three times with ethyl acetate so as to be collected. Distilled water (200 mL) was added to the collected filtrate, and the resultant was extracted with ethyl acetate (3 × 150 mL). The organic layers were combined with each other, and the combination was washed with saturated saline (2 × 100 mL), dried over sodium sulfate, filtrated and concentrated under reduced pressure. The resultant residue was purified by silica gel chromatography (hexane/ethyl acetate, 2:1→1:1→2:3→1:2→1:4→1:8) to yield the title compound (4-8) {13.2 g (18.9 mmol), 93.0% yield}.
LRMS m/z 720 $[M+Na]^+$.

Route H4; 3-O-[2,3-di-O-benzyl-4-O-(carbobenzoxy-β-alanyl)-6-thioacetyl-α-D-quinovopyranosyl]-1-O-stearoyl-glycerol (4-9):

Stearoyl chloride (8.5 g, 28.2 mmol) was added to a mixed solution of the compound (4-8) (13.1 g, 18.8 mmol) in anhydrous dichloromethane (200 mL) and anhydrous pyridine (50 mL), and then the solution was stirred at room temperature for 2 hours. After the sufficient progress of the reaction was confirmed, methanol (5 mL) was added thereto so as to terminate the reaction. The solution was concentrated under reduced pressure. A small amount of ethyl acetate was used to suspend the residue, and the suspended residue was poured into water (200 mL). The resultant was extracted with ethyl acetate (3 × 100 mL). The organic layers were combined with each other, and the combination was washed with saturated saline (2 × 100 mL), dried over sodium sulfate, filtrated and then concentrated under reduced pressure. The resultant residue was purified by silica gel chromatography (hexane/ethyl acetate, 6:1→4:1→2:1,3:2→1:1) to yield the title compound (4-9) as a colorless oily substance {7.1 g (7.4 mmol), 39.4% yield}.
LRMS m/z 987 $[M+Na]^+$.

Route I4; 3-O-[2,3-di-O-benzyl-4-O-(carbobenzoxy-β-alanyl)-6-sulfo-α-D-quinovopyranosyl]-1-O-stearoyl-glycerol (4-10):

Oxone (18.1 g, 29.5 mmol) and potassium acetate (4.0 g) were added to a solution of the compound (4-9) (7.1 g, 7.4 mmol) in acetic acid (160 g, 4 mol), and then the solution was vigorously stirred at room temperature for 48 hours. After the sufficient progress of the reaction was confirmed, the reaction solution was poured into a cold 7.5 M sodium hydroxide solution (500 mL) and the resultant was extracted with ethyl acetate (4 × 100 mL). The organic layers were combined with each other, and the combination was washed with saturated aqueous sodium hydrogen carbonate (2 × 100 mL) and saturated saline (2 × 100 mL), dried over sodium sulfate, filtrated and then concentrated under reduced

pressure. The resultant residue was purified by silica gel chromatography (chloroform/methanol, 100:1→50:1→20:1→15:1→12:1) to yield the title compound (4-10) as a colorless waxy substance {5.2 g (5.24 mmol), 71.2% yield}.
LRMS m/z 968 $[M-Na]^-$.

Route J4; 3-O-[4-O-(β-alanyl)-6-sulfo-α-D-quinovopyranosyl]-1-O-stearoyl-glycerol (4-11):

To a solution of the compound (4-10) (191 mg, 193 μmol) in methanol (10 mL), dichloromethane (10 mL) and acetic acid (0.2 mL) was added 10% palladium-activated carbon (160 mg), and then the solution was stirred at room temperature under an atmosphere of hydrogen gas for 48 hours. After the sufficient progress of the reaction was confirmed, the palladium-activated carbon was filtrated off. The filtrate was concentrated under reduced pressure. The resultant residue was purified by silica gel chromatography (chloroform/methanol/distilled water, 100:25:3→65:25:4→65:35:5→65:45:6) to yield the title compound (4-11).
LRMS m/z 654 $[M-Na]^-$.

Route K4; 3-O-[4-O-(2,3,5-triiodobenzoyl-β-alanyl)-6-sulfo-α-D-quinovopyranosyl]-1-O-stearoyl-glycerol (4-12):

Into a mixed solution of anhydrous dichloromethane (10 mL) and anhydrous pyridine (5 mL) were dissolved the compound (4-11), 2,3,5-triiodobenzoic acid (192 mg, 385 μmol), 1-ethyl-3-(3-dimethylaminopropyl)carbodiimide hydrochloride (EDCI·HCl) (73.8 mg, 385 μmol) and 4-dimethylaminopyridine (2 g, 19 μmol), and then the reactive components were caused to react with each other at room temperature for 18 hours. After the sufficient progress of the reaction was confirmed, water (10 mL) was poured into the reaction solution to terminate the reaction. Thereafter, the solution was concentrated under reduced pressure. The resultant residue was purified by silica gel chromatography (chloroform/methanol/distilled water, 50:10:1→40:10:1.2→30:10:1.5→20:10:2) to yield the title compound (4-12) {81 mg (70 μmol), 36.3% yield, which was the yield through the two routes J4 and K4}.
LRMS m/z 1136 $[M - Na]^-$.

Synthesis Example 5

A process for producing the sulfopyranosylacylglycerol derivative according to an embodiment of the present invention is illustrated in the following scheme 5, giving an α-sulfoquinovosyldiacylglycerol monoiodide derivative as an example thereof:

[Scheme 5]

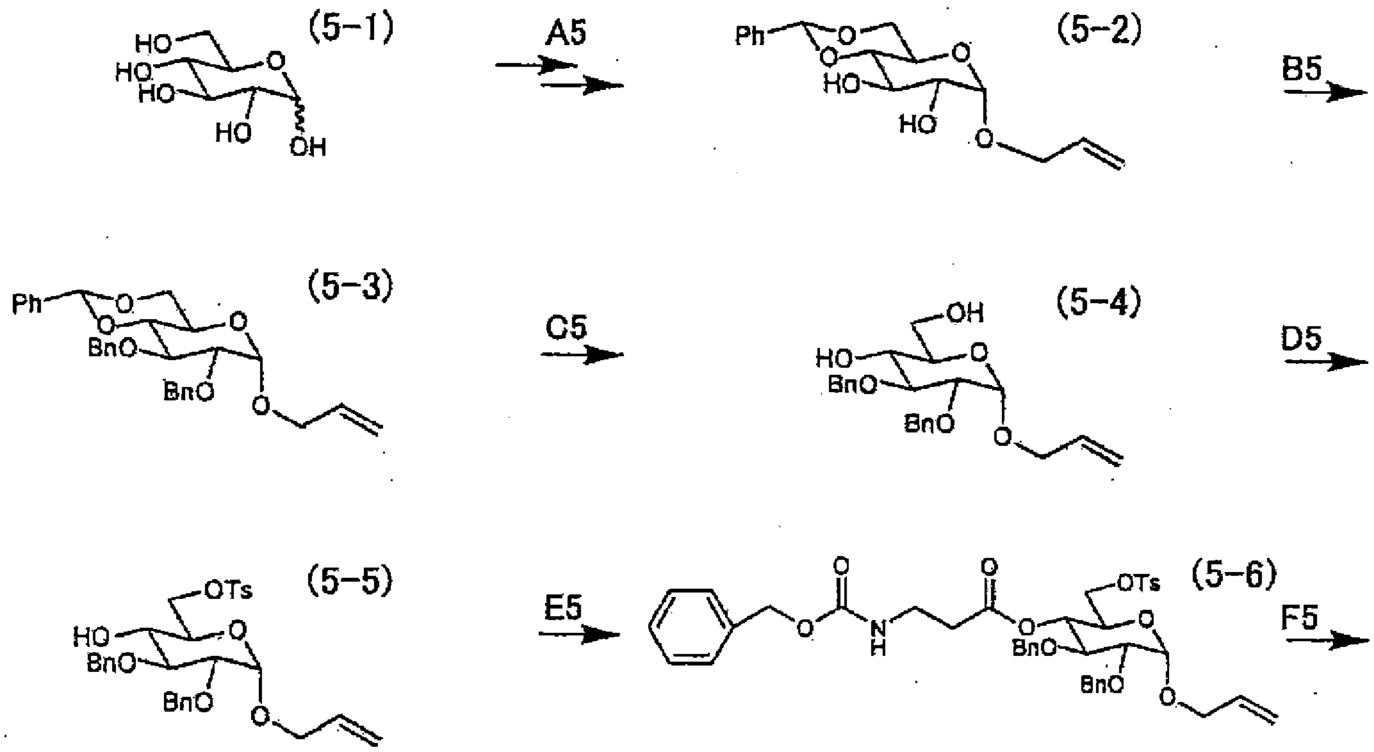


(5-1) (5-2) (5-3) (5-4) (5-5) (5-6)

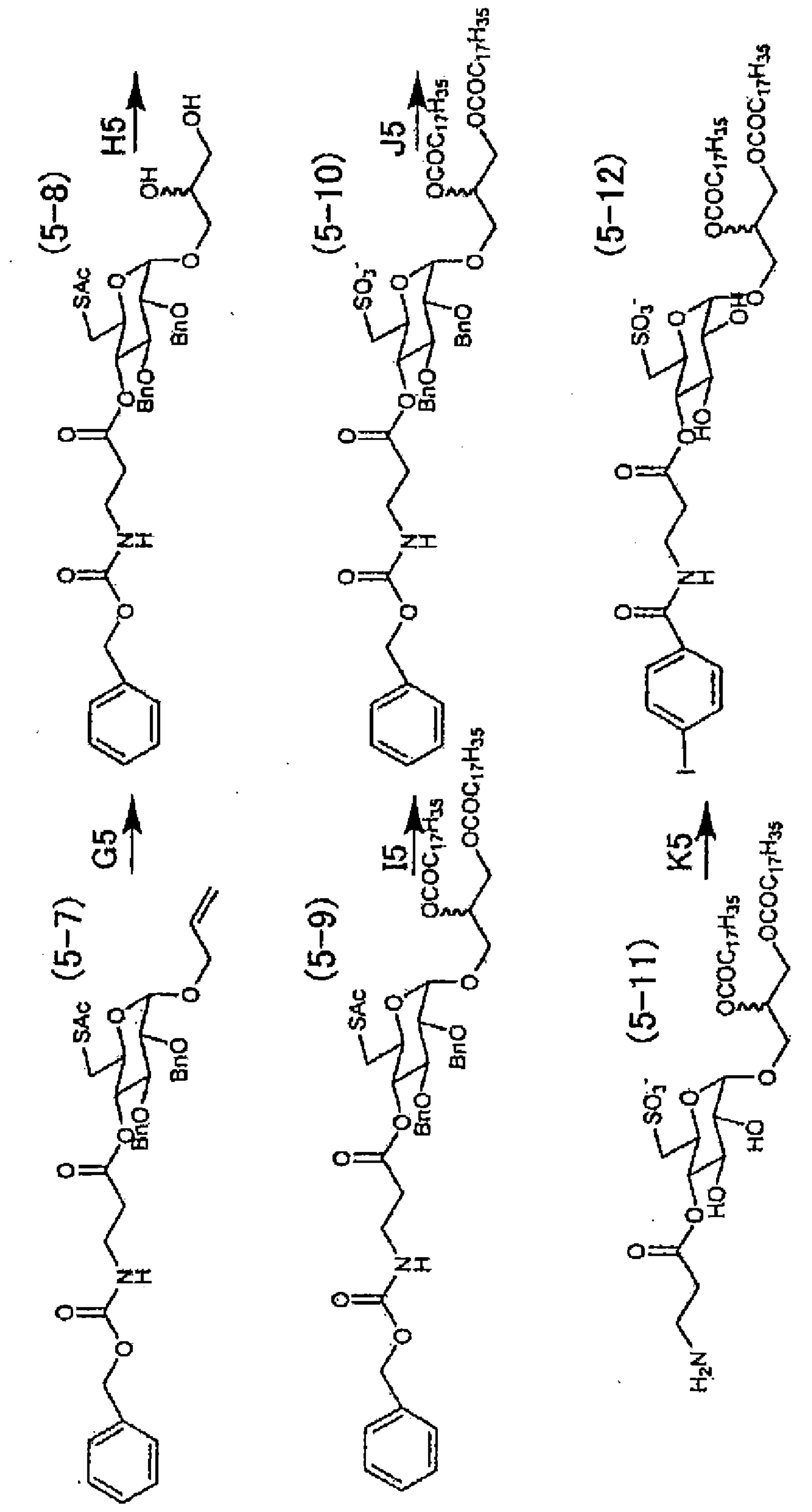


The following will describe a synthesis example through the individual steps in detail:

<Synthesis Example>

Route A5; 1-O-allyl-4,6-O-benzylidene-α-D-glucopyranoside (5-2):

Trifluoromethanesulfonic acid (1.00 mL) was added to a liquid suspension of the compound (5-1) (100 g, 555 mmol) in allyl alcohol (500 mL) at 0°C, and the reaction liquid was vigorously stirred at 80°C for 48 hours. After the sufficient progress of the reaction was confirmed, thereto was added triethylamine (3 mL) to terminate the reaction. The resultant was concentrated under reduced pressure. Next, the residue was suspended in anhydrous acetonitrile (500 mL), and thereto were added benzaldehydedimethylacetal (127 g, 1.5 equivalent) and p-toluenesulfonic acid monohydrate (5.28 g, 0.05 equivalent). The resultant liquid was stirred at 40°C for 4 hours, and then thereto was added triethylamine (10 mL) to terminate the reaction. The resultant was concentrated under reduced pressure. The residue was poured into hexane (2000 mL) and water (500 mL), and then the mixed liquid was vigorously stirred. The resultant precipitate was filtrated, and rinsed with water and hexane. The precipitate was crystallized two times from heated ethanol to yield the title compound (5-2) as a colorless needle crystal (34.5 g (112 mmol), 20.2% yield).
LRMS m/z 331 $[M+Na]^+$.

Route B5; 1-O-allyl-2,3-di-O-benzyl-4,6-O-benzylidene-α-D-glucopyranoside (5-3):

To a solution of the compound (5-2) (30.0 g, 97.3 mmol) in anhydrous N,N-dimethylformamide (DMF, 300 mL) were added benzylbromide (41.6 g, 2.5 equivalent) and sodium hydroxide (11.7 g, 3.0 equivalent), and the reaction solution was vigorously stirred at room temperature for 24 hours. After the sufficient progress of the reaction was confirmed, the reaction solution was poured into cold water (900 mL), and the resultant was extracted with ethyl acetate (3 × 300 mL). The organic layers were combined with each other, and the combination was washed with saturated saline (2 × 100 mL), dried over sodium sulfate, filtrated and then concentrated under reduced pressure. The resultant residue was crystallized two times from heated ethanol to yield the title compound (5-3) as a colorless needle crystal (33.5 g). The filtrate was concentrated, purified by silica gel chromatography (hexane/ethyl acetate, 15:1→10:1→8:1), and then crystallized from heated ethanol to yield the same compound (5-3) (6.63 g) {total amount: 40.1 g (82.1 mmol), 84.4% yield}.
LRMS m/z 511 $[M+Na]^+$.

Route C5; 1-O-ally-2,3-di-O-benzyl-α-D-glucopyranoside (5-4):

The compound (5-3) (15.6 g, 32.0 mmol) was dissolved in acetic acid (90 mL), and further distilled water (50 mL) was added thereto. The solution was stirred for 1 hour while heated and refluxed. After the sufficient progress of the reaction was confirmed, the solution was cooled to room temperature, and then the solvent was removed by evaporation. Distilled water (15 mL) was added thereto, and the solution was again concentrated under reduced pressure; this operation was repeated 4 times. Thereafter, the resultant was purified by silica gel flash chromatography (hexane/ethyl acetate, 4:1→2:1→1:1→1:2) to yield the title compound (5-4) {12.1 g (30.2 mmol), 94.5% yield}.
LRMS m/z 423 $[M+Na]^+$.

Route D5; 1-O-ally-2,3,4-di-O-benzyl-6-O-tosyl-α-D-glucopyranoside (5-5):

To a solution of the compound (5-4) (12.1 g, 30.2 mmol) in anhydrous pyridine (120 mL) were added p-toluenesulfonyl chloride (7.5 g, 39.3 mmol) and 4-dimethylaminopyridine (369 mg, 3.0 mmol), and the reaction solution was stirred at 0°C for 16 hours. After the sufficient progress of the reaction was confirmed, the reaction solution was slowly poured into ice water (100 mL). The water layer was subjected to extraction with ethyl acetate (3 × 200 mL). The organic layers were combined with each other. The combination was washed with an aqueous 1 N HCl solution until the pH became 4, washed with saturated aqueous sodium hydrogen carbonate (2 × 100 mL) and saturated saline (2 × 100 mL), dried over sodium sulfate, filtrated and then concentrated under reduced pressure. The concentrated product was purified by silica gel flash chromatography (hexane/ethyl acetate, 6:2→4:1→2:1) to yield the title compound (5-5) {15.3 g (27.6 mmol), 91.4% yield}.
LRMS m/z 577 $[M+Na]^+$.

Route E5; 1-O-allyl-2,3-di-O-benzyl-4-O-(carbobenzoxy-β-alanyl)-6-O-tosyl-α-D-glucopyranoside (5-6):

Into a mixed solution of anhydrous dichloromethane (200 mL) and anhydrous pyridine (50 mL) were dissolved the compound (5-5) (15.3 g, 27.6 mmol), N-carbobenzoxy-β-alanine (12.32 g, 55.2 mmol), 1-ethyl-3-(3-dimethylaminopropyl)carbodiimide hydrochloride (EDCI·HCl) (15.8 mg, 82.8 mmol) and 4-dimethylaminopyridine (6.7 g, 55.2 mmol),

and then the reactive components were caused to react with each other at room temperature for 18 hours. After the sufficient progress of the reaction was confirmed, water (10 mL) was poured into the reaction solution to terminate the reaction. Thereafter, the solution was concentrated under reduced pressure. The resultant residue was purified by silica gel chromatography (hexane/ethyl acetate, 4:1→3:1→2:1→3:2) to yield the title compound (5-6) {17.1 g (22.5 mmol), 81.5% yield}.
LRMS m/z 782 $[M+Na]^+$.

Route F5; 1-O-allyl-2,3-di-O-benzyl-4-O-(carbobenzoxy-β-alanyl)-6-thioacetyl-α-D-glucopyranoside (5-7):

Potassium thioacetate (5.1 g, 45.0 mmol) was added to a solution of the compound (5-6) (17.1 g, 22.5 mmol) in anhydrous N,N-dimethylformamide (300 mL), and the solution was stirred at 90°C for 3 hours. After the sufficient progress of the reaction was confirmed, the reaction solution was poured into cold water (400 mL), and the resultant was extracted with ethyl acetate (3 × 150 mL). The organic layers were combined with each other, and the combination was washed with saturated saline (2 × 100 mL), dried over sodium sulfate, filtrated and concentrated under reduced pressure. The resultant residue was purified by silica gel chromatography (hexane/ethyl acetate, 4:1→3:1→2:1→3:2) to yield the title compound (5-7) {14.2 g (20.3 mmol), 90.0% yield}.
LRMS m/z 686 $[M+Na]^+$.

Route G5; 3-O-[2,3-di-O-benzyl-4-O-(carbobenzoxy-β-alanyl)-6-thioacetyl-α-D-quinovopyranosyl-glycerol (5-8):

The compound (5-7) (14.2 g, 20.4 mmol) was dissolved in a solution (200 mL) of t-butyl alcohol and distilled water (4:1), and then thereto were added a 0.04 M osmium tetraoxide solution in t-butyl alcohol (3 mL) and trimethylamine N-oxide (3.4 g, 30.5 mmol). The solution was stirred with a stirrer at room temperature for 24 hours. After the sufficient progress of the reaction was confirmed, 3 g of activated carbon was added thereto and then the solution was stirred for 30 minutes to cause the catalyst to be adsorbed on the carbon. The solution was subjected to suction filtration through a Kiriyama funnel containing celite, so as to remove the catalyst. The reaction product remaining on the celite was washed three times with ethyl acetate so as to be collected. Distilled water (200 mL) was added to the collected filtrate, and the resultant was extracted with ethyl acetate (3 × 150 mL). The organic layers were combined with each other, and the combination was washed with saturated saline (2 × 100 mL), dried over sodium sulfate, filtrated and concentrated under reduced pressure. The resultant residue was purified by silica gel chromatography (hexane/ethyl acetate, 2:1→1:1→2:3→1:2→1:4→1:8) to yield the title compound (5-8) {13.2 g (18.9 mmol), 93.0% yield}.
LRMS m/z 720 $[M+Na]^+$.

Route H5; 3-O-[2,3-di-O-benzyl-4-O-(carbobenzoxy-β-alanyl)-6-thioacetyl-α-D-quinovopyranosyl]-1,2-di-O-stearoyl-glycerol (5-9):

Stearoyl chloride (8.5 g, 28.2 mmol) was added to a mixed solution of the compound (5-8) (13.1 g, 18.8 mmol) in anhydrous dichloromethane (200 mL) and anhydrous pyridine (50 mL), and then the solution was stirred at room temperature for 2 hours. After the sufficient progress of the reaction was confirmed, methanol (5 mL) was added thereto so as to terminate the reaction. The solution was concentrated under reduced pressure. A small amount of ethyl acetate was used to suspend the residue, and the suspended residue was poured into water (200 mL). The resultant was extracted with ethyl acetate (3 × 100 mL). The organic layers were combined with each other, and the combination was washed with saturated saline (2 × 100 mL), dried over sodium sulfate, filtrated and then concentrated under reduced pressure. The resultant residue was purified by silica gel chromatography (hexane/ethyl acetate, 6:1→4:1→2:1→3:2→1:1) to yield the title compound (5-9) as a colorless oily substance {12.2 g (9.9 mmol), 52.8% yield}.
LRMS m/z 1253 $[M+Na]^+$.

Route I5; 3-O-[2,3-di-O-benzyl-4-O-(carbobenzoxy-β-alanyl)-6-sulfo-α-D-quinovopyranosyl]-1,2-di-O-stearoyl-glycerol (5-10):

Oxone (24.4 g, 39.7 mmol) and potassium acetate (3.8 g) were added to a solution of the compound (5-9) (12.2 g, 9.9 mmol) in acetic acid (150 g, 9.9 mol), and then the solution was vigorously stirred at 45°C for 48 hours. After the sufficient progress of the reaction was confirmed, the reaction solution was poured into a cold 7.5 M sodium hydroxide solution (500 mL) and the resultant was extracted with ethyl acetate (4 × 100 mL). The organic layers were combined with each other, and the combination was washed with saturated aqueous sodium hydrogen carbonate (2 × 100 mL) and saturated saline (2 × 100 mL), dried over sodium sulfate, filtrated and then concentrated under reduced pressure. The resultant residue was purified by silica gel chromatography (chloroform/methanol, 100:1→50:1→20:1→15:1→12:1) to yield the title compound (5-10) as a colorless waxy substance {8.6 g (6.97 mmol), 70.3% yield}.

LRMS m/z 1235 [M-Na]-.

Route J5; 3-O-[4-O-(β-alanyl)-6-sulfo-α-D-quinovopyranosyl]-1,2-di-O-stearoyl-glycerol sodium salt (5-11):

To a solution of the compound (5-10) (480.8 mg, 382 μmol) in methanol (20 mL), chloroform (10 mL) and acetic acid (0.5 mL) was added 10% palladium-activated carbon (300 mg), and then the solution was stirred at room temperature under an atmosphere of hydrogen gas for 48 hours. After the sufficient progress of the reaction was confirmed, the palladium-activated carbon was filtrated off. The filtrate was concentrated under reduced pressure. The resultant residue was purified by silica gel chromatography (chloroform/methanol/distilled water, 100:25:3→65:25:4→65:35:5→65:45:6) to yield the title compound (5-11).
LRMS m/z 921 [M-Na]-.

Route K5; 3-O-[4-O-(4-iodobenzoyl-β-alanyl)-6-sulfo-α-D-quinovopyranosyl]-1,2-di-O-stearoyl-glycerol sodium salt (5-12):

The compound (5-11) was dissolved in a mixed solution of anhydrous dichloromethane (15 mL), pyridine (5 mL) and triethylamine (2 mL), and thereto was added (4-iodobenzoyl) p-nitrophenyl ester (635 mg, 1.72 mmol). While the solution was stirred with a stirrer, the reactive components were caused to react with each other at room temperature for 24 hours. After the sufficient progress of the reaction was confirmed, toluene and methanol were added to the solution. Under reduced pressure, the solvents were then removed by evaporation to concentrate the solution, while azeotropy. The resultant residue was purified by silica gel chromatography (chloroform/methanol/distilled water, 80:10:0.8→70:10:1.0→65:10:1.0→60:10:1.0) to yield the title compound (5-12) {279 mg (238 μmol), 55.2% yield, which was the yield through the two reactions J5 and K5}.
LRMS m/z 1151 [M - Na]-.

Synthesis Example 6

A process for producing the sulfopyranosylacylglycerol derivative according to an embodiment of the present invention is illustrated in the following scheme 6, giving an α-sulfoquinovosylacylglycerol monoiodide derivative as an example thereof:

[Scheme 6]

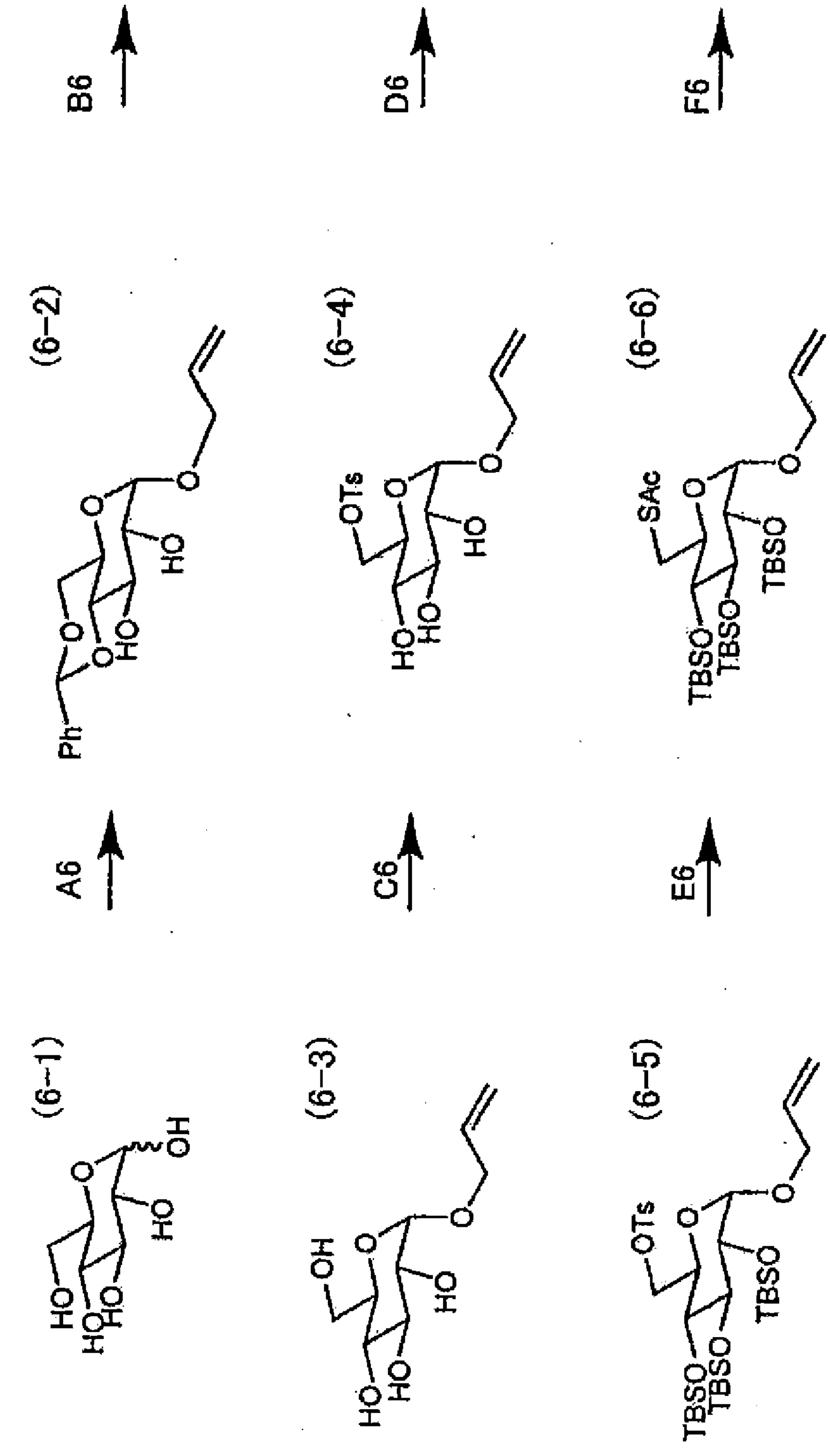


(6-1) (6-2) (6-3) (6-4) (6-5) (6-6)

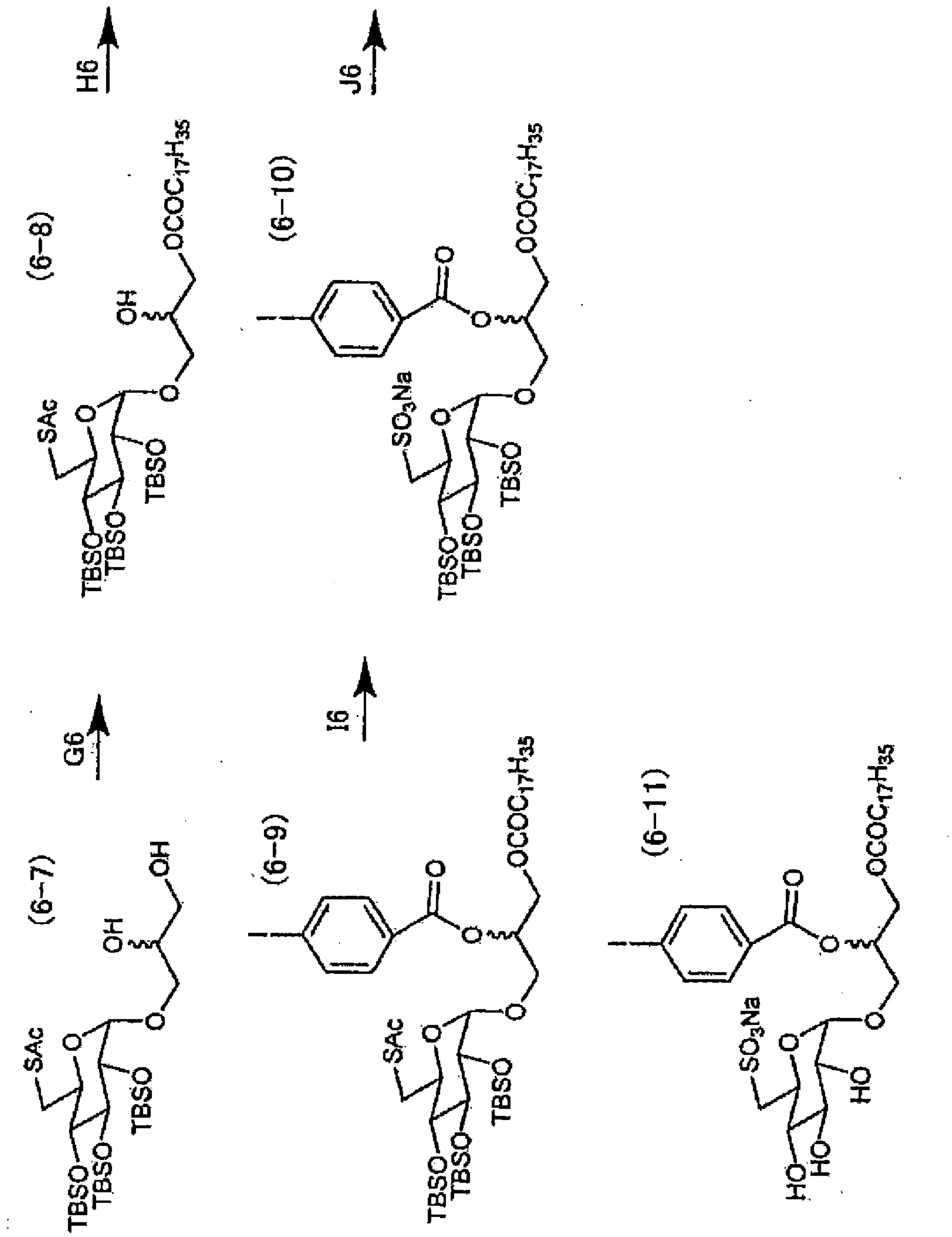


(6-7)

(6-8)

(6-9)

(6-10)

(6-11)

The following will describe a synthesis example through the individual steps in detail:

<Synthesis Example>

Route A6; 1-O-allyl-4,6-O-benzylidene-α-D-glucopyranoside (6-2):

Trifluoromethanesulfonic acid (1.00 mL) was added to a liquid suspension of the compound (6-1) (100 g, 555 mmol) in allyl alcohol (500 mL) at 0°C, and the reaction liquid was vigorously stirred at 80°C for 48 hours. After the sufficient progress of the reaction was confirmed, thereto was added triethylamine (3 mL) to terminate the reaction. The resultant was concentrated under reduced pressure. Next, the residue was suspended in anhydrous acetonitrile (500 mL), and thereto were added benzaldehydedimethylacetal (127 g, 1.5 equivalent) and p-toluenesulfonic acid monohydrate (5.28 g, 0.05 equivalent). The resultant liquid was stirred at 40°C for 4 hours, and then thereto was added triethylamine (10 mL) to terminate the reaction. The resultant was concentrated under reduced pressure. The residue was poured into hexane (2000 mL) and water (500 mL), and then the mixed liquid was vigorously stirred. The resultant precipitate was filtrated, and rinsed with water and hexane. The precipitate was crystallized two times from heated ethanol to yield the title compound (6-2) as a colorless needle crystal {34.5 g (112 mmol), 20.2% yield}.
LRMS m/z 331 [M+NaI$^+$.

Route B6; 1-O-(2-propenyl)-α-D-glucose (6-3):

The compound (6-2) (10.7 g, 34.7 mmol) was dissolved in a solution (260 mL) of acetic acid and water (8:5), and the reactive components were caused to react with each other at 100°C for 1 hour. The solution was concentrated under reduced pressure, and purified by silica gel flash chromatography (dichloromethane/methanol, 6:1) to yield the title compound (6-3) (6.3 g (28.6 mmol), 82.4% yield).

Route C6; 1-O-(2-propenyl)-6-O-(4-tolylsulfonyl)-α-D-glucose (6-4):

The compound (6-3) (6.3 g, 28.6 mmol) was dissolved in dry pyridine (200 mL), and thereto were added p-dimethylaminopyridine (DMAP) (195 mg), and p-toluenesulfonyl chloride (7.0 g). While the solution was stirred, the reactive components were caused to react with each other at room temperature for 16 hours. Thereafter, thereto was added cold distilled water (20 mL) to terminate the reaction. The resultant was extracted with ethyl acetate (3 × 200 mL), and the organic layers were combined with each other. The combination was neutralized to a pH of 4 with 1.0 M hydrochloric acid and 0.1 M hydrochloric acid, washed with saturated saline (2 × 200 mL), dried over anhydrous sodium sulfate, filtrated, concentrated under reduced pressure, and then purified by silica gel flash chromatography (dichloromethane/methanol, 20:1) to yield the title compound (6-4) (8.6 mg (23.0 mmol), 83.8% yield).

Route D6; 2,3,4-tri-O-(t-butyldimethylsilyl)-1-O-(2-propenyl)-6-O-(4-tolylsulfonyl)-α-D-glucose (6-5):

The compound (6-4) (11.2 g, 29.9 mmol) was dissolved in dry dichloromethane (25 mL), and then thereto were added t-butyldimethylsilyltrifluoromethane sulfonate (23.8 g) and 2,6-lutidine (14.4 g). While the solution was stirred under the flow of nitrogen gas, the reaction components were caused to react with each other for 16 hours. Thereafter, dichloromethane (150 mL) was added thereto so as to terminate the reaction. The solution was washed with saturated saline (2 × 100 mL), dried over anhydrous sodium sulfate, filtrated, concentrated under reduced pressure, and then purified by silica gel flash chromatography (hexane/ethyl acetate, 30:1) to yield the title compound (6-5) (19.6 g (27.4 mmol), 91.6% yield).

Route E6; 2,3,4-tri-O-(t-butyldimethylsilyl)-1-O-(2-propenyl)-6-deoxy-6-acetylthio-α-D-glucose (6-6):

The compound (6-5) (7.9 g, 11.0 mmol) was dissolved in dry ethanol (20 mL), and thereto were added potassium thioacetate (1.8 g). While the solution was stirred under reflux conditions, the reactive components were caused to react with each for 3 hours. Thereafter, thereto was added cold distilled water (100 mL) to terminate the reaction. The resultant was extracted with ethyl acetate (3 × 200 mL), and the organic layers were combined with each other. The combination was washed with saturated saline (2 × 200 mL), dried over anhydrous sodium sulfate, filtrated, concentrated under reduced pressure, and then purified by silica gel flash chromatography (hexane/ethyl acetate, 50:1) to yield the title compound (6-6) (5.6 g (9.02 mmol), 82.0% yield).

Route F6; 3-O-[2,3,4-tri-O-(t-butyldimethylsilyl)-6-deoxy-6-acetylthio-α-D-glucopyranosyl]-glycerol (6-7):

The compound (6-6) (5.6 g, 9.02 mmol) was dissolved in a solution of t-butyl alcohol and water (4:1), and then thereto were added trimethylamine N-oxide dihydrate (1.5 g) and a 0.04 M osmium tetraoxide solution in t-butyl alcohol

(15 mL). While the solution was stirred, the reactive components were caused to react with each other at room temperature for 22 hours. Thereafter, activated carbon (15 g) was added thereto. While stirred, the solution was allowed to stand at room temperature for 1.5 hours to cause osmium tetraoxide to be adsorbed thereon. The solution was then subjected to suction filtration. Next, cold distilled water (200 mL) was added thereto so as to terminate the reaction. The resultant was extracted with ethyl acetate (3 × 200 mL). The organic layers were combined with each other, and the combination was washed with saturated saline (2 × 300 mL), dried over anhydrous sodium sulfate, filtrated, concentrated under reduced pressure, and then purified by silica gel chromatography (hexane/ethyl acetate, 3:1→1:2) to yield the title compound (6-7) {5.2 g (7.94 mmol), 88.0% yield}.

Route G6; 3-O-[2,3,4-O-tert-butyldimethylsilyl-6-thioacetyl-α-D-quinovopyranosyl]-1-O-stearoyl-glycerol (6-8):

Stearoyl chloride (692 mg, 2.3 mmol) was added to a mixed solution of the compound (6-7) (1.4 g, 6.1 mmol) in anhydrous dichloromethane (20 mL) and anhydrous pyridine (5 mL), and then the solution was stirred at room temperature for 2 hours. After the sufficient progress of the reaction was confirmed, methanol (1 mL) was added thereto so as to terminate the reaction. The solution was concentrated under reduced pressure. A small amount of ethyl acetate was used to suspend the residue, and the suspended residue was poured into water (20 mL). The resultant was extracted with ethyl acetate (3 × 20 mL). The organic layers were combined with each other, and the combination was washed with saturated saline (2 × 20 mL), dried over sodium sulfate, filtrated and then concentrated under reduced pressure. The resultant residue was purified by silica gel chromatography (hexane/ethyl acetate, 10:1→8:1→6:1→4:1) to yield the title compound (6-8) as a colorless oily substance {1.6 g (1.7 mmol), 83.6% yield}.
LRMS m/z 944 $[M+Na]^+$.

Route H6; 3-O-[2,3,4-O-tert-butyldimethylsilyl-6-thioacetyl-α-Dquinovopyranosyl]-1-O-stearoyl-2-O-(4-iodobenzoyl)-glycerol (6-9):

4-Benzoyl iodochloride (555 mg, 2.1 mmol) was added to a mixed solution of the compound (6-8) (1.6 g, 1.7 mmol) in anhydrous dichloromethane (20 mL) and anhydrous pyridine (5 mL), and then the solution was stirred at room temperature for 2 hours. After the sufficient progress of the reaction was confirmed, methanol (1 mL) was added thereto so as to terminate the reaction. The solution was concentrated under reduced pressure. A small amount of ethyl acetate was used to suspend the residue, and the suspended residue was poured into water (20 mL). The resultant was extracted with ethyl acetate (3 × 20 mL). The organic layers were combined with each other, and the combination was washed with saturated saline (2 × 20 mL), dried over sodium sulfate, filtrated and then concentrated under reduced pressure. The resultant residue was purified by silica gel chromatography (hexane/ethyl acetate, 12:1→10:1→8:1→6:1→5:1→4:1) to yield the title compound (6-9) as a colorless oily substance {1.7 g (1.5 mmol), 85.0% yield}.
LRMS m/z 1174 $[M+Na]^+$.

Route 16; 3-O-[2,3,4-O-tert-butyldimethylsilyl-6-sulfo-α-D-quinovopyranosyl]-1-O-stearoyl-2-O-(4-iodobenzoyl)-glycerol (6-10):

Oxone (2.7 g, 4.4 mmol) and potassium acetate (1.25 g) were added to a solution of the compound (6-9) (1.7 g, 1.5 mmol) in acetic acid (50 mL), and then the solution was vigorously stirred at room temperature for 48 hours. After the sufficient progress of the reaction was confirmed, ethyl acetate (200 mL) was poured into the reaction solution. The solution was washed with saturated saline (2 × 50 mL), dried over sodium sulfate, and filtrated. Thereafter, ethyl acetate was removed therefrom under reduced pressure. The resultant residue containing acetic acid was obtained as the title compound (6-10), and was to be used as it was for the next reaction.

Route J6; 3-O-[6-sulfo-α-D-quinovopyranosyl]-1-O-stearoyl-2-O-(4-iodobenzoyl)-glycerol (6-11):

Tetrahydrofuran (1.5 mL) and water (1.5 mL) were added to the mixed solution containing the compound (6-10) and acetic acid, and further trifluoroacetic acid (0.3 mL) was added thereto. The solution was then stirred at room temperature for 48 hours. After the sufficient progress of the reaction was confirmed, the solution was concentrated under reduced pressure. The resultant residue was purified by silica gel chromatography (chloroform/methanol/distilled water, 100:10:0.5→80:10:0.5→60:10:1→40:10:1→60:25:2) to yield the title compound (6-11) {752 mg (899 μmol) through the two reactions in the routes I6 and J6, 60.9% yield}.
LRMS m/z 813 $[M-Na]^-$.

Synthesis Example 7

A process for producing the sulfopyranosylacylglycerol derivative according to an embodiment of the present invention is illustrated in the following scheme 7, giving an α-sulfoquinovosylacylglycerol triiodide derivative as an example thereof:

[Scheme 7]

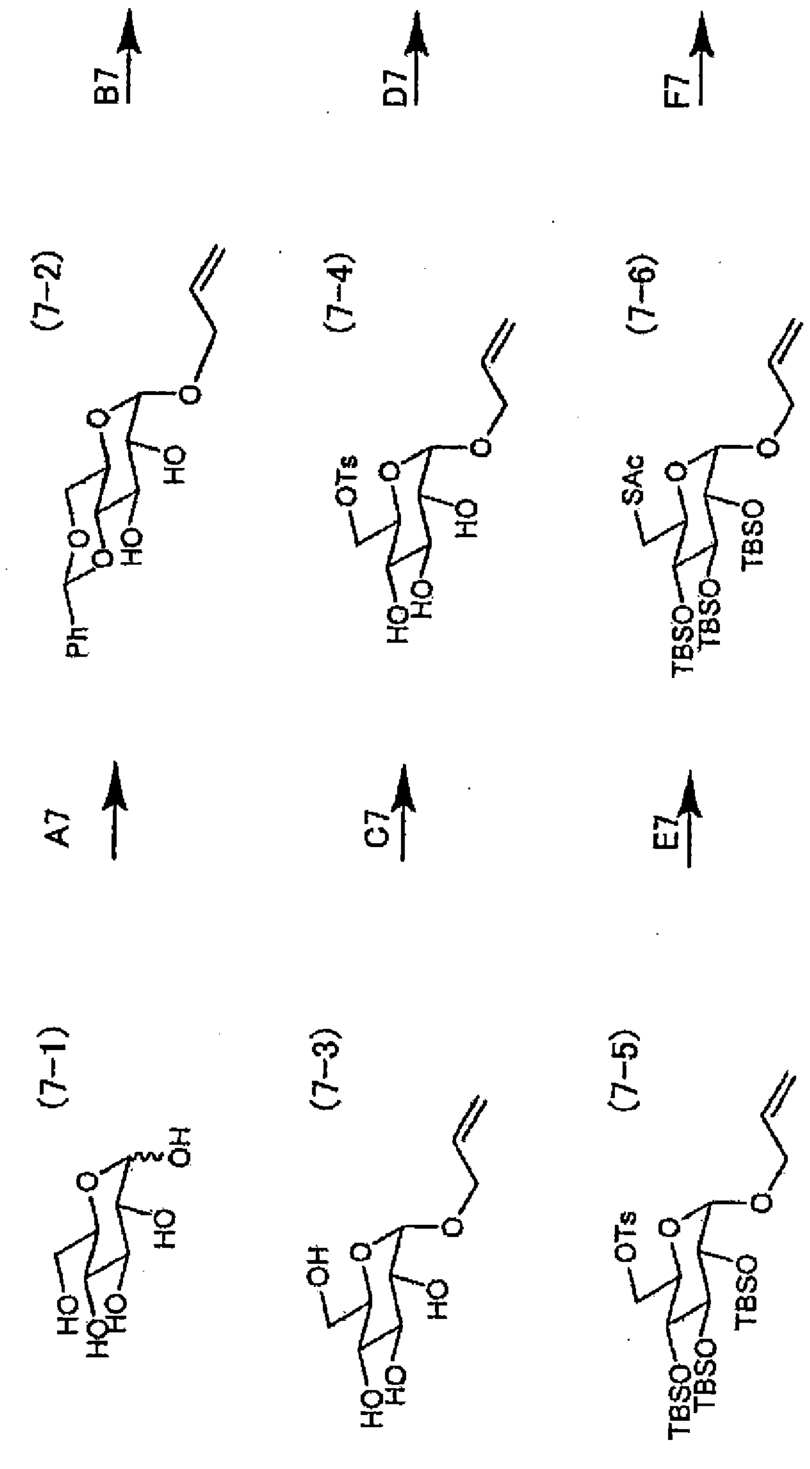


(7-1) (7-2) (7-3) (7-4) (7-5) (7-6)

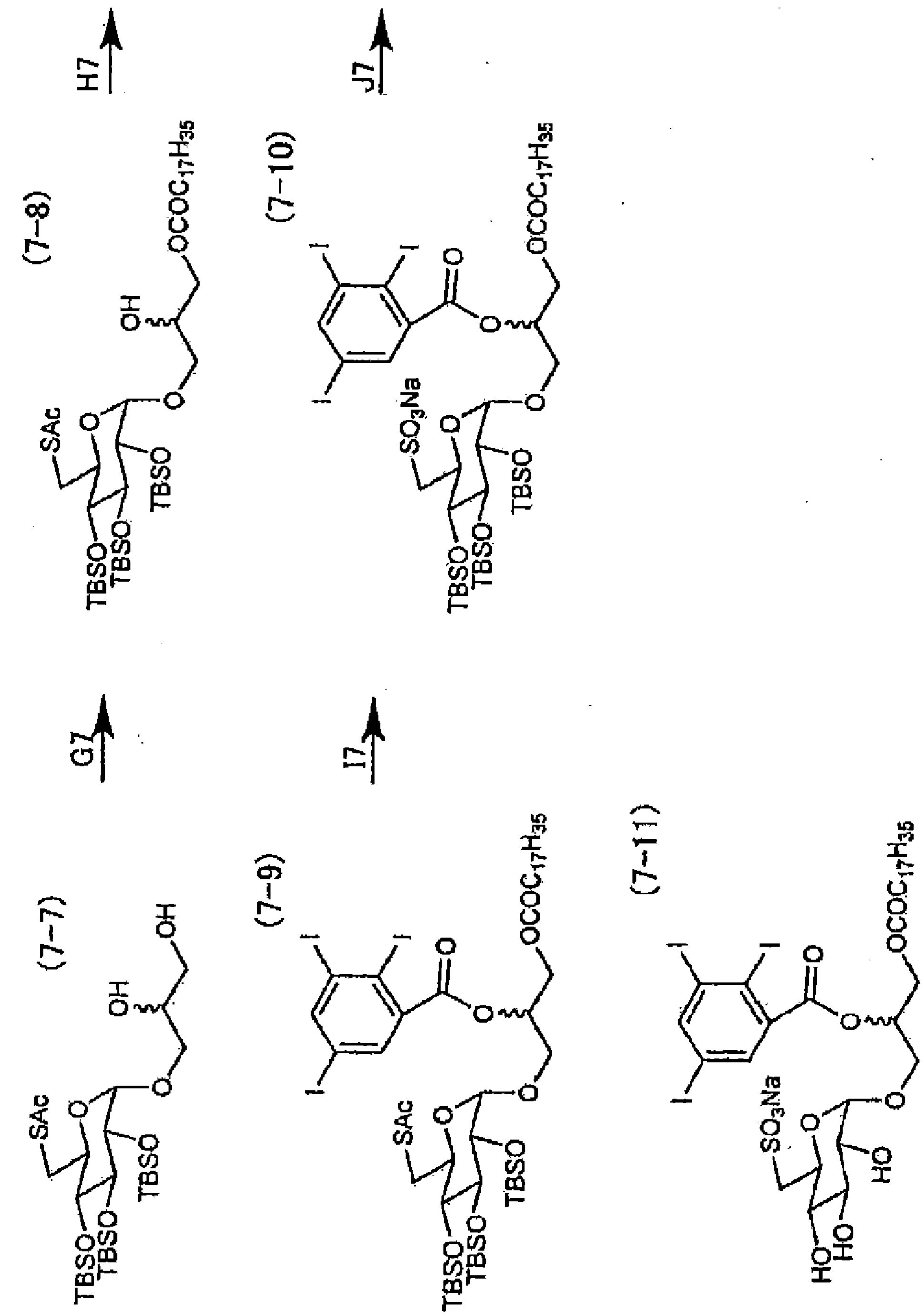


(7-7) (7-8) (7-9) (7-10) (7-11)

The following will describe a synthesis example through the individual steps in detail:

<Synthesis Example>

The compound (7-8) was yielded by the same process as in the routes A6 to G6 of Synthesis Example 6.

Route H7; 3-O-[2,3,4-O-tert-butyldimethylsilyl-6-thioacetyl-α-Dquinovopyranosyl]-1-O-stearoyl-2-O-(2,3,5-triiodobenzoyl)-glycerol (7-9):

Into anhydrous dichloromethane (10 mL) were dissolved the compound (7-8) (467 mg, 507 μmol), 2,3,5-triiodobenzoic acid (303.8 mg, 608 μmol), di-2-pyridyl carbonate (DPC) (219 mg, 1013 μmol), and 4-dimethylaminopyridine (6.2 mg, 51 μmol). The reactive components were caused to react with each other at room temperature for 48 hours. After the sufficient progress of the reaction was confirmed, water (30 mL) was poured into the reaction solution to terminate the reaction. The resultant was then extracted with dichloromethane (3 × 20 mL). The organic layers were combined with each other, and the combination was washed with saturated saline (2 × 20 mL), dried over sodium sulfate, filtrated and then concentrated under reduced pressure. The resultant residue was purified by silica gel chromatography (hexane/ethyl acetate, 12:1→10:1→8:1→6:1→5:1→4:1) to yield the title compound (7-9) as a colorless oily substance {245 mg (174 μmol), 34.4% yield}.
LRMS m/z 1425 $[M + Na]^-$.

Route I7; 3-O-[2,3,4-O-tert-butyldimethylsilyl-6-sulfo-α-D-quinovopyranosyl]-1-O-stearoyl-2-O-(2,3,5-triiodobenzoyl)-glycerol (7-10):

Oxone (322 mg, 523 μmol) and potassium acetate (250 mg) were added to a solution of the compound (7-9) (245 mg, 174 μmol) in acetic acid (10 mL), and then the solution was vigorously stirred at room temperature for 24 hours. After the sufficient progress of the reaction was confirmed, ethyl acetate (50 mL) was poured into the reaction solution. The solution was washed with saturated saline (2 × 20 mL), and the organic layer was dried over sodium sulfate, filtrated, and then concentrated under reduced pressure. The resultant residue containing acetic acid was to be used, as it was, as the title compound (7-10) for the next reaction.

Route J7; 3-O-[6-sulfo-α-D-quinovopyranosyl]-1-O-stearoyl-2-O-(2,3,5-triiodobenzoyl)-glycerol (7-11):

Tetrahydrofuran (5 mL), acetic acid (10 mL) and water (5 mL) were added to the mixed solution containing the compound (7-10) yielded through the route I7 and acetic acid, and further trifluoroacetic acid (1 mL) was added thereto. The solution was then stirred at room temperature for 48 hours. After the sufficient progress of the reaction was confirmed, the solution was concentrated under reduced pressure. The resultant residue was purified by silica gel chromatography (chloroform/methanol/distilled water, 100:10:0.5→80:10:0.5→60:10:1→40:10:1→60:25:2) to yield the title compound (7-11) {102 mg (94 μmol) through the two reactions in the routes I7 and J7, 53.7% yield}.
LRMS m/z 1065 $[M-Na]^-$.

Synthesis Example 8

A process for producing the sulfopyranosylacylglycerol derivative according to an embodiment of the present invention is illustrated in the following scheme 8, giving a β-sulfoquinovosylmonoacylglycerol monoiodide derivative as an example thereof:

[Scheme 8]

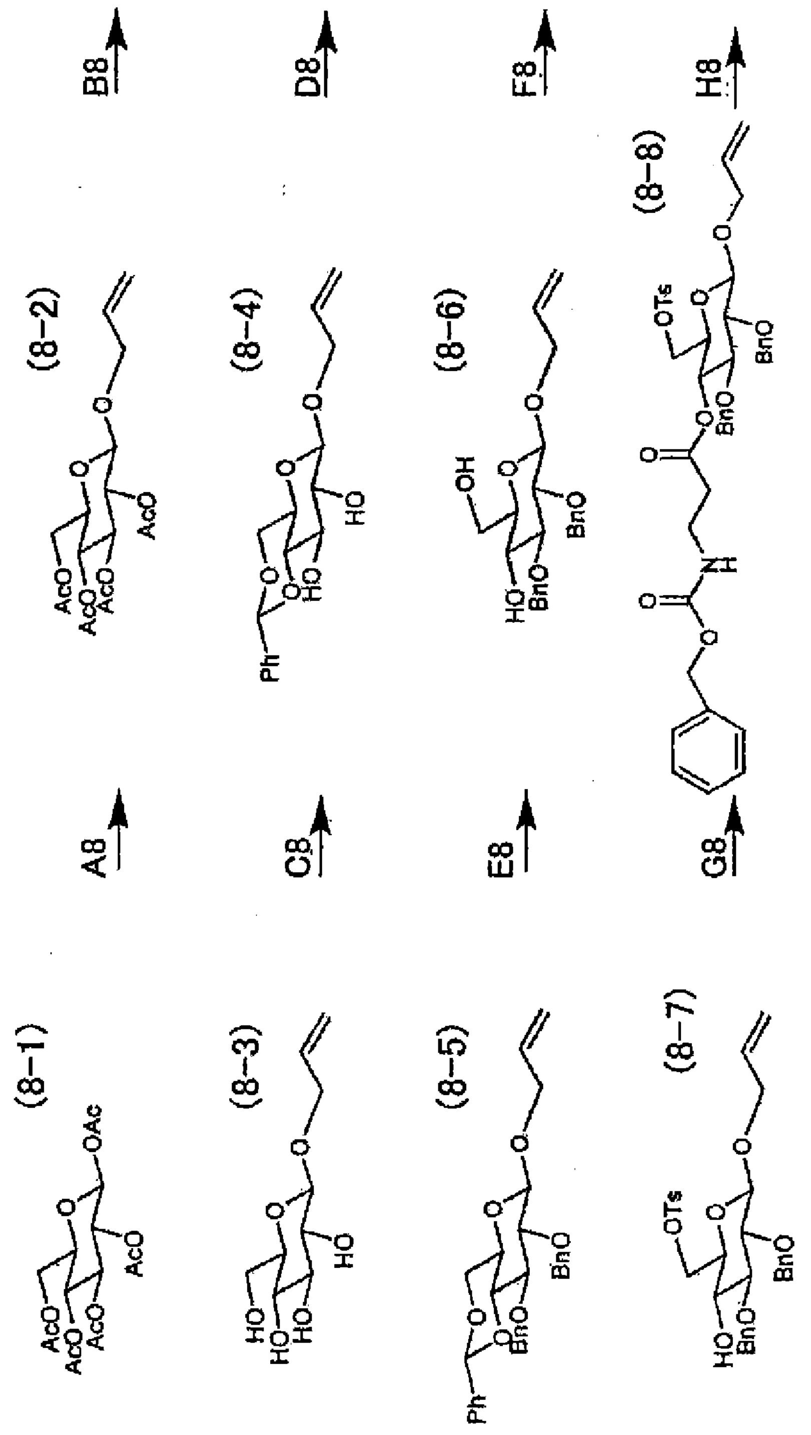


(8-1) (8-2) (8-3) (8-4) (8-5) (8-6) (8-7) (8-8)

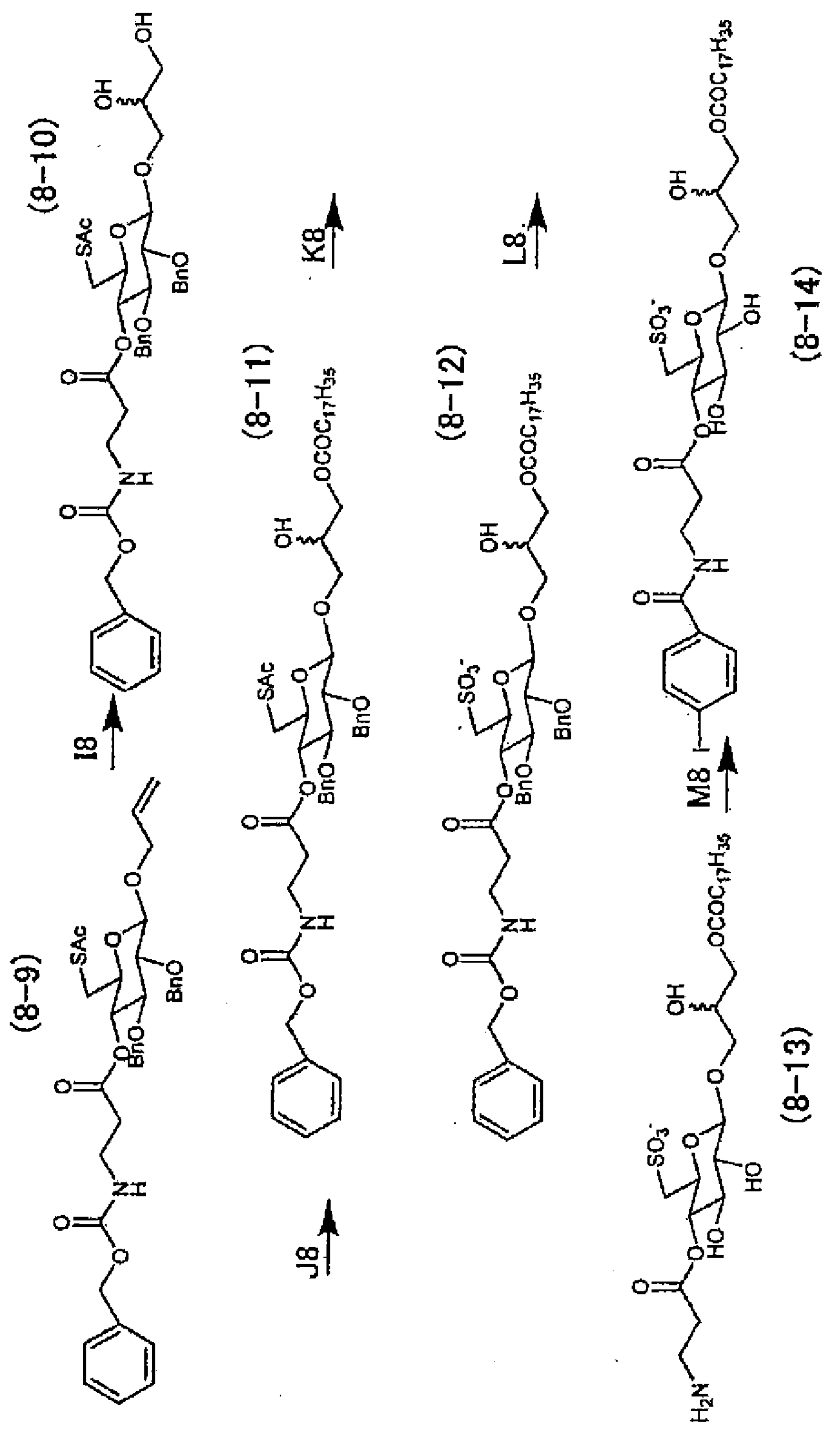


(8-9) (8-10) (8-11) (8-12) (8-13) (8-14)

The following will describe a synthesis example through the individual steps in detail:

<Synthesis Example>

Route A8; 2,3,4,6-tetra-O-acetyl-1-O-allyl-β-D-glucopyranoside (8-2):

A solution (500 mL) of the compound (8-1) (50.0 g, 128 mmol), allyl alcohol (22.3 g, 384 mmol), and zinc chloride (17.4 g, 128 mmol) in toluene was vigorously stirred at 80°C under an atmosphere of nitrogen for 48 hours. After the sufficient progress of the reaction was confirmed, the temperature of the solution was returned to room temperature. The solution was then washed with saturated aqueous sodium hydrogen carbonate (2 × 100 mL) and saturated saline (2 × 100 mL), dried over sodium sulphate, filtrated and then concentrated under reduced pressure. The concentrated product was crystallized two times from heated ethanol to yield the title compound (8-2) as a colorless needle crystal {24.8 g (64 mmol), 50.0% yield}.
LRMS m/z 411 $[M+Na]^+$.

Route B8; 1-O-allyl-β-D-glucopyranoside (8-3):

The compound (8-2) (21.0 g, 54 mmol) was dissolved in methanol (200 mL). While the solution was stirred at room temperature under an atmosphere of nitrogen, thereto was added a 28% solution (1 mL) of sodium methylate in methanol. The solution was then stirred at room temperature for 2 hours. After the sufficient progress of the reaction was confirmed, the reaction was terminated. The reaction solution was concentrated, as it was, under reduced pressure, and a crude product of the resultant title compound (8-3) was to be used for the next reaction.
LRMS m/z 243 $[M+Na]^+$.

Route C8; 1-O-allyl-4,6-O-benzylidene-β-D-glucopyranoside (8-4):

The compound (8-3) (11.9 g, 54 mmol) was suspended in anhydrous acetonitrile (60 mL), and thereto were added benzaldehyde dimethylacetal (16.4 g, 2.0 equivalent) and p-toluenesulfonic acid monohydrate (1.0 g, 0.05 equivalent). The reaction liquid was stirred at 40°C for 4 hours, and then triethylamine (0.5 mL) was added thereto so as to terminate the reaction. The resultant was concentrated under reduced pressure. The concentrated product was dissolved in a small amount of ethyl acetate, and then cold water (200 mL) was added thereto. The resultant was extracted with ethyl acetate (3 × 100 mL). The organic layers were combined with each other, and the combination was washed with saturated saline (2 × 50 mL), dried over sodium sulfate, filtrated and then concentrated under reduced pressure. The resultant residue was crystallized two times from heated ethanol to yield the title compound (8-4) as a colorless needle crystal. The filtrate was further concentrated, purified by silica gel chromatography (chloroform/methanol, 100:1→50:1→25:1→20:1), and then crystallized from heated ethanol to yield the same compound (8-4) {11.6 g (37.6 mmol), 69.6% yield}.
LRMS m/z 311 $[M+Na]^+$.

Route D8; 1-O-allyl-2,3-di-O-benzyl-4,6-O-benzylidene-β-D-glucopyranoside (8-5):

To a solution of the compound (8-4) (11.6 g, 37.6 mmol) in anhydrous N,N-dimethylformamide (DMF, 100 mL) were added benzylchloride (4.76 g, 4 equivalent) and sodium hydroxide powder (4.5 g, 3.0 equivalent), and the reaction solution was vigorously stirred at room temperature for 24 hours. After the sufficient progress of the reaction was confirmed, the reaction solution was poured into cold water (100 mL), and the resultant was extracted with ethyl acetate (3 × 50 mL). The organic layers were combined with each other, and the combination was washed with saturated saline (2 × 30 mL), dried over sodium sulfate, filtrated and then concentrated under reduced pressure. The resultant residue was crystallized two times from heated ethanol to yield the title compound (8-5) as a colorless needle crystal. The filtrate was concentrated, purified by silica gel chromatography (hexane/ethyl acetate, 12:1→10:1→8:1→6:1→4:1), and then crystallized from heated ethanol to yield the same compound (8-5). The resultant compound was collected into a flask, and was to be used as it was for the next reaction.

LRMS m/z 511 $[M+Na]^+$.

Route E8; 1-O-ally-2,3-di-O-benzyl-β-D-glucopyranoside (8-6):

The compound (8-5) yielded through the route D8 was dissolved in acetic acid (72 mL), and further distilled water (40 mL) was added thereto. The solution was stirred for 1 hour while heated and refluxed. After the sufficient progress of the reaction was confirmed, the solution was cooled to room temperature, and then the solvent was removed by evaporation. Distilled water (15 mL) was added thereto, and the solution was again concentrated under reduced

pressure; this operation was repeated 4 times. Thereafter, the resultant residue was dissolved in methanol (150 mL). While the solution was stirred at room temperature under an atmosphere of nitrogen, thereto was added a 28% solution (0.7 mL) of sodium methylate in methanol. The solution was then stirred at room temperature for 2 hours. After the sufficient progress of the reaction was confirmed, the reaction was terminated. The reaction solution was concentrated, as it was, under reduced pressure, and purified by silica gel flash chromatography (hexane/ethyl acetate, 4:1→3:1→2:1→3:2→1:1→2:3→1:2) to yield the title compound (8-6) {9.3 g (23.2 mmol), 61.7% yield, which was the yield through the two reactions in the routes D8 and E8}.
LRMS m/z 423 $[M+Na]^+$.

Route F8; 1-O-ally-2,3,4-di-O-benzyl-6-O-tosyl-β-D-glucopyranoside (8-7):

To a solution of the compound (8-6) (9.3 g, 23.2 mmol) in anhydrous pyridine (120 mL) were added p-toluenesulfonyl chloride (5.8 g, 30.2 mmol) and 4-dimethylaminopyridine (283 mg, 2.3 mmol), and the reaction solution was stirred at 0°C for 16 hours. After the sufficient progress of the reaction was confirmed, the reaction solution was slowly poured into ice water (100 mL). The water layer was subjected to extraction with ethyl acetate (3 × 200 mL). The organic layers were combined with each other. The combination was washed with an aqueous 1 N HCl solution until the pH became 4, washed with saturated aqueous sodium hydrogen carbonate (2 × 100 mL) and saturated saline (2 × 100 mL), dried over sodium sulfate, filtrated and then concentrated under reduced pressure. The concentrated product was purified by silica gel flash chromatography (hexane/ethyl acetate, 6:1→4:1→2:1) to yield the title compound (8-7) {12.3 g (22.2 mmol), 95.7% yield}.
LRMS m/z 577 $[M+Na]^+$.

Route G8; 1-O-allyl-2,3-di-O-benzyl-4-O-(carbobenzoxy-β-alanyl)-6-O-tosyl-β-D-glucopyranoside (8-8):

Into a mixed solution of anhydrous dichloromethane (200 mL) and anhydrous pyridine (50 mL) were dissolved the compound (8-7) (12.3 g, 22.2 mmol), N-carbobenzoxy-β-alanine (12.1 g, 44.4 mmol), 1-ethyl-3-(3-dimethylaminopropyl)carbodiimide hydrochloride (EDCI·HCl) (17.0 mg, 88.8 mmol) and 4-dimethylaminopyridine (5.4 g, 44.4 mmol), and then the reactive components were caused to react with each other at room temperature for 18 hours. After the sufficient progress of the reaction was confirmed, water (10 mL) was poured into the reaction solution to terminate the reaction. Thereafter, the solution was concentrated under reduced pressure. The resultant residue was purified by silica gel chromatography (hexane/ethyl acetate, 4:1→3:1→2:1→3:2) to yield the title compound (8-8) {15.7 g (20.7 mmol), 93.0% yield}.
LRMS m/z 782 $[M+Na]^+$.

Route H8; 1-O-allyl-2,3-di-O-benzyl-4-O-(carbobenzoxy-β-alanyl)-6-thioacetyl-β-D-glucopyranoside (8-9):

Potassium thioacetate (4.7 g, 41.3 mmol) was added to a solution of the compound (8-8) (15.7 g, 20.7 mmol) in anhydrous N,N-dimethylformamide (200 mL), and the solution was stirred at 90°C for 3 hours. After the sufficient progress of the reaction was confirmed, the reaction solution was poured into cold water (400 mL), and the resultant was extracted with ethyl acetate (3 × 150 mL). The organic layers were combined with each other, and the combination was washed with saturated saline (2 × 100 mL), dried over sodium sulfate, filtrated and concentrated under reduced pressure. The resultant residue was purified by silica gel chromatography (hexane/ethyl acetate, 4:1→3:1→2:1→3:2) to yield the title compound (8-9) {13.6 g (20.5 mmol), 99.2% yield}.
LRMS m/z 686 $[M+Na]^+$.

Route 18; 3-O-[2,3-di-O-benzyl-4-O-(carbobenzoxy-β-alanyl)-6-thioacetyl-β-D-quinovopyranosyl-glycerol (8-10) :

The compound (8-9) (13.6 g, 20.5 mmol) was dissolved in a solution (200 mL) of t-butyl alcohol and distilled water (4:1), and then thereto were added a 0.04 M osmium tetraoxide solution in t-butyl alcohol (5 mL) and trimethylamine N-oxide (3.4 g, 30.7 mmol). The solution was stirred with a stirrer at room temperature for 24 hours. After the sufficient progress of the reaction was confirmed, 3 g of activated carbon was added thereto and then the solution was stirred for 30 minutes to cause the catalyst to be adsorbed on the carbon. The solution was subjected to suction filtration through a Kiriyama funnel wherein celite was laid, so as to remove the catalyst. The reaction product remaining on the celite was washed three times with ethyl acetate so as to be collected. Distilled water (200 mL) was added to the collected filtrate, and the resultant was extracted with ethyl acetate (3 × 150 mL). The organic layers were combined with each other, and the combination was washed with saturated saline (2 × 100 mL), dried over sodium sulfate, filtrated and concentrated under reduced pressure. The resultant residue was purified by silica gel chromatography (toluene/ethyl acetate, 2:1→3:2→1:1→2:3→1:2→1:4) to yield the title compound (8-10) {11.7 g (16.8 mmol), 81.8% yield}.

LRMS m/z 720 $[M+Na]^+$.

Route J8; 3-O-[2,3-di-O-benzyl-4-O-(carbobenzoxy-β-alanyl)-6-thioacetyl-β-D-quinovopyranosyl]-1-O-stearoyl-glycerol (8-11):

Stearoyl chloride (5.4 g, 26.8 mmol) was added to a mixed solution of the compound (8-10) (11.7 g, 16.8 mmol) in anhydrous dichloromethane (200 mL) and anhydrous pyridine (50 mL), and then the solution was stirred at 0°C for 2 hours. After the sufficient progress of the reaction was confirmed, methanol (5 mL) was added thereto so as to terminate the reaction. The solution was concentrated under reduced pressure. A small amount of ethyl acetate was used to suspend the residue, and the suspended residue was poured into water (200 mL). The resultant was extracted with ethyl acetate (3 × 100 mL). The organic layers were combined with each other, and the combination was washed with saturated saline (2 × 100 mL), dried over sodium sulfate, filtrated and then concentrated under reduced pressure. The resultant residue was purified by silica gel chromatography (hexane/ethyl acetate, 6:1→9:1→2:1→3:2→1:1) to yield the title compound (8-11) as a colorless oily substance {10.7 g (11.1 mmol), 66.2% yield}.
LRMS m/z 987 $[M+Na]^+$.

Route K8; 3-O-[2,3-di-O-benzyl-4-O-(carbobenzoxy-β-alanyl)-6-sulfo-β-D-quinovopyranosyl]-1-O-stearoyl-glycerol (8-12):

Oxone (27.3 g, 44.4 mmol) and potassium acetate (3.7 g) were added to a solution of the compound (8-11) (10.7 g, 11.1 mmol) in acetic acid (147 g, 2.5 mol), and then the solution was vigorously stirred at room temperature for 48 hours. After the sufficient progress of the reaction was confirmed, the reaction solution was poured into a cold 7.5 M sodium hydroxide solution (500 mL) and the resultant was extracted with ethyl acetate (4 × 100 mL). The organic layers were combined with each other, and the combination was washed with saturated aqueous sodium hydrogen carbonate (2 × 100 mL) and saturated saline (2 × 100 mL), dried over sodium sulfate, filtrated and then concentrated under reduced pressure. The resultant residue was purified by silica gel chromatography (chloroform/methanol, 100:1→50:1→20:1→15:1→12.5:1→10:1→8:1) to yield the title compound (8-12) as a colorless waxy substance.
LRMS m/z 968 $[M-Na]^-$.

Route L8; 3-O-[4-O-(β-alanyl)-6-sulfo-β-D-quinovopyranosyl]-1-O-stearoyl-glycerol (8-13):

To a solution of the compound (8-12) (756 mg, 762 μmol) in methanol (10 mL), dichloromethane (10 mL) and acetic acid (0.5 mL) was added 10% palladium hydroxide-activated carbon (835 mg), and then the solution was stirred at room temperature under an atmosphere of hydrogen gas for 48 hours. After the sufficient progress of the reaction was confirmed, the palladium-activated carbon was filtrated off. The filtrate was concentrated under reduced pressure. The resultant residue (crude product of the compound (8-13)) was to be used as it was for the next reaction.
LRMS m/z 654 $[M-Na]^-$.

Route M8; 3-O-[4-O-(4-iodobenzoyl-β-alanyl)-6-sulfo-β-D-quinovopyranosyl]-1-O-stearoyl-glycerol (8-14):

The crude product of the compound (8-13) yielded through the route L8 was dissolved in a mixed solution of anhydrous dichloromethane (15 mL), pyridine (5 mL), and triethylamine (2 mL). Thereto was added (4-iodobenzoyl)-p-nitrophenyl ester (563 mg, 1.5 mmol). While the solution was stirred with a stirrer, the reactive components were caused to react with each other at room temperature for 24 hours. After the sufficient progress of the reaction was confirmed, toluene and methanol were added thereto. Under reduced pressure, the solvents were then removed by evaporation to concentrate the solution, while azeotropy. The resultant residue was purified by silica gel chromatography (chloroform/methanol/distilled water, 70:10:1→60:10:1.1→50:10:1.2→90:10:2→30:10:1.5→55:25:4) to yield the title compound (8-14) {442.5 mg (487.4 μmol), 64.0% yield}.
LRMS m/z 884 [M - Na]-.

Synthesis Example 9

A process for producing the sulfopyranosylacylglycerol derivative according to an embodiment of the present invention is illustrated in the following scheme 9, giving a β-sulfoquinovosyldiacylglycerol monoiodide derivative as an example thereof:

[Scheme 9]

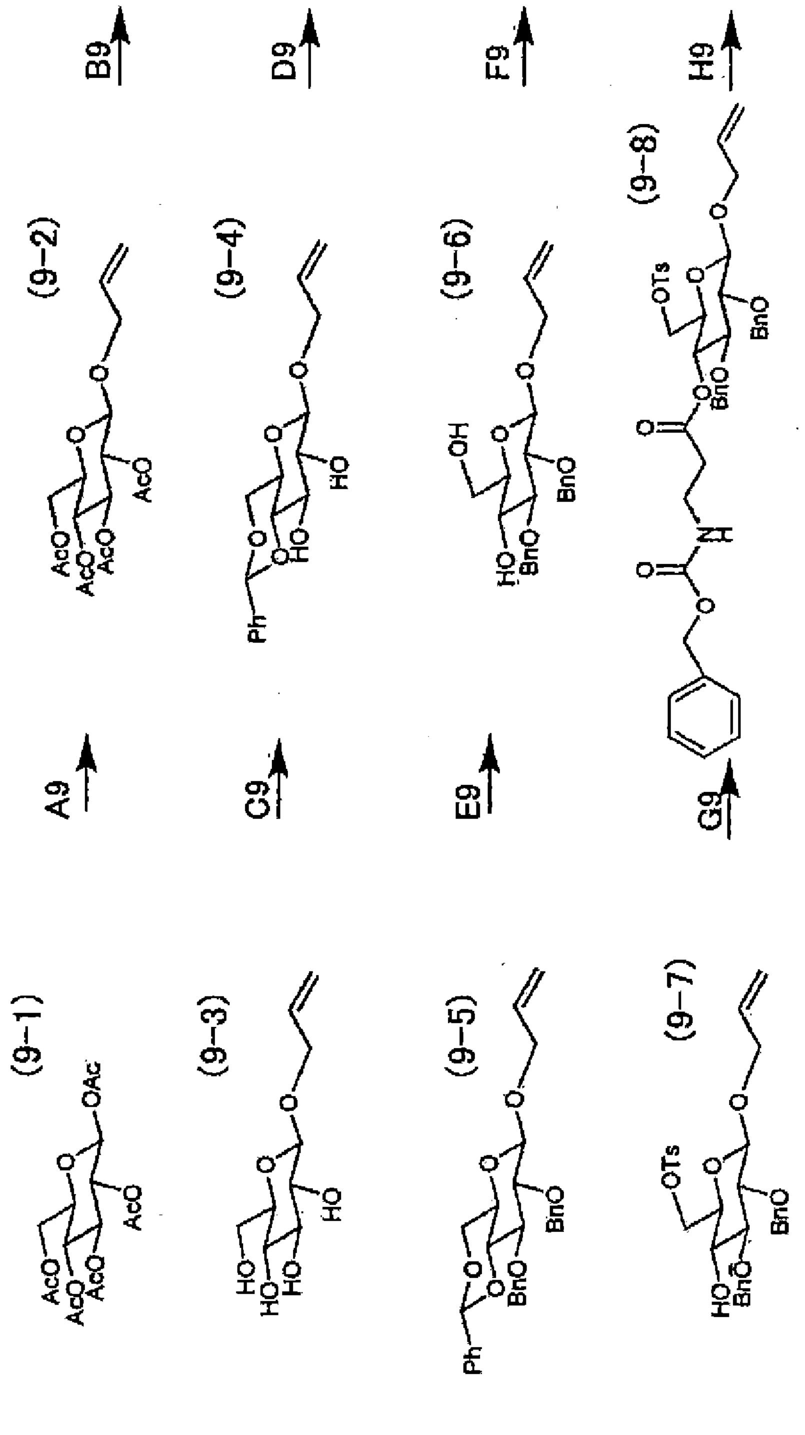

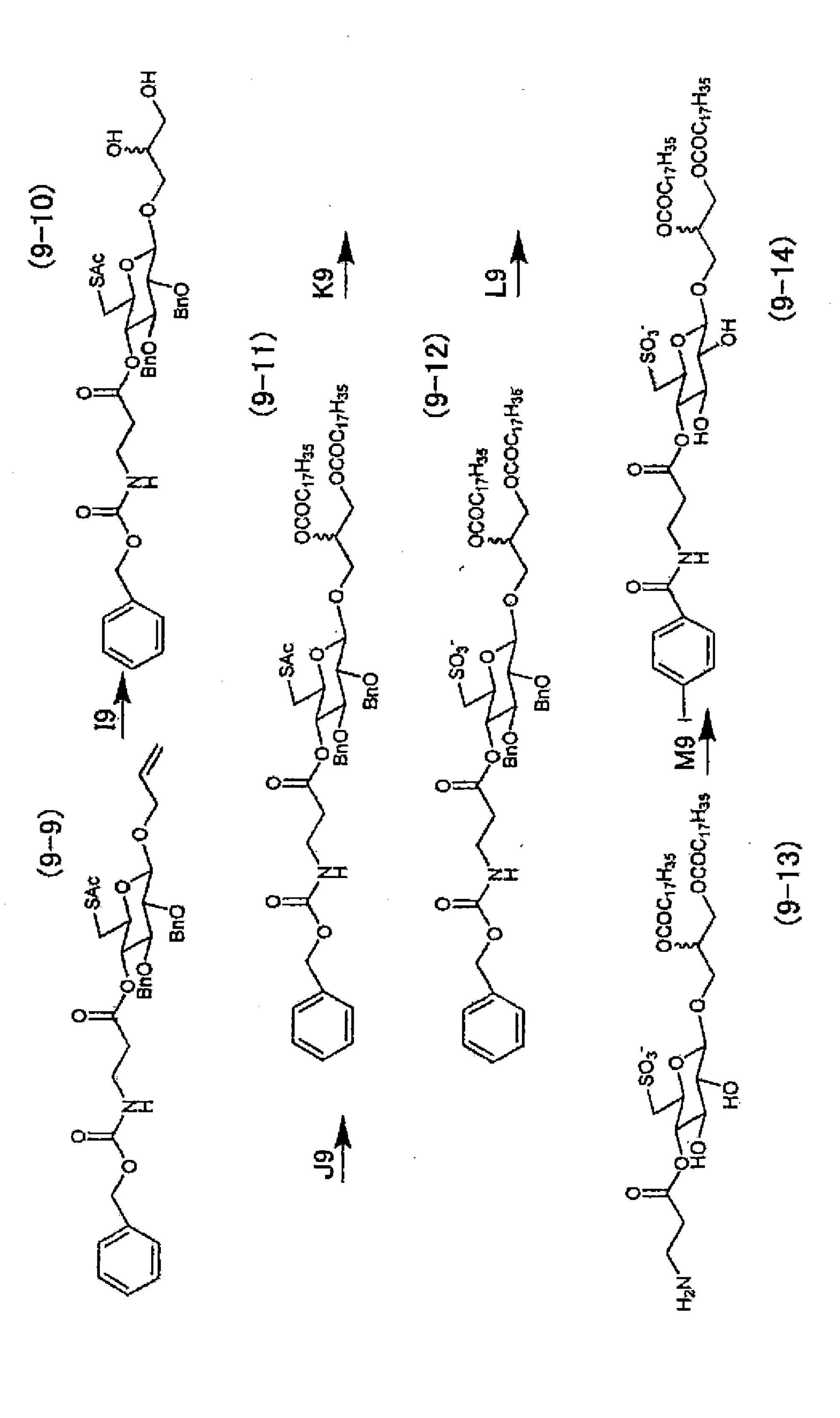


(9-9) (9-10) (9-11) (9-12) (9-13) (9-14)

The following will describe a synthesis example through the individual steps in detail:

<Synthesis Example>

Route A9; 2,3,4,6-tetra-O-acetyl-1-O-allyl-β-D-glucopyranoside (9-2):

A solution (500 mL) of the compound (9-1) (50.0 g, 128 mmol), allyl alcohol (22.3 g, 384 mmol), and zinc chloride (17.4 g, 128 mmol) in toluene was vigorously stirred at 80°C under an atmosphere of nitrogen for 48 hours. After the sufficient progress of the reaction was confirmed, the temperature of the solution was returned to room temperature. The solution was then washed with saturated aqueous sodium hydrogen carbonate (2 × 100 mL) and saturated saline (2 × 100 mL), dried over sodium sulphate, filtrated and then concentrated under reduced pressure. The concentrated product was crystallized two times from heated ethanol to yield the title compound (9-2) as a colorless needle crystal {24.8 g (64 mmol), 50.0% yield).
LRMS m/z 411 $[M+Na]^+$.

Route B9; 1-O-allyl-β-D-glucopyranoside (9-3):

The compound (9-2) (21.0 g, 54 mmol) was dissolved in methanol (200 mL). While the solution was stirred at room temperature under an atmosphere of nitrogen, thereto was added a 28% solution (1 mL) of sodium methylate in methanol. The solution was then stirred at room temperature for 2 hours. After the sufficient progress of the reaction was confirmed, the reaction was terminated. The reaction solution was concentrated, as it was, under reduced pressure, and a crude product of the resultant title compound (9-3) was to be used for the next reaction.
LRMS m/z 243 $[M+Na]^+$.

Route C9; 1-O-allyl-4, 6-O-benzylidene-β-D-glucopyranoside (9-4):

The compound (9-3) (11.9 g, 54 mmol) was suspended in anhydrous acetonitrile (60 mL), and thereto were added benzaldehyde dimethylacetal (16.4 g, 2.0 equivalent) and p-toluenesulfonic acid monohydrate (1.0 g, 0.05 equivalent). The reaction liquid was stirred at 40°C for 4 hours, and then triethylamine (0.5 mL) was added thereto so as to terminate the reaction. The resultant was concentrated under reduced pressure. The concentrated product was dissolved in a small amount of ethyl acetate, and then cold water was extracted with ethyl acetate (3 × 100 mL). The organic layers were combined with each other, and the combination was washed with saturated saline (2 × 50 mL), dried over sodium sulfate, filtrated and then concentrated under reduced pressure. The resultant residue was crystallized two times from heated ethanol to yield the title compound (9-4) as a colorless needle crystal (33.5 g). The filtrate was further concentrated, purified by silica gel chromatography (hexane/ethyl acetate, 15:1-10:1-8:1), and then crystallized from heated ethanol to yield the same compound (9-3) (6.63 g) {total amount: 40.1 g (82.1 mmol), 84.4% yield}.
LRMS m/z 511 $[M+Na]^+$.

Route D9; 1-O-allyl-2,3-di-O-benzyl-4, 6-O-benzylidene-β-D-glucopyranoside (9-5):

To a solution of the compound (9-4) (11.6 g, 37.6 mmol) in anhydrous N,N-dimethylformamide (DMF, 100 mL) were added benzylchloride (4.76 g, 4 equivalent) and sodium hydroxide powder (4.5 g, 3.0 equivalent), and the reaction solution was vigorously stirred at room temperature for 24 hours. After the sufficient progress of the reaction was confirmed, the reaction solution was poured into cold water (100 mL), and the resultant was extracted with ethyl acetate (3 × 50 mL). The organic layers were combined with each other, and the combination was washed with saturated saline (2 × 30 mL), dried over sodium sulfate, filtrated and then concentrated under reduced pressure. The resultant residue was crystallized two times from heated ethanol to yield the title compound (9-5) as a colorless needle crystal. The filtrate was concentrated, purified by silica gel chromatography (hexane/ethyl acetate, 12:1→10:1→8:1→6:1→4:1), and then crystallized from heated ethanol to yield the same compound (9-5). The resultant compound was collected into a flask, and was to be used as it was for the next reaction.
LRMS m/z 511 $[M+Na]^+$.

Route E9; 1-O-ally-2,3-di-O-benzyl-β-D-glucopyranoside (9-6):

The compound (9-5) yielded through the route D9 was dissolved in acetic acid (72 mL), and further distilled water (40 mL) was added thereto. The solution was stirred for 1 hour while heated and refluxed. After the sufficient progress of the reaction was confirmed, the solution was cooled to room temperature, and then the solvent was removed by evaporation. Distilled water (15 mL) was added thereto, and the solution was again concentrated under reduced

pressure; this operation was repeated 4 times. Thereafter, the resultant residue was dissolved in methanol (150 mL). While the solution was stirred at room temperature under an atmosphere of nitrogen, thereto was added a 28% solution (0.7 mL) of sodium methylate in methanol. The solution was then stirred at room temperature for 2 hours. After the sufficient progress of the reaction was confirmed, the reaction was terminated. The reaction solution was concentrated, as it was, under reduced pressure, and purified by silica gel flash chromatography (hexane/ethyl acetate, 4:1→3:1→2:1→3:2→1:1→2:3→1:2) to yield the title compound (9-6) {9.3 g (23.2 mmol), 61.7% yield, which was the yield through the two reactions in the routes D9 and E9}.
LRMS m/z 423 $[M+Na]^+$.

Route F9; 1-O-ally-2,3,4-di-O-benzyl-6-O-tosyl-β-D-glucopyranoside (9-7):

To a solution of the compound (9-6) (9.3 g, 23.2 mmol) in anhydrous pyridine (120 mL) were added p-toluenesulfonyl chloride (5.8 g, 30.2 mmol) and 4-dimethylaminopyridine (283 mg, 2.3 mmol), and the reaction solution was stirred at 0°C for 16 hours. After the sufficient progress of the reaction was confirmed, the reaction solution was slowly poured into ice water (100 mL). The water layer was subjected to extraction with ethyl acetate (3 × 200 mL). The organic layers were combined with each other. The combination was washed with an aqueous 1 N HCl solution until the pH became 4, washed with saturated aqueous sodium hydrogen carbonate (2 × 100 mL) and saturated saline (2 × 100 mL), dried over sodium sulfate, filtrated and then concentrated under reduced pressure. The concentrated product was purified by silica gel flash chromatography (hexane/ethyl acetate, 6;1→4:1-→2:1) to yield the title compound (9-7) {12.3 g (22.2 mmol), 95.7% yield).
LRMS m/z 577 $[M+Na]^+$.

Route G9; 1-O-allyl-2,3-di-O-benzyl-4-O-(carbobenzoxy-β-alanyl)-6-O-tosyl-β-D-glucopyranoside (9-8):

Into a mixed solution of anhydrous dichloromethane (200 mL) and anhydrous pyridine (50 mL) were dissolved the compound (9-8) (12.3 g, 22.2 mmol), N-carbobenzoxy-β-alanine (12.1 g, 44.4 mmol), 1-ethyl-3-(3-dimethylaminopropyl)carbodiimide hydrochloride (EDCI·HCl) (17.0 mg, 88.8 mmol) and 4-dimethylaminopyridine (5.4 g, 44.4 mmol), and then the reactive components were caused to react with each other at room temperature for 18 hours. After the sufficient progress of the reaction was confirmed, water (10 mL) was poured into the reaction solution to terminate the reaction. Thereafter, the solution was concentrated under reduced pressure. The resultant residue was purified by silica gel chromatography (hexane/ethyl acetate, 4:1→3:1→2:1→3:2) to yield the title compound (9-8) {15.7 g (20.7 mmol), 93.0% yield}.
LRMS m/z 782 $[M+Na]^+$.

Route H9; 1-O-allyl-2,3-di-O-benzyl-4-O-(carbobenzoxy-β-alanyl)-6-thioacetyl-β-D-glucopyranoside (9-9):

Potassium thioacetate (4.7 g, 41.3 mmol) was added to a solution of the compound (9-8) (15.7 g, 20.7 mmol) in anhydrous N,N-dimethylformamide (200 mL), and the solution was stirred at 90°C for 3 hours. After the sufficient progress of the reaction was confirmed, the reaction solution was poured into cold water (400 mL), and the resultant was extracted with ethyl acetate (3 × 150 mL). The organic layers were combined with each other, and the combination was washed with saturated saline (2 × 100 mL), dried over sodium sulfate, filtrated and concentrated under reduced pressure. The resultant residue was purified by silica gel chromatography (hexane/ethyl acetate, 4:1→3:1→2:1→3:2) to yield the title compound (9-9) {13.6 g (20.5 mmol), 99.2% yield}.
LRMS m/z 686 $[M+Na]^+$.

Route 19; 3-O-[2,3-di-O-benzyl-4-O-(carbobenzoxy-β-alanyl)-6-thioacetyl-β-D-quinovopyranosyl-glycerol (9-10):

The compound (9-9) (13.6 g, 20.5 mmol) was dissolved in a solution (200 mL) of t-butyl alcohol and distilled water (4:1), and then thereto were added a 0.04 M osmium tetraoxide solution in t-butyl alcohol (5 mL) and trimethylamine N-oxide (3.4 g, 30.7 mmol). The solution was stirred with a stirrer at room temperature for 24 hours. After the sufficient progress of the reaction was confirmed, 3 g of activated carbon was added thereto and then the solution was stirred for 30 minutes to cause the catalyst to be adsorbed on the carbon. The solution was subjected to suction filtration through a Kiriyama funnel containing celite, so as to remove the catalyst. The reaction product remaining on the celite was washed three times with ethyl acetate so as to be collected. Distilled water (200 mL) was added to the collected filtrate, and the resultant was extracted with ethyl acetate (3 × 150 mL). The organic layers were combined with each other, and the combination was washed with saturated saline (2 × 100 mL), dried over sodium sulfate, filtrated and concentrated under reduced pressure. The resultant residue was purified by silica gel chromatography (toluene/ethyl acetate, 2:1→3:2→1:1→2:3→1:2→1:4) to yield the title compound (9-10) {11.7 g (16.8 mmol), 81.8% yield } .

LRMS m/z 720 $[M+Na]^+$.

Route J9; 3-O-(2,3-di-O-benzyl-4-O-(carbobenzoxy-β-alanyl)-6-thioacetyl-β-D-quinovopyranosyl]-1,2-di-O-stearoyl-glycerol (9-11):

Stearoyl chloride (5.4 g, 26.8 mmol) was added to a mixed solution of the compound (9-10) (11.7 g, 16.8 mmol) in anhydrous dichloromethane (200 mL) and anhydrous pyridine (50 mL), and then the solution was stirred at 0°C for 2 hours. After the sufficient progress of the reaction was confirmed, methanol (5 mL) was added thereto so as to terminate the reaction. The solution was concentrated under reduced pressure. A small amount of ethyl acetate was used to suspend the residue, and the suspended residue was poured into water (200 mL). The resultant was extracted with ethyl acetate (3 × 100 mL). The organic layers were combined with each other, and the combination was washed with saturated saline (2 × 100 mL), dried over sodium sulfate, filtrated and then concentrated under reduced pressure. The resultant residue was purified by silica gel chromatography (hexane/ethyl acetate, 6:1→4:1→2:1→3:2→1:1) to yield the title compound (9-11) as a colorless oily substance {3.3 g (2.7 mmol), 16.0% yield).
LRMS m/z 1253 $[M+Na]^+$.

Route K9; 3-O-[2,3-di-O-benzyl-4-O-(carbobenzoxy-β-alanyl)-6-sulfo-β-D-quinovopyranosyl]-1,2-di-O-stearoyl-glycerol (9-12):

Oxone (6.6 g, 10.7 mmol) and potassium acetate (2.5 g) were added to a solution of the compound (9-11) (3.3 g, 2.7 mmol) in acetic acid (100 g, 2.5 mol), and then the solution was vigorously stirred at room temperature for 48 hours. After the sufficient progress of the reaction was confirmed, the reaction solution was poured into a cold 7.5 M sodium hydroxide solution (100 mL) and the resultant was extracted with ethyl acetate (4 × 50 mL). The organic layers were combined with each other, and the combination was washed with saturated aqueous sodium hydrogen carbonate (2 × 50 mL) and saturated saline (2 × 50 mL), dried over sodium sulfate, filtrated and then concentrated under reduced pressure. The resultant residue was purified by silica gel chromatography (chloroform/methanol, 100:1→50:1→20:1→15:1→12.5:1→10:1→8:1) to yield the title compound (9-12) as a colorless waxy substance.
LRMS m/z 1235 $[M-Na]^-$.

Route L9; 3-O-[4-O-(β-alanyl)-6-sulfo-(β-D-quinovopyranosyl]-1,2-di-O-stearoyl-glycerol (9-13):

To a solution of the compound (9-12) (345 mg, 274 μmol) in methanol (10 mL), dichloromethane (15 mL) and acetic acid (0.6 mL) was added 10% palladium hydroxide-activated carbon (300 mg), and then the solution was stirred at room temperature under an atmosphere of hydrogen gas for 48 hours. After the sufficient progress of the reaction was confirmed, the palladium-activated carbon was filtrated off. The filtrate was concentrated under reduced pressure. The resultant residue (crude product of the compound (9-13)) was to be used, as it was, as a substance containing the title compound (9-13) for the next reaction.
LRMS m/z 921 $[M-Na]^-$.

Route M9; 3-O-[4-O-(4-iodobenzoyl-β-alanyl)-6-sulfo-β-D-quinovopyranosyl]-1,2-di-O-stearoyl-glycerol (9-14) :

The crude product of the compound (9-13) yielded through the route L9 was dissolved in a mixed solution of anhydrous dichloromethane (15 mL), pyridine (5 mL), and triethylamine (2 mL). Thereto was added (4-iodobenzoyl)-p-nitrophenyl ester (202 mg, 548 μmol). While the solution was stirred with a stirrer, the reactive components were caused to react with each other at room temperature for 24 hours. After the sufficient progress of the reaction was confirmed, toluene and methanol were added thereto. Under reduced pressure, the solvents were then removed by evaporation to concentrate the solution, while azeotropy. The resultant residue was purified by silica gel chromatography (chloroform/methanol/distilled water, 70:10:1→50:10:1→60:14:1.6→60:18:2→60:22:2.4→65:25:3) to yield the title compound (9-14) {77.6 mg (82.1 μmol), 30.0% yield}.
LRMS m/z 1151 $[M - Na]^-$.

Example 2. Biological distribution

1) Biotinated αSQMG

$1 \times 10^6$ of human esophageal squamous cell carcinoma cells TE-8 were transplanted into the right femoral region of 11 male KSN nude mice (Japan SLC, Inc.), and the mice were bred to form a tumor mass of about 300-900 $cm^3$ volume.

Thereafter, biotinated αSQMG C18:0 (formula XI) was dissolved in physiological saline solution, and 0.1 mL of the solution per administration was intravenously administered to each group comprising three mice so that the amount of the biotinated αSQMG C18:0 could be 40 mg/kg, 20 mg/kg, and 2 mg/kg. To two reference tumor bearing mice, physiological saline was administered. Each mouse was slaughtered 9 hours after administration, and tumor was extracted and immediately frozen. Frozen segments having a thickness of 5μm were prepared from the frozen tumor, and each segment was stained by avidin which was labeled with fluorescent pigment TRITC (Tetramethylrhodamine-5-(and-6)-isothiocyanate). Each stained segment was observed by fluorescent microscope and subjected to image processing with Adobe Photoshop after digitization.

In order to evaluate a tumor region which emits fluorescence due to TRITC, binarization imaging was performed with use of Photoshop. Thereafter, a portion (fraction) containing tumor tissues was selected from the visual field, and the number of pixels of a TRITC positive region (black color region) and the entire region were counted and the ratio of the positive region was calculated according to the formula:

The proportion (%) of biotinated αSQAP detected region = [the area of the black color region (region where the biotinated αSQMG was detected)]/[the area of the whole of the selected fraction] × 100 = [the number of pixels in the TRITC positive region] / [the number of pixels in the whole fraction] × 100 (%)     formula (A)

In the same manner, a different tumor tissue portion was selected, and with respect to 5 positions the ratio of a TRITC positive region was calculated and averaged out.

The results are shown in FIG. 1, indicating that tumor tissues were strongly stained according to the dose of biotinated αSQMG C18:0 and the tumor-resident amount of biotinated αSQMG C18:0 is high.

2) Biotinated αSQAP

Human giant cell lung carcinoma cells Lu65 were transplanted into six-week-old male KSN nude mice in a number of $1 \times 10^6$ for each of the mice, and the mice were bred for 14 days to form a tumor mass of about 100-200 $mm^3$ volume in each of the mice.

Thereafter, three mice were assigned to each of the following four groups (1) to (4):

(1) a control group wherein physiological saline was administered,
(2) a high dose group wherein biotinated αSQAP was administered in a dose of 50 mg/kg,
(3) a middle dose group wherein biotinated αSQAP was administered in a dose of 5 mg/kg, and
(4) a low dose group wherein biotinated αSQAP was administered in a dose of 1 mg/kg; the used biotinated αSQAP was the compound represented by the formula (2-11) .

The biotinated αSQAP dissolved in physiological saline, or only physiological saline for the control group was administered to each of the nude mice from its tail vein so as to give a predetermined amount out of the above-mentioned amounts. After one hour from the administration, a tumor was extirpated. The tumor piece was immersed into an OCT compound, and then frozen with liquid nitrogen.

The frozen tumor of each of the test animals was sliced into a thickness of 10 μm, and the slice was fixed onto a microplate. The region wherein biotin was distributed was dyed with an Alexa 488 labeled avidin.

The slice was observed by a fluorescence microscope, and the slice was made into an image. Data on the image were input to a computer, and then positive signals of the image were quantified according to image analysis software.

Specifically, binary image processing based on Photoshop was performed to estimate a tumor region emitting fluorescence through the labeling material Alexa 488 in the slice. Thereafter, a portion (fraction) containing tumor tissues

was selected from the visual field, and the number of pixels of the Alexa 488 positive region (black region) and the entire region were counted and the ratio of the positive region was calculated according to the formula:

The proportion (%) of biotinated αSQAP detected region = [the area of the black color region (region where the biotinated αSQAP was detected)]/[the area of the whole of the selected fraction] × 100 = [the number of pixels in the Alexa 488 positive region]/[the number of pixels in the whole fraction] × 100 (%) formula (B)

In the same way, different tumor tissue fractions were selected from 5 sites thereof, and about each of the sites the proportion of the Alexa 488 positive region was calculated. The resultant proportions were then averaged.

Further, in the case of using a different labeling material, substantially the same value can be calculated in accordance with the following expression:

The proportion (%) of the region where a substance to be analyzed was detected = [the area of the region where the signal of the labeling material was generated (region where the substance to be analyzed was detected)]/[the area of the whole of the selected fraction] × 100 = (the number of pixels in the labeling material positive region)/(the number of pixels in the whole of the selected fraction) × 100 (%) formula (C)

The results are shown in FIG. 2. In the high dose group, wherein the biotinated αSQAP was administered in a dose of 50 mg/kg, the tumor tissues were intensely dyed. It was demonstrated that the amount of biotinated αSQAP in tumor was large.

Example 3. Pharmacokinetics 1

(1) Animal tests

To eight-week-old male KSN nude mice (Japan SLC, Inc.), $2 \times 10^6$ of human colon carcinoma cells (SW480) were transplanted subcutaneously, two weeks later (body weight approximately 25 g, tumor volume approximately 200 $mm^3$) αSQMG C18:0 or αSQAP C18:0 was dissolved in physiological saline solution and administered intraperitoneally at a dose of 10 mg/kg (0.1 mL). After the administration, the mice were slaughtered chronologically with carbon dioxide; 30 minute, 1 hour, 2 hours, 4 hours, 12 hours, and 24 hours after the administration. Tissues of blood, liver, kidney, lung and spleen, and tumor tissue were then collected. At each time when tissues were collected, five animals were used.

(2) Pretreatment of analytical samples

Blood plasma

Whole blood was collected from postcaval vein with use of an injection needle wetted with heparin sodium injection solution (10,000 unit/ 10 mL) to obtain blood plasma by 20 minutes of centrifugation at 6,000 g. To 90 μl of blood plasma, 5 μl of an internal standard compound (αSQMG C16:0), 5 μl of distilled water, and 4 μl of phosphoric acid were added, and denatured protein by adding 96 μl of acetonitrile. To 0.2 mL of the solution, 600 μl of 10 mM ammonium acetate solution was added, and stirred sufficiently to obtain supernatant by centrifugation at 16,000 g for 15 minutes. The supernatant was subjected to solid-phase extraction operation as described below. The solid-phase extraction was performed by washing with 1000 μl of 10 mM ammonium acetate aqueous solution and 200 μl of 10 mM ammonium acetate aqueous solution containing 10% acetonitrile, followed by elution of a test compound with 200 μl of 10 mM ammonium acetate aqueous solution containing 70% acetonitrile with use of Empore (trademark) disc cartridge (3M Company, product number 4115SD). The compound was used as a LC/MS analytical sample.

Organ samples

Each organ was extirpated after collection of blood and immediately frozen on dry ice. The frozen sample was subjected to frost shattering, and the weight was measured. Subsequently, 1/2-fold (weight ratio) internal standard compound (αSQMG C16:0) and distilled water were added, and 8-fold (weight ratio) extraction solution (2.5% NP-40, 2% phosphoric acid, 2 mM Eserine aqueous solution) was further added to the sample, followed by homogenization. The homogenized solution was subjected to protein denaturation with acetonitrile and solid phase extraction in the same manner as the above-mentioned treatment of blood plasma to obtain an analytical sample.

(3) Quantitative analysis assay

10 μl of the analytical sample was separated with a reverse phase HPLC system equipped with Capcell Pak MG column (particle diameter; 3 μm, column size; 2.1 × 50 mm, Shiseido Co., Ltd.). HPLC was performed with 68% acetonitrile and a single solvent of 10 mM ammonium acetate aqueous solution at a flow rate of 0.2 mL per minute. Mass spectrum was measured in a negative mode, and the peak area ratio of the internal standard compound was calculated by integration of the peak area from chromatogram corresponding to each ion by measuring a precursor ion; m/z = 583.1, fragment ion; m/z = 224.7 and m/z = 298.8 for αSQMG C18:0, a precursor ion; m/z = 567.1, fragment ion; m/z = 224.7 and m/z = 283.1 for αSQAP C18:0, and a precursor ion; m/z = 555.1, fragment ion; m/z = 224.7 and m/z = 298.8 for a internal standard compound αSQMG C16:0. As a method of quantitative analysis, an internal standard analysis was adopted. More specifically, a standard curve was drawn based on the peak area ratio of the internal standard compound by analyzing the sample to which the test compound was added, to determine the quantity of the test compound.

FIG. 3 shows a concentration-time transition curve in blood plasma and organs of nude mice to which αSQMG C18:0 was intraperitoneally administered in a ratio of 10 mg/kg.

As is shown by FIG. 3, in tissues including blood plasma, liver, kidney, spleen and lung, the highest concentration of αSQMG C18:0 was indicated immediately after the administration of αSQMG C18:0, thereafter the concentration of αSQMG C18:0 fell rapidly. In liver, lung, kidney, and blood plasma, four hours after the administration and in spleen 12 hours thereafter, the concentration of αSQMG C18:0 fell below the detection limit. On the other hand, in tumor tissues, the concentration of αSQMG C18:0 in tissue was low immediately after administration of αSQMG C18:0 in comparison with other tissues, but it was detectable even 24 hours after administration. Therefore, it was proved that αSQMG C18:0 resides for a long period in tumor tissues.

FIG. 4 shows a concentration-time transition curve in blood plasma and organs of nude mice to which αSQAP C18:0 was intraperitoneally administered in a ratio of 10 mg/kg.

From the results of FIG. 4, in tissues including blood plasma, liver, kidney, spleen and lung, the highest concentration of αSQAP C18:0 was indicated immediately after the administration of αSQAP C18:0, thereafter the concentration of αSQAP C18:0 fell rapidly. In spleen, lung, kidney, and blood plasma, four hours after the administration and in liver 12 hours thereafter, the concentration of αSQAP C18:0 fell below the detection limit. On the other hand, in tumor tissues, the concentration of αSQAP C18:0 in tissue was low immediately after administration of αSQAP C18:0 in comparison with other tissues, but it was detectable even 24 hours after administration. Therefore, it was proved that αSQAP C18:0 resides for a long period in tumor tissues.

Example 4. Pharmacokinetics 2

The pharmacokinetics of α8QAP C18:0 was tested by administering 1 mg/kg of αSQAP C18:0 to animals in the same manner as Example 3 except that human sophageal squamous cell carcinoma cells TE-8 were used as tumor of transplanted tumor model and that intravenous administration was adopted as substitute for interperitoneal administration.

The results are shown in FIG. 5. As is shown by FIG. 5, in tissues including blood plasma, liver, spleen and lung, the highest concentration of αSQAP C18:0 in tissue was indicated immediately after administration of αSQAP C18:0, and thereafter αSQAP C18:0 rapidly fell. In blood plasma, liver, and lung two hours after the administration, in spleen 24 hours thereafter, the concentration of αSQAP C18:0 fell below the detection limit. On the other hand, in comparison with other organs the concentration in tumor tissue was low immediately after the administration of αSQAP C18:0, but no decrease tendency was observed even after two hours and the concentration of αSQAP C18:0 was detectable even after 24 hours. Therefore, it was proved that αSQAP C18:0 resides for a long period in tumor tissues.

Industrial Applicability

The novel compound of the present invention specifically resides in a tumor and is a substance containing an acting group in the structure.

Therefore, according to the type of the acting group it can be advantageously used for drug deliver to tumor, detection and diagnosis of tumor, treatment of tumor, and the like.

**Claims**

**1.** A compound represented by chemical formula (I):

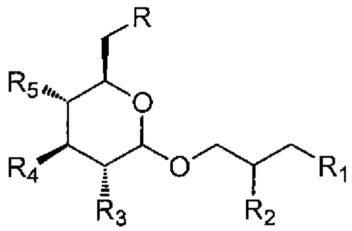

(I)

wherein R is an anionic group binding to hydrogen, $R_1$ is OH, OCOH, $OCO(CH_2)_hCH_3$, or an acting group, h being an integer of 0 or more,
$R_2$ is H, OH, OCOH, $OCO(CH_2)_iCH_3$, or an acting group, i being an integer of 0 or more,
$R_3$ is OH, $SO_3H$, or an acting group,
$R_4$ is OH, $SO_3H$, or an acting group, and
$R_5$ is OH, $SO_3H$, or an acting group,
at least one of $R_1$, $R_2$, $R_3$, $R_4$, and $R_5$ containing an acting group,
or pharmaceutically acceptable salts thereof.

**2.** A compound represented by chemical formula (II):

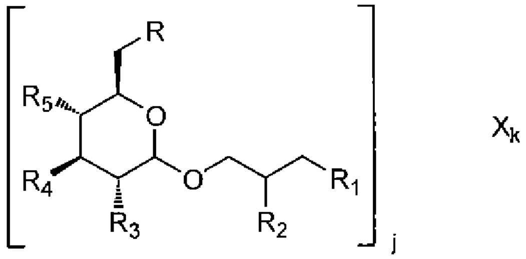

(II)

wherein R is an anionic group binding to hydrogen, $R_1$ is OH, OCOH, $OCO(CH_2)_hCH_3$, or an acting group, h being an integer of 0 or more,
$R_2$ is H, OH, OCOH, $OCO(CH_2)_iCH_3$, or an acting group, i being an integer of 0 or more,
$R_3$ is OH, $SO_3H$, or an acting group,
$R_4$ is OH, $SO_3H$, or an acting group,
$R_5$ is OH, $SO_3H$, or an acting group,
j is an integer of 1 or more,
X is an acting group or a cationic group, and
k is an integer of 1 or more,
at least one of $R_1$, $R_2$, $R_3$, $R_4$, $R_5$, and X containing an acting group,
or pharmaceutically acceptable salts thereof.

**3.** The compound or salts according to claim 1 or 2,
wherein R is $SO_3H$,
$R_3$ is OH,
$R_4$ is OH,
$R_5$ is OH,
$R_1$ is OH, OCOH, $OCO(CH_2)_hCH_3$, O- (an acting group), OCO- (an acting group), or $OCO(CH_2)_hCH_2$- (an acting group), h being an integer of 0 or more, and
$R_2$ is H, OH, OCOH, $OCO(CH_2)_iCH_3$, an acting group, O- (an acting group), OCO- (an acting group), or $OCO(CH_2)_iCH_2$- (an acting group), i being an integer of 0 or more,
at least one of $R_1$ and $R_2$ containing an acting group.

**4.** The compound or salts according to claim 1 or 2,
wherein R is $SO_3H$,
$R_1$ is OH, OCOH, $OCO(CH_2)_hCH_3$, or an acting group, h being an integer of 0 or more,
$R_2$ is H, OH, OCOH, $OCO(CH_2)_iCH_3$ or an acting group, i being an integer of 0 or more, and
$R_3 = R_4 = R_5$ is OH, or at least one of $R_3$, $R_4$ and $R_5$ contains an acting group and the other groups are OH,
at least one of $R_1$, $R_2$, $R_3$, $R_4$, and $R_5$ containing an acting group.

**5.** Sulfopyranosyl(acyl)glycerol, sulfopyranosyl(acyl)propanediol, or sulfoquinovosylacyl propanediol, wherein any of side residues binds to an acting group,
or pharmaceutically acceptable salts.

**6.** The compound or salts according to any one of claims 1-5, wherein the acting group is a labeled substance or an antitumor substance.

**7.** A method for allowing the compound represented by formula (I) or (II) or salts to reside in a tumor, wherein the compound or salts according to any one of claims 1-6 are administered to a subject.

**8.** A method for detecting tumor, comprising:

administering the compound or salts according to any one of claims 1-5 to a subject, wherein the acting group is a labeled substance;
detecting the labeled substance when the compound or salts exist in a higher concentration in a tumor than in other tissues except a tumor; and
detecting tumor in the subject based on results of the detecting.

**9.** A method for diagnosing tumor, comprising:

administering the compound or salts according to any one of claims 1-5 to a subject, wherein the acting group is a labeled substance;
detecting the labeled substance when the compound or salts exist in a higher concentration in a tumor than in other tissues except a tumor; and
determining presence or absence of tumor in the subject based on results of the detecting.

**10.** A method according to claim 8 or 9, wherein the detecting is carried out by a noninvasive means.

11. A method for treating tumor, comprising administering the compound or salts according to any one of claims 1-5 to a subject, wherein the acting group is an antitumor substance.

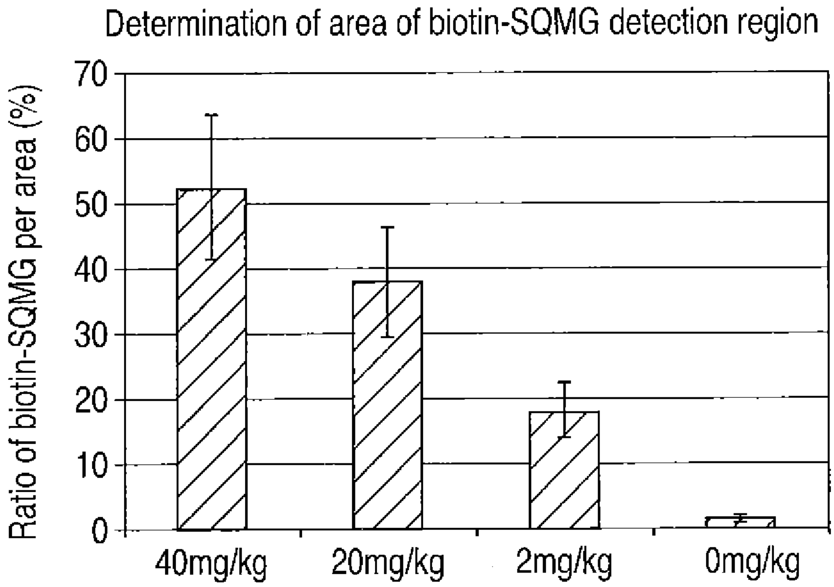
Determination of area of biotin-SQMG detection region
Ratio of biotin-SQMG per area (%)
70
60
50
40
30
20
10
0
40mg/kg
20mg/kg
2mg/kg
0mg/kg

FIG. 1

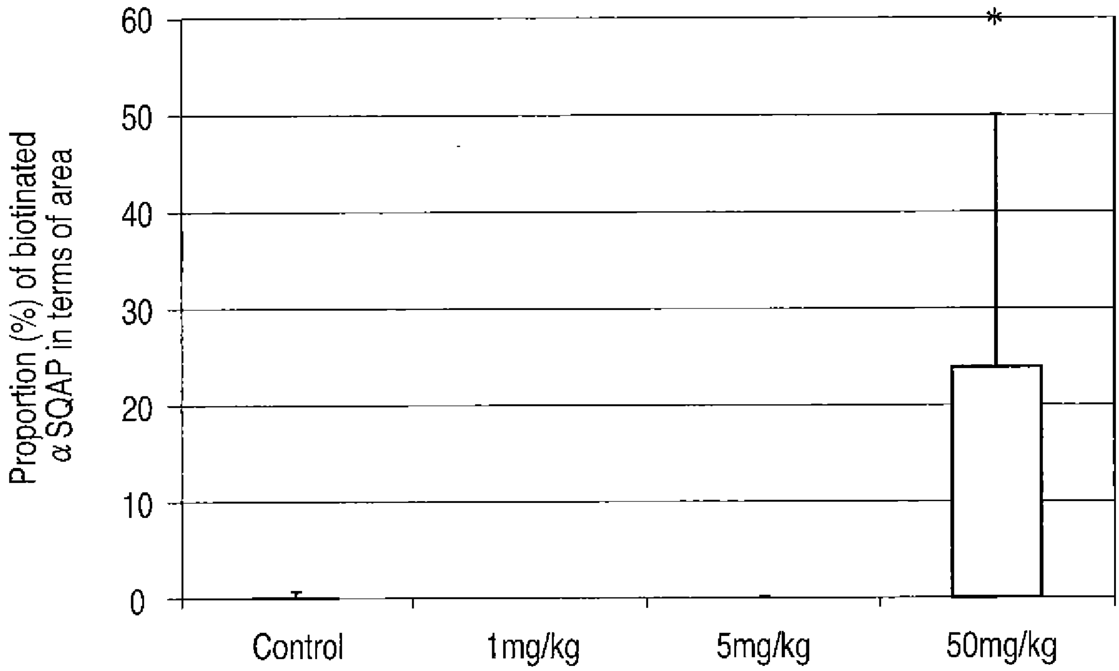


* ; A significant difference ($p<0.05$) is observed compared with the value of the control group (Student's t-test)

FIG. 2

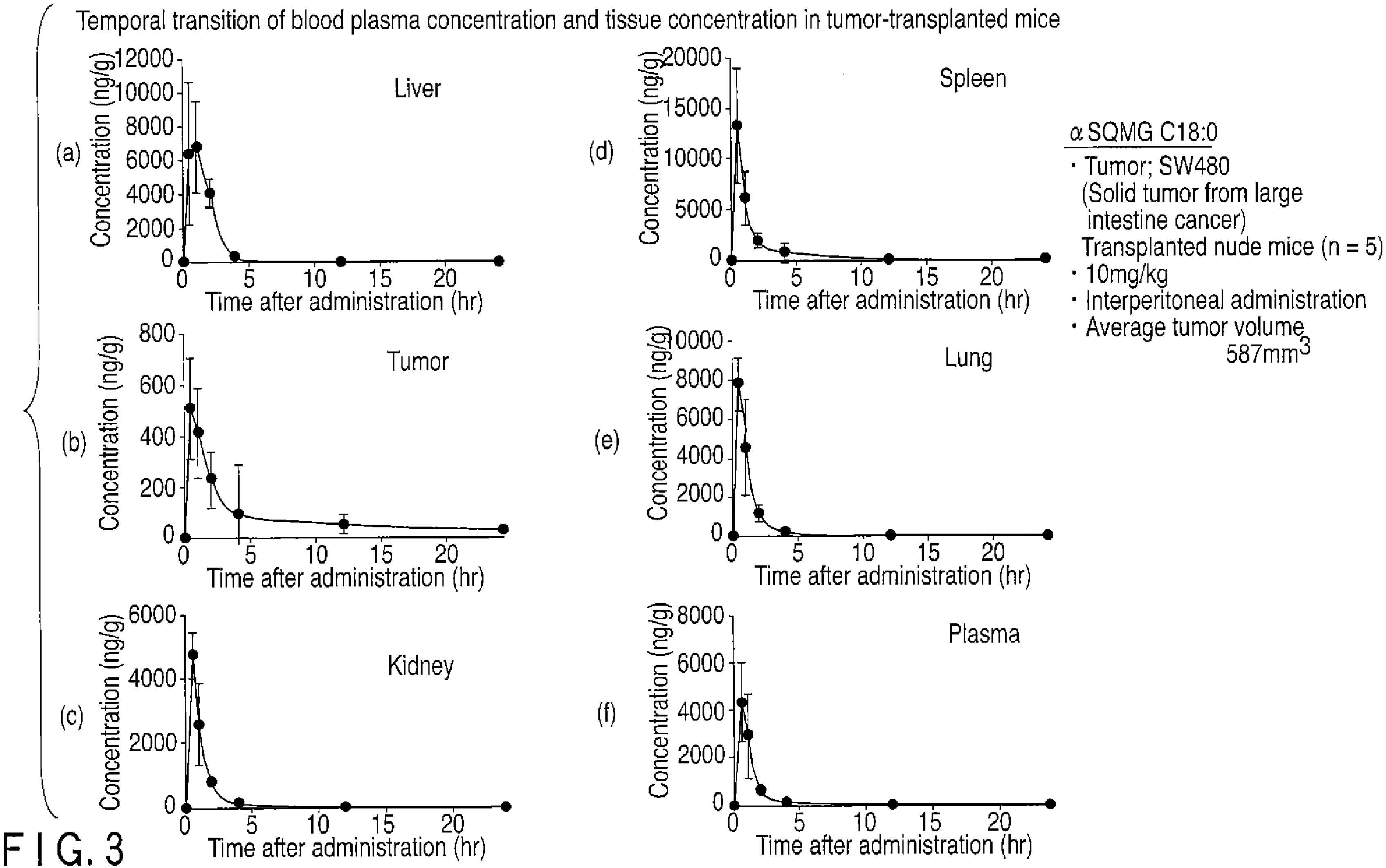
Temporal transition of blood plasma concentration and tissue concentration in tumor-transplanted mice
(a)
Liver
Concentration (ng/g)
12000
10000
8000
6000
4000
2000
0
Time after administration (hr)
0
5
10
15
20
(b)
Tumor
Concentration (ng/g)
800
600
400
200
0
Time after administration (hr)
(c)
Kidney
Concentration (ng/g)
6000
4000
2000
0
Time after administration (hr)
(d)
Spleen
Concentration (ng/g)
20000
15000
10000
5000
0
Time after administration (hr)
(e)
Lung
Concentration (ng/g)
10000
8000
6000
4000
2000
0
Time after administration (hr)
(f)
Plasma
Concentration (ng/g)
8000
6000
4000
2000
0
Time after administration (hr)
α SQMG C18:0
· Tumor; SW480
(Solid tumor from large
intestine cancer)
Transplanted nude mice (n = 5)
· 10mg/kg
· Interperitoneal administration
· Average tumor volume
587mm3

FIG. 3

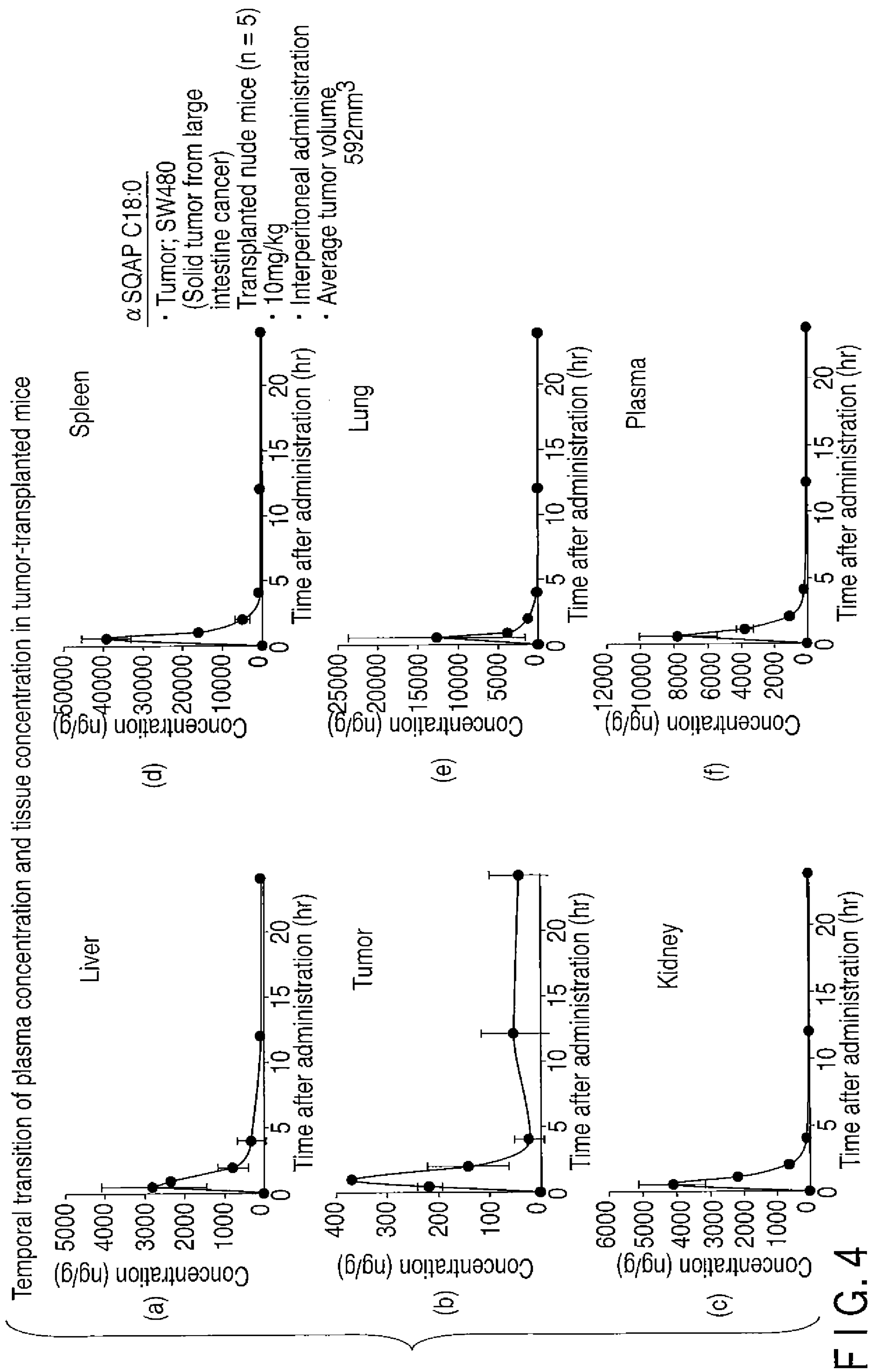
Temporal transition of plasma concentration and tissue concentration in tumor-transplanted mice
(a)
Liver
(b)
Tumor
(c)
Kidney
(d)
Spleen
(e)
Lung
(f)
Plasma
Concentration (ng/g)
Time after administration (hr)
α SQAP C18:0
· Tumor; SW480
(Solid tumor from large intestine cancer)
Transplanted nude mice (n = 5)
· 10mg/kg
· Interperitoneal administration
· Average tumor volume 592mm3

FIG. 4

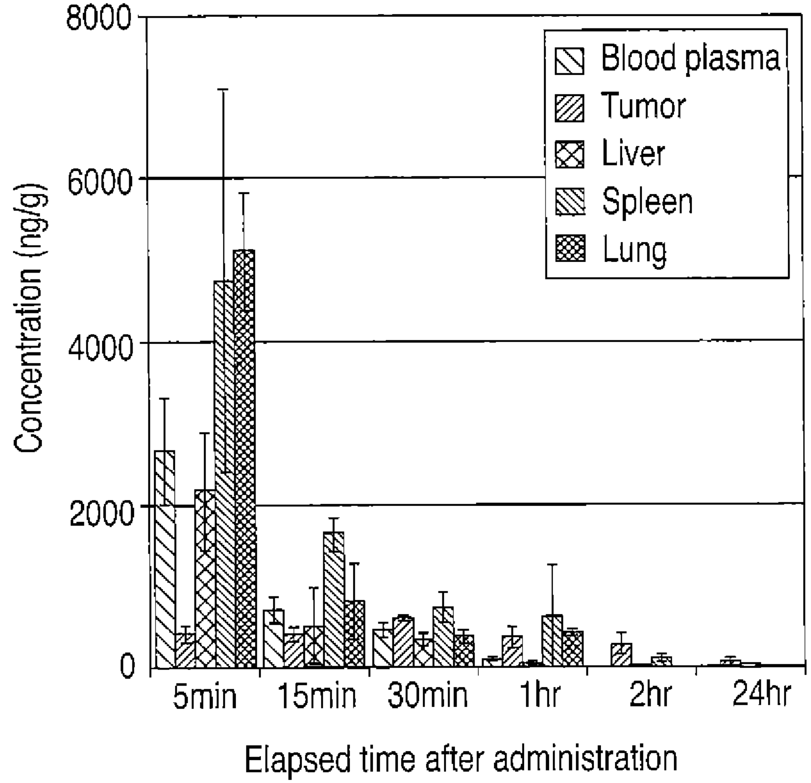


*α* SQAP C18:0

- Transplanted tumor; TE-8
  (Solid tumor from esophageal cancer)
- Animal: KSN nude mice (n = 3)
- Dose 1 mg/kg
- Intravenous administration
- Average tumor volume; 1700 $mm^3$

FIG. 5

**INTERNATIONAL SEARCH REPORT**

International application No.
PCT/JP2010/050432

A. CLASSIFICATION OF SUBJECT MATTER
*C07H15/26*(2006.01)i, *A61K47/48*(2006.01)i, *A61K49/00*(2006.01)i, *A61K49/04*(2006.01)i, *A61K51/00*(2006.01)i

According to International Patent Classification (IPC) or to both national classification and IPC

B. FIELDS SEARCHED

Minimum documentation searched (classification system followed by classification symbols)
C07H15/26, A61K47/48, A61K49/00, A61K49/04, A61K51/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
PubMed, Science Direct, CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | YAMAZAKI, T. et al, Synthesis of an immunosuppressant SQAG9 and determination of the binding peptide by T7 phage display, Bioorganic & Medicinal Chemistry Letters, 2004, Vol.14, No.16, p.4343-4346, compound 2 | 1-2,4-6 |
| X | AOKI, S. et al, Mammalian mitotic centromere-associated kinesin (MCAK): a new molecular target of sulfoquinovosylacylglycerols novel antitumor and immunosuppressive agents, FEBS J, 2005, Vol.272, No.9, p.2132-2140, compound B | 1-2,4-6 |
| A | HANASHIMA, S. et al, Structure-activity relationship of a novel group of mammalian DNA polymerase inhibitors, synthetic sulfoquinovosylacylglycerols, Jpn J Cancer Res, 2000, Vol.91, No.10, p.1073-1083, entire text | 1-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 April, 2010 (06.04.10) | 20 April, 2010 (20.04.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/050432

C (Continuation). DOCUMENTS CONSIDERED TO BE RELEVANT

| Category* | Citation of document, with indication, where appropriate, of the relevant passages | Relevant to claim No. |
|---|---|---|
| A | HANASHIMA, S. et al, Structural determination of sulfoquinovosyldiacylglycerol by chiral syntheses, Tetrahedron Lett, 2000, Vol.41, p.4403-4407, entire text | 1-6 |
| A | HANASHIMA, S. et al, Synthesis of sulfoquinovosylacylglycerols, inhibitors of eukaryotic DNA polymerase alpha and beta, Bioorg Med Chem, 2001, Vol.9, No.2, p.367-376, entire text | 1-6 |
| A | SAHARA, H. et al, In vivo anti-tumour effect of 3'-sulphonoquinovosyl 1'-monoacylglyceride isolated from sea urchin (Strongylocentrotus intermedius) intestine, Br J Cancer, 1997, Vol.75, No.3, p.324-332, entire text | 1-6 |

Form PCT/ISA/210 (continuation of second sheet) (July 2009)

**INTERNATIONAL SEARCH REPORT**

International application No.
PCT/JP2010/050432

**Box No. II Observations where certain claims were found unsearchable (Continuation of item 2 of first sheet)**

This international search report has not been established in respect of certain claims under Article 17(2)(a) for the following reasons:

1. [X] Claims Nos.: 7-11
because they relate to subject matter not required to be searched by this Authority, namely:
Claims 7 and 11 include methods for treatment of the human body or animal body by surgery or therapy and thus relate to a subject matter on which this International Searching Authority is not required to carry out a search.
(Continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest and, where applicable, the payment of a protest fee.
[ ] The additional search fees were accompanied by the applicant's protest but the applicable protest fee was not paid within the time limit specified in the invitation.
[ ] No protest accompanied the payment of additional search fees.

**INTERNATIONAL SEARCH REPORT**

International application No.
PCT/JP2010/050432

Continuation of Box No.II-1 of continuation of first sheet(2)

Claims 8 to 10 include diagnosis of the human body or animal body and thus relate to a subject matter on which this International Searching Authority is not required to carry out a search.